# EUROPEAN PATENT APPLICATION

(11) **EP 1 712 619 A1**
(43) Date of publication of application: **18.10.2006**
(21) Application number: 04808074.1
(22) Date of filing: 22.12.2004
(51) Int. Cl.: C12N 15/00, A61K 39/395, A61K 45/00, A61P 35/00, A61P 43/00, G01N 33/15, G01N 33/50, C07K 16/18

(54) **PREVENTIVE/REMEDY FOR CANCER**

(30) Priority: 24.12.2003 JP 2003427782
(71) Applicant: Takeda Pharmaceutical Company Limited, Osaka-shi, Osaka 541-0045 (JP)
(72) Inventor: SUNAHARA, Eiji, c/o Takeda Pharm. Co Ltd., Osaka-shi, Osaka 532-8686 (JP); ISHII, Takafumi, c/oTakeda Pharmac. Company Ltd., Osaka-shi, Osaka 532-8686 (JP)
(74) Representative: Rickard, Timothy Mark Adrian
(86) International application number: PCT/JP2004/019724
(87) International publication number: WO 2005/061704

(57) **Abstract**

The present invention provides a substance that inhibits the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof; an agent for preventing/treating cancer, etc., an agent for promoting apoptosis in cancer cells, an agent for inhibiting cancer cell growth, and so on, which comprises the substance described above.

SEQUENCE LISTING

<110> Takeda Pharmaceutical Company Limited

<120> Preventing or treating agent for cancer

<130> P04-215PCT

<150> JP 2003-427782
<151> 2003-12-24

<160> 38

<210> 1
<211> 837
<212> PRT
<213> Homo sapiens

<400> 1
Met Leu Arg Thr Ala Met Gly Leu Arg Ser Trp Leu Ala Ala Pro Trp
                5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Met Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg

                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Lys Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu

                805                 810                 815
Val Ser Pro Val Cys Pro Arg Pro Arg Val Arg Leu Gly Ser Glu Ile
            820                 825                 830
Arg Asp Ser Val Val
        835

<210> 2
<211> 2511
<212> DNA
<213> Homo sapiens

<400> 2
atgctgcgca ccgcgatggg cctgaggagc tggctcgccg ccccatgggg cgcgctgccg 60
cctcggccac cgctgctgct gctcctgctg ctgctgctcc tgctgcagcc gccgcctccg 120
acctgggcgc tcagcccccg gatcagcctg cctctgggct ctgaagagcg gccattcctc 180
agattcgaag ctgaacacat ctccaactac acagcccttc tgctgagcag ggatggcagg 240
accctgtacg tgggtgctcg agaggccctc tttgcactca gtagcaacct cagcttcctg 300
ccaggcgggg agtaccagga gctgctttgg ggtgcagacg cagagaagaa acagcagtgc 360
agcttcaagg gcaaggaccc acagcgcgac tgtcaaaact acatcaagat cctcctgccg 420
ctcagcggca gtcacctgtt cacctgtggc acagcagcct tcagccccat gtgtacctac 480
atcaacatgg agaacttcac cctggcaagg gacgagaagg ggaatgtcct cctggaagat 540
ggcaagggcc gttgtccctt cgacccgaat ttcaagtcca ctgccctggt ggttgatggc 600
gagctctaca ctggaacagt cagcagcttc caagggaatg acccggccat ctcgcggagc 660
caaagccttc gccccaccaa gaccgagagc tccctcaact ggctgcaaga cccagctttt 720
gtggcctcag cctacattcc tgagagcctg ggcagcttgc aaggcgatga tgacaagatc 780
tactttttct tcagcgagac tggccaggaa tttgagttct ttgagaacac cattgtgtcc 840
cgcattgccc gcatctgcaa gggcgatgag ggtggagagc gggtgctaca gcagcgctgg 900
acctccttcc tcaaggccca gctgctgtgc tcacggcccg acgatggctt ccccttcaac 960
gtgctgcagg atgtcttcac gctgagcccc agcccccagg actggcgtga cacccttttc 1020
tatggggtct tcacttccca gtggcacagg ggaactacag aaggctctgc cgtctgtgtc 1080
ttcacaatga aggatgtgca gagagtcttc agcggcctct acaaggaggt gaaccgtgag 1140
acacagcaga tggtacaccg tgacccaccc gtgcccacac cccggcctgg agcgtgcatc 1200
accaacagtg cccgggaaag gaagatcaac tcatccctgc agctcccaga ccgcgtgctg 1260
aactttctca aggaccactt cctgatggac gggcaggtcc gaagccgcat gctgctgctg 1320
cagccccagg ctcgctacca gcgcgtggct gtacaccgcg tccctggcct gcaccacacc 1380
tacgatgtcc tcttcctggg cactggtgac ggccggctcc acaaggcagt gagcgtgggc 1440
ccccgggtgc acatcattga ggagctgcag atcttctcat cgggacagcc cgtgcagaat 1500
ctgctcctgg acacccacag ggggctgctg tatgcggcct cacactcggg cgtagtccag 1560
gtgcccatgg ccaactgcag cctgtaccgg agctgtgggg actgcctcct cgcccgggac 1620
ccctactgtg cttggagcgg ctccagctgc aagcacgtca gcctctacca gcctcagctg 1680
gccaccaggc cgtggatcca ggacatcgag ggagccagcg ccaaggacct ttgcagcgcg 1740
tcttcggttg tgtccccgtc ttttgtacca acaggggaga agccatgtga gcaagtccag 1800
ttccagccca acacagtgaa cactttggcc tgcccgctcc tctccaacct ggcgacccga 1860
ctctggctac gcaacggggc ccccgtcaat gcctcggcct cctgccacgt gctacccact 1920
ggggacctgc tgctggtggg cacccaacag ctgggggagt tccagtgctg gtcactagag 1980
gagggcttcc agcagctggt agccagctac tgcccagagg tggtggagga cggggtggca 2040
gaccaaacag atgagggtgg cagtgtaccc gtcattatca gcacatcgcg tgtgagtgca 2100
ccagctggtg gcaaggccag ctggggtgca gacaggtcct actggaagga gttcctggtg 2160
atgtgcacgc tctttgtgct ggccgtgctg ctcccagttt tattcttgct ctaccggcac 2220
cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccactcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 3
<211> 3766
<212> DNA
<213> Homo sapiens

<400> 3
gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctgctg ctgctcctgc tgcagccgcc gcctccgacc 360
tgggcgctca gcccccggat cagcctgcct ctgggctctg aagagcggcc attcctcaga 420
ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatggaga acttcaccct ggcaagggac gagaagggga atgtcctcct ggaagatggc 780
aagggccgtt gtcccttcga cccgaatttc aagtccactg ccctggtggt tgatggcgag 840
ctctacactg gaacagtcag cagcttccaa gggaatgacc cggccatctc gcggagccaa 900
agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080
attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc ccccaggact ggcgtgacac ccttttctat 1260
ggggtcttca cttcccagtg gcacagggga actacagaag gctctgccgt ctgtgtcttc 1320
acaatgaagg atgtgcagag agtcttcagc ggcctctaca aggaggtgaa ccgtgagaca 1380
cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
tttctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtaccggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg ggggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcacccca 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300
ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540

ccaccatatc caccctcgct ccatctttga actcaaacac gaggaactaa ctgcaccctg 3600
gtcctctccc cagtccccag ttcaccctcc atccctcacc ttcctccact ctaagggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 4
<211> 837
<212> PRT
<213> Homo sapiens

<400> 4
   Met Leu Arg Thr Ala Met Gly Leu Arg Ser Trp Leu Ala Ala Pro Trp
                   5                   10                  15
   Gly Ala Leu Pro Pro Arg Pro Pro Leu Leu Leu Leu Leu Leu Leu Leu
               20                  25                  30
   Leu Leu Leu Gln Pro Pro Pro Pro Thr Trp Ala Leu Ser Pro Arg Ile
           35                  40                  45
   Ser Leu Pro Leu Gly Ser Glu Glu Arg Pro Phe Leu Arg Phe Glu Ala
       50                  55                  60
   Glu His Ile Ser Asn Tyr Thr Ala Leu Leu Leu Ser Arg Asp Gly Arg
   65                  70                  75                  80
   Thr Leu Tyr Val Gly Ala Arg Glu Ala Leu Phe Ala Leu Ser Ser Asn
                   85                  90                  95
   Leu Ser Phe Leu Pro Gly Gly Glu Tyr Gln Glu Leu Leu Trp Gly Ala
               100                 105                 110
   Asp Ala Glu Lys Lys Gln Gln Cys Ser Phe Lys Gly Lys Asp Pro Gln
           115                 120                 125
   Arg Asp Cys Gln Asn Tyr Ile Lys Ile Leu Leu Pro Leu Ser Gly Ser
       130                 135                 140
   His Leu Phe Thr Cys Gly Thr Ala Ala Phe Ser Pro Met Cys Thr Tyr
   145                 150                 155                 160
   Ile Asn Met Glu Asn Phe Thr Leu Ala Arg Asp Glu Lys Gly Asn Val
                   165                 170                 175
   Leu Leu Glu Asp Gly Lys Gly Arg Cys Pro Phe Asp Pro Asn Phe Lys
               180                 185                 190
   Ser Thr Ala Leu Val Val Asp Gly Glu Leu Tyr Thr Gly Thr Val Ile
           195                 200                 205
   Ser Phe Gln Gly Asn Asp Pro Ala Ile Ser Arg Ser Gln Ser Leu Arg
       210                 215                 220
   Pro Thr Lys Thr Glu Ser Ser Leu Asn Trp Leu Gln Asp Pro Ala Phe
   225                 230                 235                 240
   Val Ala Ser Ala Tyr Ile Pro Glu Ser Leu Gly Ser Leu Gln Gly Asp
                   245                 250                 255
   Asp Asp Lys Ile Tyr Phe Phe Phe Ser Glu Thr Gly Gln Glu Phe Glu
               260                 265                 270
   Phe Phe Glu Asn Thr Ile Val Ser Arg Ile Ala Arg Ile Cys Lys Gly
           275                 280                 285
   Asp Glu Gly Gly Glu Arg Val Leu Gln Gln Arg Trp Thr Ser Phe Leu
       290                 295                 300
   Lys Ala Gln Leu Leu Cys Ser Arg Pro Asp Asp Gly Phe Pro Phe Asn
   305                 310                 315                 320
   Val Leu Gln Asp Val Phe Thr Leu Ser Pro Ser Pro Gln Asp Trp Arg
                   325                 330                 335
   Asp Thr Leu Phe Tyr Gly Val Phe Thr Ser Gln Trp His Arg Gly Thr
               340                 345                 350
   Thr Glu Gly Ser Ala Val Cys Val Phe Thr Met Lys Asp Val Gln Arg
           355                 360                 365
   Val Phe Ser Gly Leu Tyr Lys Glu Val Asn Arg Glu Thr Gln Gln Met
       370                 375                 380
   Val His Arg Asp Pro Pro Val Pro Thr Pro Arg Pro Gly Ala Cys Ile
   385                 390                 395                 400
   Thr Asn Ser Ala Arg Glu Arg Lys Ile Asn Ser Ser Leu Gln Leu Pro
                   405                 410                 415
   Asp Arg Val Leu Asn Phe Leu Lys Asp His Phe Leu Met Asp Gly Gln
               420                 425                 430
   Val Arg Ser Arg Met Leu Leu Leu Gln Pro Gln Ala Arg Tyr Gln Arg
           435                 440                 445
   Val Ala Val His Arg Val Pro Gly Leu His His Thr Tyr Asp Val Leu
       450                 455                 460
   Phe Leu Gly Thr Gly Asp Gly Arg Leu His Lys Ala Val Ser Val Gly
   465                 470                 475                 480
   Pro Arg Val His Ile Ile Glu Glu Leu Gln Ile Phe Ser Ser Gly Gln
                   485                 490                 495
   Pro Val Gln Asn Leu Leu Leu Asp Thr His Arg Gly Leu Leu Tyr Ala
               500                 505                 510
   Ala Ser His Ser Gly Val Val Gln Val Pro Met Ala Asn Cys Ser Leu
           515                 520                 525
   Tyr Arg Ser Cys Gly Asp Cys Leu Leu Ala Arg Asp Pro Tyr Cys Ala
       530                 535                 540
   Trp Ser Gly Ser Ser Cys Lys His Val Ser Leu Tyr Gln Pro Gln Leu
   545                 550                 555                 560
   Ala Thr Arg Pro Trp Ile Gln Asp Ile Glu Gly Ala Ser Ala Lys Asp
                   565                 570                 575
   Leu Cys Ser Ala Ser Ser Val Val Ser Pro Ser Phe Val Pro Thr Gly
               580                 585                 590
   Glu Lys Pro Cys Glu Gln Val Gln Phe Gln Pro Asn Thr Val Asn Thr
           595                 600                 605
   Leu Ala Cys Pro Leu Leu Ser Asn Leu Ala Thr Arg Leu Trp Leu Arg
       610                 615                 620
   Asn Gly Ala Pro Val Asn Ala Ser Ala Ser Cys His Val Leu Pro Thr
   625                 630                 635                 640
   Gly Asp Leu Leu Leu Val Gly Thr Gln Gln Leu Gly Glu Phe Gln Cys
                   645                 650                 655
   Trp Ser Leu Glu Glu Gly Phe Gln Gln Leu Val Ala Ser Tyr Cys Pro
               660                 665                 670
   Glu Val Val Glu Asp Gly Val Ala Asp Gln Thr Asp Glu Gly Gly Ser
           675                 680                 685
   Val Pro Val Ile Ile Ser Thr Ser Arg Val Ser Ala Pro Ala Gly Gly
       690                 695                 700
   Lys Ala Ser Trp Gly Ala Asp Arg Ser Tyr Trp Lys Glu Phe Leu Val
   705                 710                 715                 720
   Met Cys Thr Leu Phe Val Leu Ala Val Leu Leu Pro Val Leu Phe Leu
                   725                 730                 735
   Leu Tyr Arg His Arg Asn Ser Met Lys Val Phe Leu Lys Gln Gly Glu
               740                 745                 750
   Cys Ala Ser Val His Pro Lys Thr Cys Pro Val Val Leu Pro Pro Glu
           755                 760                 765
   Thr Arg Pro Leu Asn Gly Leu Gly Pro Pro Ser Thr Pro Leu Asp His
       770                 775                 780
   Arg Gly Tyr Gln Ser Leu Ser Asp Ser Pro Pro Gly Ser Arg Val Phe
   785                 790                 795                 800
   Thr Glu Ser Glu Lys Arg Pro Leu Ser Ile Gln Asp Ser Phe Val Glu
                   805                 810                 815
   Val Ser Pro Val Cys Pro Arg Pro Arg Val Arg Leu Gly Ser Glu Ile
               820                 825                 830
   Arg Asp Ser Val Val
           835

<210> 5
<211> 2511

<212> DNA
<213> Homo sapiens

<400> 5
atgctgcgca ccgcgatggg cctgaggagc tggctcgccg ccccatgggg cgcgctgccg 60
cctcggccac cgctgctgct gctcctgcta ctgctgctcc tgctgcagcc accgcctccg 120
acctgggcgc tcagcccccg gatcagcctg cctctgggct ctgaagagcg gccattcctc 180
agattcgaag ctgaacacat ctccaactac acagcccttc tgctgagcag ggatggcagg 240
accctgtacg tgggtgctcg agaggccctc tttgcactca gtagcaacct cagcttcctg 300
ccaggcgggg agtaccagga gctgctttgg ggtgcagacg cagagaagaa acagcagtgc 360
agcttcaagg gcaaggaccc acagcgcgac tgtcaaaact acatcaagat cctcctgccg 420
ctcagcggca gtcacctgtt cacctgtggc acagcagcct tcagccccat gtgtacctac 480
atcaacatgg agaacttcac cctggcaagg gacgagaagg ggaatgtcct cctggaagat 540
ggcaagggcc gttgtccctt cgacccgaat ttcaagtcca ctgccctggt ggttgatggc 600
gagctctaca ctggaacagt catcagcttc caagggaatg acccggccat ctcgcggagc 660
caaagccttc gccccaccaa gaccgagagc tccctcaact ggctgcaaga cccagctttt 720
gtggcctcag cctacattcc tgagagcctg ggcagcttgc aaggcgatga tgacaagatc 780
tactttttct tcagcgagac tggccaggaa tttgagttct ttgagaacac cattgtgtcc 840
cgcattgccc gcatctgcaa gggcgatgag ggtggagagc gggtgctaca gcagcgctgg 900
acctccttcc tcaaggccca gctgctgtgc tcacggcccg acgatggctt ccccttcaac 960
gtgctgcagg atgtcttcac gctgagcccc agcccccagg actggcgtga cacccttttc 1020
tatggggtct tcacttccca gtggcacagg ggaactacag aaggctctgc cgtctgtgtc 1080
ttcacaatga aggatgtgca gagagtcttc agcggcctct acaaggaggt gaaccgtgag 1140
acacagcaga tggtacaccg tgacccaccc gtgcccacac cccggcctgg agcgtgcatc 1200
accaacagtg cccgggaaag gaagatcaac tcatccctgc agctcccaga ccgcgtgctg 1260
aactttctca aggaccactt cctgatggac gggcaggtcc gaagccgcat gctgctgctg 1320
cagccccagg ctcgctacca gcgcgtggct gtacaccgcg tccctggcct gcaccacacc 1380
tacgatgtcc tcttcctggg cactggtgac ggccggctcc acaaggcagt gagcgtgggc 1440
ccccgggtgc acatcattga ggagctgcag atcttctcat cgggacagcc cgtgcagaat 1500
ctgctcctgg acacccacag ggggctgctg tatgcggcct cacactcggg cgtagtccag 1560
gtgcccatgg ccaactgcag cctgtaccgg agctgtgggg actgcctcct cgcccgggac 1620
ccctactgtg cttggagcgg ctccagctgc aagcacgtca gcctctacca gcctcagctg 1680
gccaccaggc cgtggatcca ggacatcgag ggagccagcg ccaaggacct ttgcagcgcg 1740
tcttcggttg tgtccccgtc ttttgtacca acaggggaga agccatgtga gcaagtccag 1800
ttccagccca acacagtgaa cactttggcc tgcccgctcc tctccaacct ggcgacccga 1860
ctctggctac gcaacggggc ccccgtcaat gcctcggcct cctgccacgt gctacccact 1920
ggggacctgc tgctggtggg cacccaacag ctgggggagt tccagtgctg gtcactagag 1980
gagggcttcc agcagctggt agccagctac tgcccagagg tggtggagga cggggtggca 2040
gaccaaacag atgagggtgg cagtgtaccc gtcattatca gcacatcgcg tgtgagtgca 2100
ccagctggtg gcaaggccag ctggggtgca gacaggtcct actggaagga gttcctggtg 2160
atgtgcacgc tctttgtgct ggccgtgctg ctcccagttt tattcttgct ctaccggcac 2220
cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccactcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 6
<211> 3766
<212> DNA
<213> Homo sapiens

<400> 6
gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctactg ctgctcctgc tgcagccacc gcctccgacc 360
tgggcgctca gcccccggat cagcctgcct ctgggctctg aagagcggcc attcctcaga 420

ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatggaga acttcaccct ggcaagggac gagaagggga atgtcctcct ggaagatggc 780
aagggccgtt gtcccttcga cccgaatttc aagtccactg ccctggtggt tgatggcgag 840
ctctacactg gaacagtcat cagcttccaa gggaatgacc cggccatctc gcggagccaa 900
agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080
attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc ccccaggact ggcgtgacac ccttttctat 1260
ggggtcttca cttcccagtg gcacagggga actacagaag gctctgccgt ctgtgtcttc 1320
acaatgaagg atgtgcagag agtcttcagc ggcctctaca aggaggtgaa ccgtgagaca 1380
cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
tttctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtaccggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg gggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcacccca 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300
ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540
ccaccatatc caccctcgct ccatctttga actcaaacac gaggaactaa ctgcaccctg 3600
gtcctctccc cagtccccag ttcaccctcc atccctcacc ttcctccact ctaaggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 7
<211> 837
<212> PRT

<213> Homo sapiens

<400> 7
Met Leu Arg Thr Ala Met Gly Leu Arg Ser Trp Leu Ala Ala Pro Trp
                5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Ile Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Lys Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu

450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 8
<211> 2511
<212> DNA
<213> Homo sapiens

<400> 8
atgctgcgca ccgcgatggg cctgaggagc tggctcgccg ccccatgggg cgcgctgccg      60
cctcggccac cgctgctgct gctcctgctg ctgctgctcc tgctgcagcc gccgcctccg     120
acctgggcgc tcagcccccg gatcagccta cctctgggct ctgaagagcg gccattcctc     180
agattcgaag ctgaacacat ctccaactac acagcccttc tgctgagcag ggatggcagg     240

accctgtacg tgggtgctcg agaggccctc tttgcactca gtagcaacct cagcttcctg 300
ccaggcgggg agtaccagga gctgctttgg ggtgcagacg cagagaagaa acagcagtgc 360
agcttcaagg gcaaggaccc acagcgcgac tgtcaaaact acatcaagat cctcctgccg 420
ctcagcggca gtcacctgtt cacctgtggc acagcagcct tcagccccat gtgtacctac 480
atcaacatag agaacttcac cctggcaagg gacgagaagg ggaatgttct cctggaagat 540
ggcaagggcc gttgtccctt cgacccgaat ttcaagtcca ctgccctggt ggttgatggc 600
gagctctaca ctggaacagt cagcagcttc caagggaatg acccggccat ctcgcggagc 660
caaagccttc gccccaccaa gaccgagagc tccctcaact ggctgcaaga cccagctttt 720
gtggcctcag cctacattcc tgagagcctg ggcagcttgc aaggcgatga tgacaagatc 780
tactttttct tcagcgagac tggccaggaa tttgagttct ttgagaacac cattgtgtcc 840
cgcattgccc gcatctgcaa gggcgatgag ggtggagagc gggtgctaca gcagcgctgg 900
acctccttcc tcaaggccca gctgctgtgc tcacggcccg acgatggctt ccccttcaac 960
gtgctgcagg atgtcttcac gctgagcccc agcccccagg actggcgtga cacccttttc 1020
tatggggtct tcacttccca gtggcacagg ggaactacag aaggctctgc cgtctgtgtc 1080
ttcacaatga aggatgtgca gagagtcttc agcggcctct acaaggaggt gaaccgtgag 1140
acacagcaga tggtacaccg tgacccaccc gtgcccacac cccggcctgg agcgtgcatc 1200
accaacagtg cccgggaaag gaagatcaac tcatccctgc agctcccaga ccgcgtgctg 1260
aacttcctca aggaccactt cctgatggac gggcaggtcc gaagccgcat gctgctgctg 1320
cagccccagg ctcgctacca gcgcgtggct gtacaccgcg tccctggcct gcaccacacc 1380
tacgatgtcc tcttcctggg cactggtgac ggccggctcc acaaggcagt gagcgtgggc 1440
ccccgggtgc acatcattga ggagctgcag atcttctcat cgggacagcc cgtgcagaat 1500
ctgctcctgg acacccacag ggggctgctg tatgcggcct cacactcggg cgtagtccag 1560
gtgcccatgg ccaactgcag cctgtacagg agctgtgggg actgcctcct cgcccgggac 1620
ccctactgtg cttggagcgg ctccagctgc aagcacgtca gcctctacca gcctcagctg 1680
gccaccaggc cgtggatcca ggacatcgag ggagccagcg ccaaggacct ttgcagcgcg 1740
tcttcggttg tgtccccgtc ttttgtacca acaggggaga agccatgtga gcaagtccag 1800
ttccagccca acacagtgaa cactttggcc tgcccgctcc tctccaacct ggcgacccga 1860
ctctggctac gcaacggggc ccccgtcaat gcctcggcct cctgccacgt gctacccact 1920
ggggacctgc tgctggtggg cacccaacag ctgggggagt tccagtgctg gtcactagag 1980
gagggcttcc agcagctggt agccagctac tgcccagagg tggtggagga cggggtggca 2040
gaccaaacag atgagggtgg cagtgtaccc gtcattatca gcacatcgcg tgtgagtgca 2100
ccagctggtg gcaaggccag ctggggtgca gacaggtcct actggaagga gttcctggtg 2160
atgtgcacgc tctttgtgct ggccgtgctg ctcccagttt tattcttgct ctaccggcac 2220
cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccgctcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 9
<211> 3766
<212> DNA
<213> Homo sapiens

<400> 9
gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctgctg ctgctcctgc tgcagccgcc gcctccgacc 360
tgggcgctca gcccccggat cagcctacct ctgggctctg aagagcggcc attcctcaga 420
ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatagaga acttcaccct ggcaagggac gagaagggga atgttctcct ggaagatggc 780
aagggccgtt gtcccttcga cccgaatttc aagtccactg ccctggtggt tgatggcgag 840
ctctacactg gaacagtcag cagcttccaa gggaatgacc cggccatctc gcggagccaa 900

agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080
attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc ccccaggact ggcgtgacac ccttttctat 1260
ggggtcttca cttcccagtg gcacagggga actacagaag gctctgccgt ctgtgtcttc 1320
acaatgaagg atgtgcagag agtcttcagc ggcctctaca aggaggtgaa ccgtgagaca 1380
cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
ttcctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtacaggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg ggggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcaccccg 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300
ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540
ccaccatatc caccctcgct ccatctttga actcaaacac gaggaactaa ctgcaccctg 3600
gtcctctccc cagtccccag ttcaccctcc atccctcacc ttcctccact ctaagggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 10
<211> 837
<212> PRT
<213> Homo sapiens

<400> 10
Met Leu Arg Thr Ala Met Gly Leu Arg Ser Trp Leu Ala Ala Pro Trp
1               5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile

        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Met Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Asn Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu

        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 11
<211> 2511
<212> DNA
<213> Homo sapiens

<400> 11
   atgctgcgca ccgcgatggg cctgaggagc tggctcgccg ccccatgggg cgcgctgccg 60
   cctcggccac cgctgctgct gctcctgctg ctgctgctcc tgctgcagcc gccgcctccg 120
   acctgggcgc tcagcccccg gatcagcctg cctctgggct ctgaagagcg gccattcctc 180
   agattcgaag ctgaacacat ctccaactac acagcccttc tgctgagcag ggatggcagg 240
   accctgtacg tgggtgctcg agaggccctc tttgcactca gtagcaacct cagcttcctg 300
   ccaggcgggg agtaccagga gctgctttgg ggtgcagacg cagagaagaa acagcagtgc 360
   agcttcaagg gcaaggaccc acagcgcgac tgtcaaaact acatcaagat cctcctgccg 420
   ctcagcggca gtcacctgtt cacctgtggc acagcagcct tcagccccat gtgtacctac 480
   atcaacatgg agaacttcac cctggcaagg gacgagaagg ggaatgtcct cctggaagat 540
   ggcaagggcc gttgtccctt cgacccgaat ttcaagtcca ctgccctggt ggttgatggc 600
   gagctctaca ctggaacagt cagcagcttc caaggaatgg acccggccat ctcgcggagc 660
   caaagccttc gccccaccaa gaccgagagc tccctcaact ggctgcaaga cccagctttt 720
   gtggcctcag cctacattcc tgagagcctg ggcagcttgc aaggcgatga tgacaagatc 780
   tactttttct tcagcgagac tggccaggaa tttgagttct ttgagaacac cattgtgtcc 840
   cgcattgccc gcatctgcaa gggcgatgag ggtggagagc gggtgctaca gcagcgctgg 900
   acctccttcc tcaaggccca gctgctgtgc tcacggcccg acgatggctt ccccttcaac 960
   gtgctgcagg atgtcttcac gctgagcccc agcccccagg actggcgtga cacccttttc 1020
   tatggggtct tcacttccca gtggcacagg ggaactacag aaggctctgc cgtctgtgtc 1080
   ttcacaatga atgatgtgca gagagtcttc agcggcctct acaaggaggt gaaccgtgag 1140
   acacagcaga tggtacaccg tgacccaccc gtgcccacac cccggcctgg agcgtgcatc 1200
   accaacagtg cccgggaaag gaagatcaac tcatccctgc agctcccaga ccgcgtgctg 1260
   aactttctca aggaccactt cctgatggac gggcaggtcc gaagccgcat gctgctgctg 1320
   cagccccagg ctcgctacca gcgcgtggct gtacaccgcg tccctggcct gcaccacacc 1380
   tacgatgtcc tcttcctggg cactggtgac ggccggctcc acaaggcagt gagcgtgggc 1440
   ccccgggtgc acatcattga ggagctgcag atcttctcat cgggacagcc cgtgcagaat 1500
   ctgctcctgg acacccacag ggggctgctg tatgcggcct cacactcggg cgtagtccag 1560
   gtgcccatgg ccaactgcag cctgtaccgg agctgtgggg actgcctcct cgcccgggac 1620
   ccctactgtg cttggagcgg ctccagctgc aagcacgtca gcctctacca gcctcagctg 1680
   gccaccaggc cgtggatcca ggacatcgag ggagccagcg ccaaggacct ttgcagcgcg 1740
   tcttcggttg tgtccccgtc ttttgtacca acaggggaga agccatgtga gcaagtccag 1800
   ttccagccca acacagtgaa cactttggcc tgcccgctcc tctccaacct ggcgacccga 1860
   ctctggctac gcaacggggc ccccgtcaat gcctcggcct cctgccacgt gctacccact 1920
   ggggacctgc tgctggtggg cacccaacag ctgggggagt tccagtgctg gtcactagag 1980
   gagggcttcc agcagctggt agccagctac tgcccagagg tggtggagga cggggtggca 2040
   gaccaaacag atgagggtgg cagtgtaccc gtcattatca gcacatcgcg tgtgagtgca 2100
   ccagctggtg gcaaggccag ctggggtgca gacaggtcct actggaagga gttcctggtg 2160
   atgtgcacgc tctttgtgct ggccgtgctg ctcccagttt tattcttgct ctaccggcac 2220
   cggaacagca tgaaagtctt cctgaagcag ggggaatgtg ccagcgtgca ccccaagacc 2280
   tgccctgtgg tgctgccccc tgagacccgc ccactcaacg gcctagggcc ccctagcacc 2340
   ccactcgatc accgagggta ccagtccctg tcagacagcc ccccggggtc ccgagtcttc 2400
   actgagtcag agaagaggcc actcagcatc caagacagct tcgtggaggt atccccagtg 2460
   tgcccccggc cccgggtccg ccttggctcg gagatccgtg actctgtggt g 2511

<210> 12
<211> 3766
<212> DNA
<213> Homo sapiens

<400> 12
gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctgctg ctgctcctgc tgcagccgcc gcctccgacc 360
tgggcgctca gcccccggat cagcctgcct ctgggctctg aagagcggcc attcctcaga 420
ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatggaga acttcaccct ggcaagggac gagaagggga atgtcctcct ggaagatggc 780
aagggccgtt gtcccttcga cccgaatttc aagtccactg ccctggtggt tgatggcgag 840
ctctacactg gaacagtcag cagcttccaa gggaatgacc cggccatctc gcggagccaa 900
agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080
attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc ccccaggact ggcgtgacac ccttttctat 1260
ggggtcttca cttcccagtg gcacagggga actacagaag gctctgccgt ctgtgtcttc 1320
acaatgaatg atgtgcagag agtcttcagc ggcctctaca aggaggtgaa ccgtgagaca 1380

cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
tttctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtaccggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg ggggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcacccca 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300
ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540
ccaccatatc caccctcgct ccatctttga actcaaacac gaggaactaa ctgcaccctg 3600
gtcctctccc cagtccccag ttcaccctcc atccctcacc ttcctccact ctaagggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 13
<211> 20
<212> DNA
<213> Artificial Sequence

<220>
<223> Oligonucleotide

<400> 13
cagtgccaac ctagccctct 20

<210> 14
<211> 20
<212> DNA
<213> Artificial Sequence

<220>
<223> Oligonucleotide

<400> 14
tctcccgatc caaccgtgac                                                    20

<210> 15
<211> 20
<212> DNA
<213> Artificial Sequence

<220>
<223> Oligonucleotide

<400> 15
caacaactac atcctcggct                                                    20

<210> 16
<211> 20
<212> DNA
<213> Artificial Sequence

<220>
<223> Oligonucleotide

<400> 16
tcggctccta catcaacaac                                                    20

<210> 17
<211> 24
<212> DNA
<213> Artificial Sequence

<220>
<223> Primer

<400> 17
cctcgcccgg gacccctact gtgc                                               24

<210> 18
<211> 27
<212> DNA
<213> Artificial Sequence

<220>
<223> Primer

<400> 18
cttggcgctg gctccctcga tgtcctg                                            27

<210> 19
<211> 28
<212> DNA
<213> Artificial Sequence

<220>
<223> Primer

<400> 19
aattgaattc atgctgcgca ccgcgatg                                           28

<210> 20
<211> 30

<212> DNA
<213> Artificial Sequence

<220>
<223> Primer

<400> 20
aagctctaga caccacagag tcacggatct                                        30

<210> 21
<211> 30
<212> DNA
<213> Artificial Sequence

<220>
<223> Primer

<400> 21
aagctctaga tcacaccaca gagtcacgga                                        30

<210> 22
<211> 12
<212> PRT
<213> Homo sapiens

<400> 22
Asn Ser Ala Arg Glu Arg Lys Ile Asn Ser Ser Cys
                5                   10

<210> 23
<211> 15
<212> PRT
<213> Homo sapiens

<400> 23
Ser Val Val Ser Pro Ser Phe Val Pro Thr Gly Glu Lys Pro Cys
                5                   10                  15

<210> 24
<211> 15
<212> PRT
<213> Homo sapiens

<400> 24
Pro Leu Asp His Arg Gly Tyr Gln Ser Leu Ser Asp Ser Pro Cys
                5                   10                  15

<210> 25
<211> 14
<212> PRT
<213> Homo sapiens

<400> 25
Ser Arg Val Phe Thr Glu Ser Glu Lys Arg Pro Leu Ser Cys
                5                   10

<210> 26
<211> 2135
<212> PRT
<213> Homo sapiens

<400> 26
Met Pro Ala Leu Gly Pro Ala Leu Leu Gln Ala Leu Trp Ala Gly Trp
                5                   10                  15
Val Leu Thr Leu Gln Pro Leu Pro Pro Thr Ala Phe Thr Pro Asn Gly
            20                  25                  30
Thr Tyr Leu Gln His Leu Ala Arg Asp Pro Thr Ser Gly Thr Leu Tyr
        35                  40                  45
Leu Gly Ala Thr Asn Phe Leu Phe Gln Leu Ser Pro Gly Leu Gln Leu
    50                  55                  60
Glu Ala Thr Val Ser Thr Gly Pro Val Leu Asp Ser Arg Asp Cys Leu
65                  70                  75                  80
Pro Pro Val Met Pro Asp Glu Cys Pro Gln Ala Gln Pro Thr Asn Asn
                85                  90                  95
Pro Asn Gln Leu Leu Leu Val Ser Pro Gly Ala Leu Val Val Cys Gly
            100                 105                 110
Ser Val His Gln Gly Val Cys Glu Gln Arg Arg Leu Gly Gln Leu Glu
        115                 120                 125
Gln Leu Leu Leu Arg Pro Glu Arg Pro Gly Asp Thr Gln Tyr Val Ala
    130                 135                 140
Ala Asn Asp Pro Ala Val Ser Thr Val Gly Leu Val Ala Gln Gly Leu
145                 150                 155                 160
Ala Gly Glu Pro Leu Leu Phe Val Gly Arg Gly Tyr Thr Ser Arg Gly
                165                 170                 175
Val Gly Gly Gly Ile Pro Pro Ile Thr Thr Arg Ala Leu Trp Pro Pro
            180                 185                 190
Asp Pro Gln Ala Ala Phe Ser Tyr Glu Glu Thr Ala Lys Leu Ala Val
        195                 200                 205
Gly Arg Leu Ser Glu Tyr Ser His His Phe Val Ser Ala Phe Ala Arg
    210                 215                 220
Gly Ala Ser Ala Tyr Phe Leu Phe Leu Arg Arg Asp Leu Gln Ala Gln
225                 230                 235                 240
Ser Arg Ala Phe Arg Ala Tyr Val Ser Arg Val Cys Leu Arg Asp Gln
                245                 250                 255
His Tyr Tyr Ser Tyr Val Glu Leu Pro Leu Ala Cys Glu Gly Gly Arg
            260                 265                 270
Tyr Gly Leu Ile Gln Ala Ala Ala Val Ala Thr Ser Arg Glu Val Ala
        275                 280                 285
His Gly Glu Val Leu Phe Ala Ala Phe Ser Ser Ala Ala Pro Pro Thr
    290                 295                 300
Val Gly Arg Pro Pro Ser Ala Ala Ala Gly Ala Ser Gly Ala Ser Ala
305                 310                 315                 320
Leu Cys Ala Phe Pro Leu Asp Glu Val Asp Arg Leu Ala Asn Arg Thr
                325                 330                 335
Arg Asp Ala Cys Tyr Thr Arg Glu Gly Arg Ala Glu Asp Gly Thr Glu
            340                 345                 350
Val Ala Tyr Ile Glu Tyr Asp Val Asn Ser Asp Cys Ala Gln Leu Pro
        355                 360                 365
Val Asp Thr Leu Asp Ala Tyr Pro Cys Gly Ser Asp His Thr Pro Ser
    370                 375                 380
Pro Met Ala Ser Arg Val Pro Leu Glu Ala Thr Pro Ile Leu Glu Trp
385                 390                 395                 400
Pro Gly Ile Gln Leu Thr Ala Val Ala Val Thr Met Glu Asp Gly His
                405                 410                 415
Thr Ile Ala Phe Leu Gly Asp Ser Gln Gly Gln Leu His Arg Val Tyr
            420                 425                 430
Leu Gly Pro Gly Ser Asp Gly His Pro Tyr Ser Thr Gln Ser Ile Gln
        435                 440                 445
Gln Gly Ser Ala Val Ser Arg Asp Leu Thr Phe Asp Gly Thr Phe Glu
    450                 455                 460

His Leu Tyr Val Met Thr Gln Ser Thr Leu Leu Lys Val Pro Val Ala
465                 470                 475                 480
Ser Cys Ala Gln His Leu Asp Cys Ala Ser Cys Leu Ala His Arg Asp
                485                 490                 495
Pro Tyr Cys Gly Trp Cys Val Leu Leu Gly Arg Cys Ser Arg Arg Ser
            500                 505                 510
Glu Cys Ser Arg Gly Gln Gly Pro Glu Gln Trp Leu Trp Ser Phe Gln
        515                 520                 525
Pro Glu Leu Gly Cys Leu Gln Val Ala Ala Met Ser Pro Ala Asn Ile
    530                 535                 540
Ser Arg Glu Glu Thr Arg Glu Val Phe Leu Ser Val Pro Asp Leu Pro
545                 550                 555                 560
Pro Leu Trp Pro Gly Glu Ser Tyr Ser Cys His Phe Gly Glu His Gln
                565                 570                 575
Ser Pro Ala Leu Leu Thr Gly Ser Gly Val Met Cys Pro Ser Pro Asp
            580                 585                 590
Pro Ser Glu Ala Pro Val Leu Pro Arg Gly Ala Asp Tyr Val Ser Val
        595                 600                 605
Ser Val Glu Leu Arg Phe Gly Ala Val Val Ile Ala Lys Thr Ser Leu
    610                 615                 620
Ser Phe Tyr Asp Cys Val Ala Val Thr Glu Leu Arg Pro Ser Ala Gln
625                 630                 635                 640
Cys Gln Ala Cys Val Ser Ser Arg Trp Gly Cys Asn Trp Cys Val Trp
                645                 650                 655
Gln His Leu Cys Thr His Lys Ala Ser Cys Asp Ala Gly Pro Met Val
            660                 665                 670
Ala Ser His Gln Ser Pro Leu Val Ser Pro Asp Pro Pro Ala Arg Gly
        675                 680                 685
Gly Pro Ser Pro Ser Pro Pro Thr Ala Pro Lys Ala Leu Ala Thr Pro
    690                 695                 700
Ala Pro Asp Thr Leu Pro Val Glu Pro Gly Ala Pro Ser Thr Ala Thr
705                 710                 715                 720
Ala Ser Asp Ile Ser Pro Gly Ala Ser Pro Ser Leu Leu Ser Pro Trp
                725                 730                 735
Gly Pro Trp Ala Gly Ser Gly Ser Ile Ser Ser Pro Gly Ser Thr Gly
            740                 745                 750
Ser Pro Leu His Glu Glu Pro Ser Pro Pro Ser Pro Gln Asn Gly Pro
        755                 760                 765
Gly Thr Ala Val Pro Ala Pro Thr Asp Phe Arg Pro Ser Ala Thr Pro
    770                 775                 780
Glu Asp Leu Leu Ala Ser Pro Leu Ser Pro Ser Glu Val Ala Ala Val
785                 790                 795                 800
Pro Pro Ala Asp Pro Gly Pro Glu Ala Leu His Pro Thr Val Pro Leu
                805                 810                 815
Asp Leu Pro Pro Ala Thr Val Pro Ala Thr Thr Phe Pro Gly Ala Met
            820                 825                 830
Gly Ser Val Lys Pro Ala Leu Asp Trp Leu Thr Arg Glu Gly Gly Glu
        835                 840                 845
Leu Pro Glu Ala Asp Glu Trp Thr Gly Gly Asp Ala Pro Ala Phe Ser
    850                 855                 860
Thr Ser Thr Leu Leu Ser Gly Asp Gly Asp Ser Ala Glu Leu Glu Gly
865                 870                 875                 880
Pro Pro Ala Pro Leu Ile Leu Pro Ser Ser Leu Asp Tyr Gln Tyr Asp
                885                 890                 895
Thr Pro Gly Leu Trp Glu Leu Glu Glu Ala Thr Leu Gly Ala Ser Ser
            900                 905                 910
Cys Pro Cys Val Glu Ser Val Gln Gly Ser Thr Leu Met Pro Val His
        915                 920                 925
Val Glu Arg Glu Ile Arg Leu Leu Gly Arg Asn Leu His Leu Phe Gln
    930                 935                 940

Asp Gly Pro Gly Asp Asn Glu Cys Val Met Glu Leu Glu Gly Leu Glu
945                 950                 955                 960
Val Val Val Glu Ala Arg Val Glu Cys Glu Pro Pro Pro Asp Thr Gln
                965                 970                 975
Cys His Val Thr Cys Gln Gln His Gln Leu Ser Tyr Glu Ala Leu Gln
            980                 985                 990
Pro Glu Leu Arg Val Gly Leu Phe Leu Arg Arg Ala Gly Arg Leu Arg
        995                 1000                1005
Val Asp Ser Ala Glu Gly Leu His Val Val Leu Tyr Asp Cys Ser Val
    1010                1015                1020
Gly His Gly Asp Cys Ser Arg Cys Gln Thr Ala Met Pro Gln Tyr Gly
1025                1030                1035                1040
Cys Val Trp Cys Glu Gly Glu Arg Pro Arg Cys Val Thr Arg Glu Ala
                1045                1050                1055
Cys Gly Glu Ala Glu Ala Val Ala Thr Gln Cys Pro Ala Pro Leu Ile
            1060                1065                1070
His Ser Val Glu Pro Leu Thr Gly Pro Val Asp Gly Gly Thr Arg Val
        1075                1080                1085
Thr Ile Arg Gly Ser Asn Leu Gly Gln His Val Gln Asp Val Leu Gly
    1090                1095                1100
Met Val Thr Val Ala Gly Val Pro Cys Ala Val Asp Ala Gln Glu Tyr
1105                1110                1115                1120
Glu Val Ser Ser Ser Leu Val Cys Ile Thr Gly Ala Ser Gly Glu Glu
                1125                1130                1135
Val Ala Gly Ala Thr Ala Val Glu Val Pro Gly Arg Gly Arg Gly Val
            1140                1145                1150
Ser Glu His Asp Phe Ala Tyr Gln Asp Pro Lys Val His Ser Ile Phe
        1155                1160                1165
Pro Ala Arg Gly Pro Arg Ala Gly Gly Thr Arg Leu Thr Leu Asn Gly
    1170                1175                1180
Ser Lys Leu Leu Thr Gly Arg Leu Glu Asp Ile Arg Val Val Val Gly
1185                1190                1195                1200
Asp Gln Pro Cys His Leu Leu Pro Glu Gln Gln Ser Glu Gln Leu Arg
                1205                1210                1215
Cys Glu Thr Ser Pro Arg Pro Thr Pro Ala Thr Leu Pro Val Ala Val
            1220                1225                1230
Trp Phe Gly Ala Thr Glu Arg Arg Leu Gln Arg Gly Gln Phe Lys Tyr
        1235                1240                1245
Thr Leu Asp Pro Asn Ile Thr Ser Ala Gly Pro Thr Lys Ser Phe Leu
    1250                1255                1260
Ser Gly Gly Arg Glu Ile Cys Val Arg Gly Gln Asn Leu Asp Val Val
1265                1270                1275                1280
Gln Thr Pro Arg Ile Arg Val Thr Val Val Ser Arg Met Leu Gln Pro
                1285                1290                1295
Ser Gln Gly Leu Gly Arg Arg Arg Arg Val Val Pro Glu Thr Ala Cys
            1300                1305                1310
Ser Leu Gly Pro Ser Cys Ser Ser Gln Gln Phe Glu Glu Pro Cys His
        1315                1320                1325
Val Asn Ser Ser Gln Leu Ile Thr Cys Arg Thr Pro Ala Leu Pro Gly
    1330                1335                1340
Leu Pro Glu Asp Pro Trp Val Arg Val Glu Phe Ile Leu Asp Asn Leu
1345                1350                1355                1360
Val Phe Asp Phe Ala Thr Leu Asn Pro Thr Pro Phe Ser Tyr Glu Ala
                1365                1370                1375
Asp Pro Thr Leu Gln Pro Leu Asn Pro Glu Asp Pro Thr Met Pro Phe
            1380                1385                1390
Arg His Lys Pro Gly Ser Val Phe Ser Val Glu Gly Glu Asn Leu Asp
        1395                1400                1405
Leu Ala Met Ser Lys Glu Glu Val Val Ala Met Ile Gly Asp Gly Pro
    1410                1415                1420

Cys Val Val Lys Thr Leu Thr Arg His His Leu Tyr Cys Glu Pro Pro
1425                1430                1435                1440
Val Glu Gln Pro Leu Pro Arg His His Ala Leu Arg Glu Ala Pro Asp
                1445                1450                1455
Ser Leu Pro Glu Phe Thr Val Gln Met Gly Asn Leu Arg Phe Ser Leu
            1460                1465                1470
Gly His Val Gln Tyr Asp Gly Glu Ser Pro Gly Ala Phe Pro Val Ala
        1475                1480                1485
Ala Gln Val Gly Leu Gly Val Gly Thr Ser Leu Leu Ala Leu Gly Val
    1490                1495                1500
Ile Ile Ile Val Leu Met Tyr Arg Arg Lys Ser Lys Gln Ala Leu Arg
1505                1510                1515                1520
Asp Tyr Lys Lys Val Gln Ile Gln Leu Glu Asn Leu Glu Ser Ser Val
                1525                1530                1535
Arg Asp Arg Cys Lys Lys Glu Phe Thr Asp Leu Met Thr Glu Met Thr
            1540                1545                1550
Asp Leu Thr Ser Asp Leu Leu Gly Ser Gly Ile Pro Phe Leu Asp Tyr
        1555                1560                1565
Lys Val Tyr Ala Glu Arg Ile Phe Phe Pro Gly His Arg Glu Ser Pro
    1570                1575                1580
Leu His Arg Asp Leu Gly Val Pro Glu Ser Arg Arg Pro Thr Val Glu
1585                1590                1595                1600
Gln Gly Leu Gly Gln Leu Ser Asn Leu Leu Asn Ser Lys Leu Phe Leu
                1605                1610                1615
Thr Lys Phe Ile His Thr Leu Glu Ser Gln Arg Thr Phe Ser Ala Arg
            1620                1625                1630
Asp Arg Ala Tyr Val Ala Ser Leu Leu Thr Val Ala Leu His Gly Lys
        1635                1640                1645
Leu Glu Tyr Phe Thr Asp Ile Leu Arg Thr Leu Leu Ser Asp Leu Val
    1650                1655                1660
Ala Gln Tyr Val Ala Lys Asn Pro Lys Leu Met Leu Arg Arg Thr Glu
1665                1670                1675                1680
Thr Val Val Glu Lys Leu Leu Thr Asn Trp Met Ser Ile Cys Leu Tyr
                1685                1690                1695
Thr Phe Val Arg Asp Ser Val Gly Glu Pro Leu Tyr Met Leu Phe Arg
            1700                1705                1710
Gly Ile Lys His Gln Val Asp Lys Gly Pro Val Asp Ser Val Thr Gly
        1715                1720                1725
Lys Ala Lys Tyr Thr Leu Asn Asp Asn Arg Leu Leu Arg Glu Asp Val
    1730                1735                1740
Glu Tyr Arg Pro Leu Thr Leu Asn Ala Leu Leu Ala Val Gly Pro Gly
1745                1750                1755                1760
Ala Gly Glu Ala Gln Gly Val Pro Val Lys Val Leu Asp Cys Asp Thr
                1765                1770                1775
Ile Ser Gln Ala Lys Glu Lys Met Leu Asp Gln Leu Tyr Lys Gly Val
            1780                1785                1790
Pro Leu Thr Gln Arg Pro Asp Pro Arg Thr Leu Asp Val Glu Trp Arg
        1795                1800                1805
Ser Gly Val Ala Gly His Leu Ile Leu Ser Asp Glu Asp Val Thr Ser
    1810                1815                1820
Glu Val Gln Gly Leu Trp Arg Arg Leu Asn Thr Leu Gln His Tyr Lys
1825                1830                1835                1840
Val Pro Asp Gly Ala Thr Val Ala Leu Val Pro Cys Leu Thr Lys His
                1845                1850                1855
Val Leu Arg Glu Asn Gln Asp Tyr Val Pro Gly Glu Arg Thr Pro Met
            1860                1865                1870
Leu Glu Asp Val Asp Glu Gly Gly Ile Arg Pro Trp His Leu Val Lys
        1875                1880                1885
Pro Ser Asp Glu Pro Glu Pro Pro Arg Pro Arg Arg Gly Ser Leu Arg
    1890                1895                1900

Gly Gly Glu Arg Glu Arg Ala Lys Ala Ile Pro Glu Ile Tyr Leu Thr
1905                1910                1915                1920
Arg Leu Leu Ser Met Lys Gly Thr Leu Gln Lys Phe Val Asp Asp Leu
                1925                1930                1935
Phe Gln Val Ile Leu Ser Thr Ser Arg Pro Val Pro Leu Ala Val Lys
            1940                1945                1950
Tyr Phe Phe Asp Leu Leu Asp Glu Gln Ala Gln Gln His Gly Ile Ser
        1955                1960                1965
Asp Gln Asp Thr Ile His Ile Trp Lys Thr Asn Ser Leu Pro Leu Arg
    1970                1975                1980
Phe Trp Ile Asn Ile Ile Lys Asn Pro Gln Phe Val Phe Asp Val Gln
1985                1990                1995                2000
Thr Ser Asp Asn Met Asp Ala Val Leu Leu Val Ile Ala Gln Thr Phe
                2005                2010                2015
Met Asp Ala Cys Thr Leu Ala Asp His Lys Leu Gly Arg Asp Ser Pro
            2020                2025                2030
Ile Asn Lys Leu Leu Tyr Ala Arg Asp Ile Pro Arg Tyr Lys Arg Met
        2035                2040                2045
Val Glu Arg Tyr Tyr Ala Asp Ile Arg Gln Thr Val Pro Ala Ser Asp
    2050                2055                2060
Gln Glu Met Asn Ser Val Leu Ala Glu Leu Ser Trp Asn Tyr Ser Gly
2065                2070                2075                2080
Asp Leu Gly Ala Arg Val Ala Leu His Glu Leu Tyr Lys Tyr Ile Asn
                2085                2090                2095
Lys Tyr Tyr Asp Gln Ile Ile Thr Ala Leu Glu Glu Asp Gly Thr Ala
            2100                2105                2110
Gln Lys Met Gln Leu Gly Tyr Arg Leu Gln Gln Ile Ala Ala Ala Val
        2115                2120                2125
Glu Asn Lys Val Thr Asp Leu
    2130                2135

<210> 27
<211> 28
<212> DNA
<213> Artificial Sequence

<220>
<223> Primer

<400> 27
gcaatgccac agtcctatcc cttaccag                                          28

<210> 28
<211> 22
<212> DNA
<213> Artificial Sequence

<220>
<223> Primer

<400> 28
ggccggcccg acgcagaaac ag                                                22

<210> 29
<211> 29
<212> DNA
<213> Artificial Sequence

<220>
<223> Primer

<400> 29
acatcagccg agaggagacg agggaggtt                                          29

<210> 30
<211> 23
<212> DNA
<213> Artificial Sequence

<220>
<223> Primer

<400> 30
acggggcggc tggtgctgag aat                                                23

<210> 31
<211> 27
<212> DNA
<213> Artificial Sequence

<220>
<223> Primer

<400> 31
cggctagcga agcctggtcc tcctctg                                            27

<210> 32
<211> 28
<212> DNA
<213> Artificial Sequence

<220>
<223> Primer

<400> 32
cggatccgcg ctctcgccgt catactgc                                           28

<210> 33
<211> 30
<212> DNA
<213> Artificial Sequence

<220>
<223> Primer

<400> 33
cgggatccca aatcttgtga cacacctccc                                         30

<210> 34
<211> 30
<212> DNA
<213> Artificial Sequence

<220>
<223> Primer

<400> 34
caagctttca tttacccgga gacagggaga                                         30

<210> 35

<211> 7308
<212> DNA
<213> Homo sapiens

<400> 35
gcccggccga cgcggctttg tctcctttgt tcccggcggt ggcagcgccg cgcgggaggg 60
gcgggcagcg ggcgcagttt tccgcccctc ggtctccggg taacagctgc ggctccacca 120
gacccgggga gaggccgctg cgcgcggagc ccgagcccgg agcggccgac gcccgcctcg 180
gcgcgcacat cccgcggggc ccggccgggt ggtgactccc acacgggtca tgctgttgtc 240
tcctgatcca gccggccctg ccaggtgacc atgcctgctc tgggcccagc tcttctccag 300
gctctctggg ccgggtgggt cctcaccctc cagccccttc caccaactgc attcactccc 360
aatggcacgt atctgcagca cctggcaagg gaccccacct caggcaccct ctacctgggg 420
gctaccaact tcctgttcca gctgagccct gggctgcagc tggaggccac agtgtccacc 480
ggccctgtgc tagacagcag ggactgcctg ccacctgtga tgcctgatga gtgcccccag 540
gcccagccta ccaacaaccc gaatcagctg ctcctggtga gcccaggggc cctggtggta 600
tgcgggagcg tgcaccaggg ggtctgtgaa cagcggcgcc tggggcagct cgagcagctg 660
ctgctgcggc cagagcggcc tggggacaca caatatgtgg ctgccaatga tcctgcggtc 720
agcacggtgg ggctggtagc ccagggcttg gcaggggagc ccctcctgtt tgtggggcga 780
ggatacacca gcaggggtgt ggggggtggc attccaccca tcacaacccg ggccctgtgg 840
ccgcccgacc cccaagctgc cttctcctat gaggagacag ccaagctggc agtgggccgc 900
ctctccgagt acagccacca cttcgtgagt gcctttgcac gtggggccag cgcctacttc 960
ctgttcctgc ggcgggacct gcaggctcag tctagagctt ttcgtgccta tgtatctcga 1020
gtgtgtctcc gggaccagca ctactactcc tatgtggagt tgcctctggc ctgcgaaggt 1080
ggccgctacg ggctgatcca ggctgcagct gtggccacgt ccagggaggt ggcgcatggg 1140
gaggtgctct ttgcagcttt ctcctcggct gcacccccca ctgtgggccg gcccccatcg 1200
gcggctgctg gggcatctgg agcctctgcc ctctgtgcct tccccctgga tgaggtggac 1260
cggcttgcta atcgcacgcg agatgcctgc tacacccggg agggtcgtgc tgaggatggg 1320
accgaggtgg cctacatcga gtatgatgtc aattctgact gtgcacagct gccagtggac 1380
accctggatg cttatccctg tggctcagac cacacgccca gccccatggc cagccgggtc 1440
ccgctggaag ccacaccaat tctggagtgg ccagggattc agctaacagc tgtggcagtc 1500
accatggaag atggacacac catcgctttc ctgggtgata gtcaagggca gctgcacagg 1560
gtctacttgg gcccagggag cgatggccac ccatactcca cacagagcat ccagcagggg 1620
tctgcagtga gcagagacct cacctttgat gggacctttg agcacctgta tgtcatgacc 1680
cagagcacac ttctgaaggt tcctgtggct tcctgtgctc agcacctgga ctgtgcatct 1740
tgccttgctc acagggaccc atactgtggg tggtgcgtgc tccttggcag gtgcagtcgc 1800
cgttctgagt gctcgagggg ccagggccca gagcagtggc tatggagctt ccagcctgag 1860
ctgggctgtc tgcaagtggc agccatgagt cctgccaaca tcagccgaga ggagacgagg 1920
gaggttttcc tatcagtgcc agacctgcca cccctgtggc caggggagtc atattcctgc 1980
cactttgggg aacatcagag tcctgccctg ctgactggtt ctggtgtgat gtgcccctcc 2040
ccagacccta gtgaggcccc agtgctgccg agaggagccg actacgtatc cgtgagcgtg 2100
gagctcagat ttggcgctgt tgtgatcgcc aaaacttccc tctctttcta tgactgtgtg 2160
gcggtcactg aactccgccc atctgcgcag tgccaggcct gtgtgagcag ccgctggggg 2220
tgtaactggt gtgtctggca gcacctgtgc acccacaagg cctcgtgtga tgctgggccc 2280
atggttgcaa gccatcagag cccgcttgtc tccccagacc ctcctgcaag aggtggaccc 2340
agcccctccc cacccacagc ccccaaagcc ctggccaccc ctgctcctga cacccttccc 2400
gtggagcctg gggctccctc cacagccaca gcttcggaca tctcacctgg ggctagtcct 2460
tccctgctca gcccctgggg gccatgggca ggttctggct ccatatcttc ccctggctcc 2520
acagggtcgc ctctccatga ggagccctcc cctcccagcc cccaaaatgg acctggaacc 2580
gctgtccctg cccccactga cttcagaccc tcagccacac ctgaggacct cttggcctcc 2640
ccgctgtcac cgtcagaggt agcagcagtg ccccctgcag accctggccc cgaggctctt 2700
catcccacag tgcccctgga cctgccccct gccactgttc ctgccaccac tttcccaggg 2760
gccatgggct ccgtgaagcc cgccctggac tggctcacga gagaaggcgg cgagctgccc 2820
gaggcggacg agtggacggg gggtgacgca cccgccttct ccacttccac cctcctctca 2880
ggtgatggag actcagcaga gcttgagggc cctcccgccc ccctcatcct cccgtccagc 2940
ctcgactacc agtatgacac ccccgggctc tgggagctgg aagaggcgac cttgggggca 3000
agctcctgcc cctgtgtgga gagcgttcag ggctccacgt tgatgccggt ccatgtggag 3060
cgggaaatcc ggctgctagg caggaacctg caccttttcc aggatggccc aggagacaat 3120
gagtgtgtga tggagctgga gggcctcgag gtggtggttg aggcccgggt cgagtgtgag 3180
ccacctccag atacccagtg ccatgtcacc tgccagcagc accagctcag ctatgaggct 3240
ctgcagccgg agctccgtgt ggggctgttt ctgcgtcggg ccggccgtct gcgtgtggac 3300

agtgctgagg ggctgcatgt ggtactgtat gactgttccg tgggacatgg agactgcagc 3360
cgctgccaaa ctgccatgcc ccagtatggc tgtgtgtggt gtgaggggga gcgtccacgt 3420
tgtgtgaccc gggaggcctg tggtgaggct gaggctgtgg ccacccagtg cccagcgccc 3480
ctcatccact cggtggagcc actgactggg cctgtagacg gaggcacccg tgtcaccatc 3540
aggggctcca acctgggcca gcatgtgcag gatgtgctgg gcatggtcac ggtggctgga 3600
gtgccctgtg ctgtggatgc ccaggagtac gaggtctcca gcagcctcgt gtgcatcacc 3660
ggggccagtg gggaggaggt ggccggcgcc acagcggtgg aggtgccggg aagaggacgt 3720
ggtgtctcag aacacgactt tgcctaccag gatccgaagg tccattccat cttcccggcc 3780
cgcggcccca gagctggggg cacccgtctc accctgaatg gctccaagct cctgactggg 3840
cggctggagg acatccgagt ggtggttgga gaccagcctt gtcacttgct gccggagcag 3900
cagtcagaac aactgcggtg tgagaccagc ccacgcccca cgcctgccac gctccctgtg 3960
gctgtgtggt ttggggccac ggagcggagg cttcaacgcg gacagttcaa gtataccttg 4020
gaccccaaca tcacctctgc tggccccacc aagagcttcc tcagtggagg acgtgagata 4080
tgcgtccgtg gccagaatct ggacgtggta cagacgccaa gaatccgggt gaccgtggtc 4140
tcgagaatgc tgcagcccag ccaggggctt ggacggaggc gtcgcgtggt cccggagacg 4200
gcatgttccc ttggaccctc ctgcagtagc cagcaatttg aggagccgtg ccatgtcaac 4260
tcctcccagc tcatcacgtg ccgcacacct gccctcccag gcctgcctga ggacccctgg 4320
gtccgggtgg aatttatcct tgacaacctg gtctttgact ttgcaacact gaaccccaca 4380
cctttctcct atgaggccga ccccaccctg cagccactca accctgagga ccccaccatg 4440
ccattccggc acaagcctgg gagtgtgttc tccgtggagg gggagaacct ggaccttgca 4500
atgtccaagg aggaggtggt ggctatgata ggggatggcc cctgtgtggt gaagacgctg 4560
acgcggcacc acctgtactg cgagcccccc gtggagcagc ccctgccacg gcaccatgcc 4620
ctccgagagg cacctgactc tttgcctgag ttcacggtgc agatggggaa cttgcgcttc 4680
tccctgggtc acgtgcagta tgacggcgag agccctgggg cttttcctgt ggcagcccag 4740
gtgggcttgg gggtgggcac ctctcttctg gctctgggtg tcatcatcat tgtcctcatg 4800
tacaggagga agagcaagca ggccctgagg gactataaga aggttcagat ccagctggag 4860
aatctggaga gcagtgtgcg ggaccgctgc aagaaggaat tcacagacct catgactgag 4920
atgaccgatc tcaccagtga cctcctgggc agcggcatcc ccttcctcga ctacaaggtg 4980
tatgcggaga ggatcttctt ccctgggcac cgcgagtcgc ccttgcaccg ggacctgggt 5040
gtgcctgaga gcagacggcc cactgtggag caagggctgg ggcagctctc taacctgctc 5100
aacagcaagc tcttcctcac caagttcatc cacacgctgg agagccagcg caccttttca 5160
gctcgggacc gtgcctacgt ggcatctctg ctcaccgtgg cactgcatgg gaagcttgag 5220
tatttcactg acatcctccg cactctgctc agtgacctgg ttgcccagta tgtggccaag 5280
aaccccaagc tgatgctgcg caggacagag actgtggtgg agaagctgct caccaactgg 5340
atgtccatct gtctgtatac cttcgtgagg gactccgtag gggagcctct gtacatgctc 5400
tttcgaggga ttaagcacca agtggataag gggccagtgg acagtgtgac aggcaaggcc 5460
aaatacacct tgaacgacaa ccgcctgctc agagaggatg tggagtaccg tcccctgacc 5520
ttgaatgcac tattggctgt ggggcctggg gcaggagagg cccagggcgt gcccgtgaag 5580
gtcctagact gtgacaccat ctcccaggca aaggagaaga tgctggacca gctttataaa 5640
ggagtgcctc tcacccagcg gccagaccct cgcacccttg atgttgagtg gcggtctggg 5700
gtggccgggc acctcattct ttctgacgag gatgtcactt ctgaggtcca gggtctgtgg 5760
aggcgcctga acacactgca gcattacaag gtcccagatg gagcaactgt ggccctcgtc 5820
ccctgcctca ccaagcatgt gctccgggaa aaccaggatt atgtccctgg agagcggacc 5880
ccaatgctgg aggatgtaga tgaggggggc atccggccct ggcacctggt gaagccaagt 5940
gatgagccgg agccgcccag gcctcggagg ggcagccttc ggggcgggga gcgtgagcgc 6000
gccaaggcca tccctgagat ctacctgacc cgcctgctgt ccatgaaggg caccctgcag 6060
aagttcgtgg atgacctgtt ccaggtgatt ctcagcacca gccgccccgt gccgctcgct 6120
gtgaagtact tctttgacct gctggatgag caggcccagc agcatggcat ctccgaccag 6180
gacaccatcc acatctggaa gaccaacagc ttgcctctga ggttctggat caatataata 6240
aaaaacccgc agtttgtgtt cgacgtgcaa acatctgata acatggatgc ggtgctcctt 6300
gtcattgcac agaccttcat ggacgcctgc accctggccg accacaagct gggccgggac 6360
tccccgatca acaaacttct gtatgcacgg gacattcccc ggtacaagcg gatggtggaa 6420
aggtactatg cagacatcag acagactgtc ccagccagcg accaagagat gaactctgtc 6480
ctggctgaac tgtcctggaa ctactccgga gacctcgggg cgcgagtggc cctgcatgaa 6540
ctctacaagt acatcaacaa gtactatgac cagatcatca ctgccctgga ggaggatggc 6600
acggcccaga agatgcagct gggctatcgg ctccagcaga ttgcagctgc tgtggaaaac 6660
aaggtcacag atctatagga acccaggagc cacggcctgc tgttgcttca gcctggcctg 6720
ggcagccctg gaagctcgga ggagaggcca ccttcttagg tgcctgtagt gactgacaag 6780
cagagttagt ggaaggtgac tcccagtctc ctggtggctc tggcctcggc cctgctggat 6840
ccacctccta gacccggggc ctcaaggctc atggggtagt acccagcctg ctccccgagt 6900

ccagcgaccc tgtgacaccg gtctgcaggg agttggggac taagggcttc cagagagtgg 6960
ctggaagaga ctccaggccc ctggggagac tgtactgttc ctgaacactg gccttggcca 7020
cactgggatt cggagaggaa ggaggagagc cccatgcttc ctgtctgcct cctccaccat 7080
ccctgacctc agttgagctg cctctggcct tgttgctgct gccacatcct aggtctaaga 7140
gttgaacgcc tctcctaggc cactacaaac tgacccctca gcagggctgg ctgccacagg 7200
gctgccctgc ctcataggta gccatggtga gggctatctg ctgcaggggg gtcttgggga 7260
gagtggtgac tccattgacc cagcttttca ttaaaggata acacactg 7308

<210> 36
<211> 20
<212> DNA
<213> Artificial Sequence

<220>
<223> Primer

<400> 36
gaattcatgc tgcgcaccgc 20

<210> 37
<211> 50
<212> DNA
<213> Artificial Sequence

<220>
<223> Primer

<400> 37
aagcttcact tgtcgtcatc gtccttgtag tcctccttcc agtaggacct 50

<210> 38
<211> 45
<212> DNA
<213> Artificial Sequence

<220>
<223> Primer

<400> 38
aagcttcagt gatgatgatg atgatgctcc ttccagtagg acctg 45

## Description

### TECHNICAL FIELD

The present invention relates to substances that inhibit the binding of SEMA4B proteins, etc. to Plexin B1 proteins, etc. (e.g., antibodies), substances that inhibit the activities of SEMA4B proteins, etc. and Plexin B1 proteins (e.g., antibodies), agents for preventing/treating cancer or agents for promoting the apoptosis of cancer comprising the substances, screening of the agents for preventing/treating cancer or agents for promoting the apoptosis of cancer, and so on.

### BACKGROUND ART

Recent advance in microarray/oligonucleotide array technology has enabled exhaustive analysis of gene expression. It is predicted that a cancer could also be assessed for its pathological conditions by microarray profiling data for the gene. Actually in leukemia, it is reportedly possible to classify leukemia by gene expression profiles. By clarifying the gene expression profile of each cancerous tissue and accumulating its classification, it is considered possible to predict any response to a particular cancer therapy or discover a novel drug development target protein for a particular cancer. Specifically, where enhanced expression of a certain protein is observed in a certain cancer, it becomes possible to induce an anti-tumor activity in patients newly diagnosed to be antigen positive, by means of (i) reducing its expression level, (ii) suppressing its function, (iii) eliciting immune response of host to the protein, etc. At the same time, patients diagnosed to be antigen negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based drugs.

The semaphorin family is a large protein family composed of both secreted molecules and membrane-bound molecules and reportedly has at least 19 genes in vertebrates and 3 genes in non-vertebrates (Cell, 97, 551-552, 1999)

The semaphorin family is known to be involved in a wide range of neurogenetic process as typified by neuronal axon guidance, synapse formation, etc. In recent years, light is shedding on a participation of the semaphorin family in the immune system (Trends in Immunol., 22, 670-676, 2001) or involvement in organogenesis/angiogenesis. Human-derived semaphorin 3B and semaphorin 3F, which belong to the semaphorin family, are reported to be tumor suppressor genes (Proc. Natl Acad. Sci. USA, 98, 13954-13959, 2001, Cancer Res., 62, 542-546, 2002, Cancer Res., 62, 2637-2643, 2002). It is reported that human semaphorin 3C is overexpressed in human lung cancer tissues (J. Surg. Oncol., 72, 18-23, 1999, Proc. Natl Acad. Sci. USA, 94, 14713-14718, 1997). It is reported that human semaphorin 3E is expressed in metastatic cells (Cancer Res., 58, 1238-1244, 1998).

Human semaphorin 4B (hereinafter sometimes abbreviated as SEMA4B; SEQ ID NO: 1) has 41% homology to human semaphorin 4D (hereinafter sometimes abbreviated as SEMA4D) on an amino acid level and is reportedly one of the genes, which expression increases under hypoxic conditions (WO 02/46465). There is a further report that several hundreds of base sequences including SEMA4B, etc. can be used for diagnosis of lung cancer or survey of compounds for treating lung cancer based on gene chip analysis (WO 02/86443). It is reported that NOV7 having 93% homology to SEMA4B on an amino acid level is overexpressed in cancer (WO 02/06329). In WO 03/003906 there are reported methods for diagnosis of bladder cancer using polynucleotides or polypeptides having more than 60% homology to SEMA4B, etc., antibodies, screening of compounds modulating bladder cancer-related proteins, etc. It is also known that a ligand for human Plexin B 1 (hereinafter sometimes simply referred to as Plexin B1; GenBank: AB007867) is SEMA4B (Cell, 99, 71-80, 1999) and that Plexin B1 forms a complex with human hepatocytes growth factor (HGF) and stimulation of SEMA4D results in phosphorylation of both Plexin B1 and HGF receptors to promote cell growth (Nature Cell Biol., 4, 720-724, 2002).

A safe drug to target a molecule specifically expressed in cancer cells and induce cancer cell growth inhibition has been earnestly desired.

### DISCLOSURE OF THE INVENTION

In order to solve the foregoing problems, the present inventors made extensive investigations and as a result, found that SEMA4B binds to Plexin B1, apoptosis in cancer cells is promoted by inhibiting the binding of both proteins and antibodies to SEMA4B inhibit the binding of SEMA4B to Plexin B1. Based on these findings, the present invention has come to be accomplished.

That is, the present invention provides the following features, and so on.
[1] A substance that inhibits the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.
[2] The substance according to [1], wherein the substance is an antibody to a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
   [2a] The substance according to [2], which is an antibody further recognizing a protein comprising the same or substantially the same amino acid sequence as the amino acid sequence represented by SEQ ID NO: 26, its partial peptide or a salt thereof.
[3] The substance according to [1], wherein the substance is an antibody having the activity of neutralizing cancer cell growth stimulation induced by the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.
[4] The substance according to [2] or [3], wherein the antibody is a monoclonal antibody.
[5] An agent for preventing/treating cancer, which comprises the substance according to [1].
[6] An agent for promoting the apoptosis in cancer cells, which comprises the substance according to [1].
[7] An agent for inhibiting the growth of cancer cells, which comprises the substance according to [1].
[8] An agent for inhibiting the growth of cancer cells, which comprises a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
   [8a] An agent for preventing/treating cancer, which comprises a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
   [8b] An agent for promoting the apoptosis in cancer cells, which comprises a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
[9] A substance that inhibits the activity of a protein comprising the same or substantially the same amino acid sequence as the amino acid sequence represented by SEQ ID NO: 26, its partial peptide or a salt thereof.
   [9a] A substance that inhibits the expression of a gene for a protein comprising the same or substantially the same amino acid sequence as the amino acid sequence represented by SEQ ID NO: 26, its partial peptide or a salt thereof.
[10] The substance according to [9], wherein the substance is an antibody having the activity of inhibiting phosphorylation of a protein comprising the same or substantially the same amino acid sequence as the amino acid sequence represented by SEQ ID NO: 26, its partial peptide or a salt thereof.
[11] The substance according to [10], wherein the substance is an antibody having the activity of neutralizing cancer cell growth stimulation induced by the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.
[12] An agent for preventing/treating cancer, which comprises the substance according to [9].
   [12a] An agent for preventing/treating cancer, which comprises the substance according to [9a].
[13] An agent for promoting the apoptosis in cancer cells, which comprises the substance according to [9].
   [13a] An agent for promoting the apoptosis in cancer cells, which comprises the substance according to [9a].
[14] An agent for inhibiting the growth of cancer cells, which comprises the substance according to [9].
   [14a] An agent for inhibiting the growth of cancer cells, which comprises the substance according to [9a].
   [14b] An agent for preventing/treating cancer, which comprises an antisense polynucleotide comprising a base sequence complimentary or substantially complimentary to a polynucleotide encoding a protein comprising the same or substantially the same amino acid sequence as the amino acid sequence represented by SEQ ID NO: 26, its partial peptide or a salt thereof, or a part of the base sequence.
   [14c] An agent for promoting the apoptosis in cancer cells, which comprises an antisense polynucleotide comprising a base sequence complimentary or substantially complimentary to a polynucleotide encoding a protein comprising the same or substantially the same amino acid sequence as the amino acid sequence represented by SEQ ID NO: 26, its partial peptide or a salt thereof, or a part of the base sequence.
   [14d] An agent for inhibiting the growth of cancer cells, which comprises an antisense polynucleotide comprising a base sequence complimentary or substantially complimentary to a polynucleotide encoding a protein comprising the same or substantially the same amino acid sequence as the amino acid sequence represented by SEQ ID NO: 26, its partial peptide or a salt thereof, or a part of the base sequence.
[15] A method of screening a substance that inhibits the binding of (a) a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to (b) a protein comprising the same or substantially the same amino acid sequence as the amino acid sequence represented by SEQ ID NO: 26, its partial peptide or a salt thereof, which is characterized by using (a) the aforesaid protein, its partial peptide or a salt thereof, and (b) the aforesaid protein, its partial peptide or a salt thereof.
[16] A kit for screening a substance that inhibits the binding of (a) a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to (b) a protein comprising the same or substantially the same amino acid sequence as the amino acid sequence represented by SEQ ID NO: 26, its partial peptide or a salt thereof, which is characterized by comprising (a) the aforesaid protein, its partial peptide or a salt thereof, and (b) the aforesaid protein, its partial peptide or a salt thereof.
   [16a] A substance which is obtainable using the screening method according to [15] or the screening kit according to [16].
   [16b] An agent for preventing/treating cancer, an agent for promoting the apoptosis in cancer cells or an agent for inhibiting the growth of cancer cells, which comprises the substance according to [16a].
[17] A method of screening a substance that inhibits the activity of a protein comprising the same or substantially the same amino acid sequence as the amino acid sequence represented by SEQ ID NO: 26, its partial peptide or a salt thereof, which is characterized by using the protein, its partial peptide or a salt thereof.
   [17a] A method of screening a substance that inhibits the expression of a gene for a protein comprising the same or substantially the same amino acid sequence as the amino acid sequence represented by SEQ ID NO: 26, its partial peptide or a salt thereof, which is characterized by using a gene for the protein comprising the same or substantially the same amino acid sequence as the amino acid sequence represented by SEQ ID NO: 26, its partial peptide or a salt thereof.
[18] A kit for screening a substance that inhibits the activity of a protein comprising the same or substantially the same amino acid sequence as the amino acid sequence represented by SEQ ID NO: 26, its partial peptide or a salt thereof, which is characterized by comprising a protein comprising the same or substantially the same amino acid sequence as the amino acid sequence represented by SEQ ID NO: 26, its partial peptide or a salt thereof.
   [18a] A substance which is obtainable using the screening method according to [17] or the screening kit according to [18].
   [18b] An agent for preventing/treating cancer, an agent for promoting the apoptosis in cancer cells or an agent for inhibiting the growth of cancer cells, which comprises the substance according to [18a].
[19] A method of preventing/treating cancer, a method of promoting the apoptosis in cancer cells and/or a method of inhibiting the growth of cancer cells, which is characterized by inhibiting the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.
[20] The method according to [19], which comprises using an antibody to a protein comprising the same or substantially the same as the amino acid sequence represented by SEQ ID NO: 1, SEQ ID NO: 4, SEQ ID NO: 7 or SEQ ID NO: 10, its partial peptide or a salt thereof.
[21] A method of preventing/treating cancer, a method of promoting the apoptosis in cancer cells and/or a method of inhibiting the growth of cancer cells, which is characterized by inhibiting the phosphorylation of a protein comprising the same or substantially the same amino acid sequence as the amino acid sequence represented by SEQ ID NO: 26, its partial peptide or a salt thereof.
   [21a] A method of preventing/treating cancer, a method of promoting the apoptosis in cancer cells and/or a method of inhibiting the growth of cancer cells, which is characterized by inhibiting the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
[22] The method according to [21], which comprises using an antibody to a protein comprising the same or substantially the same as the amino acid sequence represented by SEQ ID NO: 1, SEQ ID NO: 4, SEQ ID NO: 7 or SEQ ID NO: 10, its partial peptide or a salt thereof.
   [22a] The substance according to [22], which is an antibody further recognizing a protein comprising the same or substantially the same amino acid sequence as the amino acid sequence represented by SEQ ID NO: 26, its partial peptide or a salt thereof.
   [22b] The substance according to [21a], which comprises using an antibody further recognizing a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
   [22c] The substance according to [22b], which is an antibody further recognizing a protein comprising the same or substantially the same amino acid sequence as the amino acid sequence represented by SEQ ID NO: 26, its partial peptide or a salt thereof.
[23] A method of preventing/treating cancer, a method of promoting the apoptosis in cancer cells and/or a method of inhibiting the growth of cancer cells, which comprises administering to a mammal an effective dose of the substance according to [1] or [9].
   [23a] A method of preventing/treating cancer, a method of promoting the apoptosis in cancer cells and/or a method of inhibiting the growth of cancer cells, which comprises administering to a mammal an effective dose of a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.
[24] Use of the substance according to [1] or [9] to manufacture an agent for preventing/treating cancer, an agent for promoting the apoptosis in cancer cells and/or an agent for inhibiting the growth of cancer cells.
   [24a] Use of a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to manufacture an agent for preventing/treating cancer, an agent for promoting the apoptosis in cancer cells and/or an agent for inhibiting the growth of cancer cells.

### BEST MODE FOR CARRYING OUT THE INVENTION

The protein comprising the same or substantially the same amino acid sequence as the amino acid sequence represented by SEQ ID NO: 1, SEQ ID NO: 4, SEQ ID NO: 7 or SEQ ID NO: 10 (hereinafter these proteins are sometimes referred to as the protein of the present invention or as the protein used in the present invention) may be any protein derived from any cells of human and warm-blooded animals (e.g., guinea pig, rat, mouse, fowl, rabbit, swine, sheep, bovine, simian, etc.) such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence comprising substantially the same amino acid sequence as that represented by SEQ ID NO: 1 includes amino acid sequences having at least about 95% homology, preferably at least about 98% homology and most preferably at least about 99% homology, to the amino acid sequence shown by SEQ ID NO: 1; etc.

Preferred examples of the protein comprising substantially the same amino acid sequence as the amino acid sequence represented by SEQ ID NO: 1 include proteins comprising substantially the same amino acid sequence as the amino acid sequence represented by SEQ ID NO: 1 and having a property substantially equivalent to that of the protein containing the amino acid sequence represented by SEQ ID NO: 1, etc.

The amino acid sequence comprising substantially the same amino acid sequence as that represented by SEQ ID NO: 4 includes amino acid sequences having at least about 99.9% homology, to the amino acid sequence shown by SEQ ID NO: 4; etc.

Preferred examples of the protein comprising substantially the same amino acid sequence as the amino acid sequence represented by SEQ ID NO: 4 include proteins comprising substantially the same amino acid sequence as the amino acid sequence represented by SEQ ID NO: 4 and having a property substantially equivalent to that of the protein containing the amino acid sequence represented by SEQ ID NO: 4, etc.

The amino acid sequence comprising substantially the same amino acid sequence as that represented by SEQ ID NO: 7 includes amino acid sequences having at least about 99.9% homology, to the amino acid sequence shown by SEQ ID NO: 7; etc.

Preferred examples of the protein comprising substantially the same amino acid sequence as the amino acid sequence represented by SEQ ID NO: 7 include proteins comprising substantially the same amino acid sequence as the amino acid sequence represented by SEQ ID NO: 7 and having a property substantially equivalent to that of the protein containing the amino acid sequence represented by SEQ ID NO: 7, etc.

The amino acid sequence comprising substantially the same amino acid sequence as that represented by SEQ ID NO: 10 includes amino acid sequences having at least about 99.9% homology, to the amino acid sequence shown by SEQ ID NO: 10; etc.

Preferred examples of the protein comprising substantially the same amino acid sequence as the amino acid sequence represented by SEQ ID NO: 10 include proteins comprising substantially the same amino acid sequence as the amino acid sequence represented by SEQ ID NO: 10 and having a property substantially equivalent to that of the protein containing the amino acid sequence represented by SEQ ID NO: 10, etc.

Homology of the amino acid sequences can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

As the substantially equivalent activity described above, there are, for example, the activity of binding the receptor used in the present invention (later described), the activity of inducing/promoting phosphorylation of the receptor used in the present invention, and the like. The substantially equivalent is used to mean that the nature of these properties is equivalent in terms of quality (e.g., physiologically or pharmacologically). Thus, the activity of binding the receptor used in the present invention, the activity of inducing/promoting phosphorylation of the receptor used in the present invention, and the like are preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The binding activity described above can be assayed by publicly known methods, e.g., EIA, immunoprecipitation, or a modification of these methods. Specifically, the protein used in the present invention and the receptor used in the present invention are expressed as tagged recombinant proteins, respectively, in animal cells. FLAG, His, V5, myc, HA, etc. are used as the tag. A tag (Tag A) employed to attach the tag to the protein used in the present invention is different from a tag (Tag B) employed to attach the tag to the receptor used in the present invention. A mixture of the Tag A-tagged protein and the Tag B-tagged receptor described above is immunoprecipitated with an antibody to Tag B. By analyzing the precipitates obtained by western blotting with an antibody to Tag A, the amount of the protein used in the present invention, which is bound to the receptor used in the present invention, can be determined.

The phosphorylation inducing/promoting activity described above can be assayed by publicly known methods, e.g., the method described in Methods in Enzymology, 200, 98-107, 1991, or a modification of the method. Specifically, the receptor used in the present invention, which is tagged with, for example, a tag (e.g., FLAG, His, V5, myc, HA, etc.) at the C-terminus, is expressed as a recombinant protein in animal cells. After separately reacting with the protein used in the present invention, the cells are disrupted to prepare a cell-free extract and immunoprecipitated with an anti-tag antibody. The amount of the resulting receptor used in the present invention, which has been phosphorylated, can be determined by publicly known methods (e.g., western blotting, etc.) using an anti-phosphorylated tyrosine antibody, etc.

Examples of the protein used in the present invention include so-called muteins such as proteins having (1) (i) the amino acid sequence represented by SEQ ID NO: 1, of which at least 1 or 2 (e.g., about 1 to about 50, preferably about 1 to about 30, more preferably about 1 to about 10 and most preferably several (1 to 5)) amino acids are deleted; (ii) the amino acid sequence represented by SEQ ID NO: 1, to which at least 1 or 2 (e.g., about 1 to about 50, preferably about 1 to about 30, more preferably about 1 to about 10 and most preferably several (1 to 5)) amino acids are added; (iii) the amino acid sequence represented by SEQ ID NO: 1, in which at least 1 or 2 (e.g., about 1 to about 50, preferably about 1 to about 30, more preferably about 1 to about 10 and most preferably several (1 to 5)) amino acids are inserted; (iv) the amino acid sequence represented by SEQ ID NO: 1, in which at least 1 or 2 (e.g., about 1 to about 50, preferably about 1 to about 30, more preferably about 1 to about 10 and most preferably several (1 to 5)) amino acids are substituted by other amino acids; or (v) a combination of these amino acid sequences, which is so-called mutein; and the like.
(2) (i) the amino acid sequence represented by SEQ ID NO: 4, SEQ ID NO: 7 or SEQ ID NO: 10, of which at least 1 or 2 amino acids are deleted; (ii) the amino acid sequence represented by SEQ ID NO: 4, SEQ ID NO: 7 or SEQ ID NO: 10, to which at least 1 or 2 (e.g., about 1 to about 50, preferably about 1 to about 30, more preferably about 1 to about 10 and most preferably several (1 to 5)) amino acids are added; (iii) the amino acid sequence represented by SEQ ID NO: 4, SEQ ID NO: 7 or SEQ ID NO: 10, in which at least 1 or 2 amino acids are inserted; (iv) the amino acid sequence represented by SEQ ID NO: 4, SEQ ID NO: 7 or SEQ ID NO: 10, in which at least 1 or 2 amino acids are substituted by other amino acids; or (v) a combination of these amino acid sequences, which is so-called mutein; and the like.

Where the amino acid sequence is inserted, deleted or substituted as described above, the position of its insertion, deletion or substitution is not particularly limited.

Specific examples of the protein used in the present invention include a protein comprising the amino acid sequence represented by SEQ ID NO: 1, a protein comprising the amino acid sequence represented by SEQ ID NO: 4, a protein comprising the amino acid sequence represented by SEQ ID NO: 7, a protein comprising the amino acid sequence represented by SEQ ID NO: 10, and the like.

The protein comprising the same or substantially the same amino acid sequence as the amino acid sequence represented by SEQ ID NO: 26 (hereinafter these proteins are sometimes referred to as the receptor used in the present invention) may be any protein derived from any cells of human and warm-blooded animals (e.g., guinea pig, rat, mouse, fowl, rabbit, swine, sheep, bovine, simian, etc.) such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence comprising substantially the same amino acid sequence as that represented by SEQ ID NO: 26 includes amino acid sequences having at least about 50% homology, preferably at least about 60% homology, preferably at least about 70% homology, preferably at least about 80% homology, preferably at least about 90% homology and preferably at least about 99% homology, to the amino acid sequence shown by SEQ ID NO: 26; etc.

Preferred examples of the protein comprising substantially the same amino acid sequence as the amino acid sequence represented by SEQ ID NO: 26 include proteins comprising substantially the same amino acid sequence as the amino acid sequence represented by SEQ ID NO: 26 and having a property substantially equivalent to that of the protein containing the amino acid sequence represented by SEQ ID NO: 26, etc.

As the substantially equivalent activity described above, there are, for example, the activity of binding the protein used in the present invention, to be phosphorylated (e.g., to be phosphorylated by stimulation of the protein of the present invention, etc.), and the like. The substantially equivalent is used to mean that the nature of these properties is equivalent in terms of quality (e.g., physiologically or pharmacologically). Thus, the activities described above are preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The phosphorylated activity can be assayed by publicly known methods, e.g., the method described in Methods in Enzymology, 200, 98-107, 1991, or a modification of the method. Specifically, the receptor used in the present invention, which is tagged with, for example, a tag (e.g., FLAG, His, V5, myc, HA, etc.) at the C-terminus, is expressed as a recombinant protein in animal cells. After separately reacting with the protein used in the present invention, the cells are disrupted to prepare a cell-free extract and immunoprecipitated with an anti-tag antibody. The amount of the resulting receptor used in the present invention, which has been phosphorylated, can be quantified by publicly known methods (e.g., western blotting, etc.) using an anti-phosphorylated tyrosine antibody, etc.

Examples of the receptor used in the present invention include so-called muteins such as proteins having (i) the amino acid sequence represented by SEQ ID NO: 26, of which at least 1 or 2 (e.g., about 1 to about 50, preferably about 1 to about 30, more preferably about 1 to about 10 and most preferably several (1 to 5)) amino acids are deleted; (ii) the amino acid sequence represented by SEQ ID NO: 26, to which at least 1 or 2 (e.g., about 1 to about 50, preferably about 1 to about 30, more preferably about 1 to about 10 and most preferably several (1 to 5)) amino acids are added; (iii) the amino acid sequence represented by SEQ ID NO: 26, in which at least 1 or 2 (e.g., about 1 to about 50, preferably about 1 to about 30, more preferably about 1 to about 10 and most preferably several (1 to 5)) amino acids are inserted; (iv) the amino acid sequence represented by SEQ ID NO: 26, in which at least 1 or 2 (e.g., about 1 to about 50, preferably about 1 to about 30, more preferably about 1 to about 10 and most preferably several (1 to 5)) amino acids are substituted by other amino acids; or (v) a combination of these amino acid sequences, which is so-called mutein; and the like.

Where the amino acid sequence is inserted, deleted or substituted as described above, the position of its insertion, deletion or substitution is not particularly limited.

Specific examples of the receptor used in the present invention include a protein comprising the amino acid sequence represented by SEQ ID NO: 26, etc.

The receptor used in the present invention may also form a complex with hepatocytes growth factor (HGF).

Throughout the specification, the proteins are represented in accordance with the conventional way of describing proteins, that is, the N-terminus (amino terminus) at the left hand and the C-terminus (carboxyl terminus) at the right hand. In the protein and receptor used in the present invention, the C-terminus may be in any form of a carboxyl group (-COOH), a carboxylate (-COO⁻), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

The partial peptide of the protein or the receptor used in the present invention (partial peptide used in the present invention) may be any peptide as long as it is a partial peptide of the protein or the receptor used in the present invention described above and preferably has the property equivalent to that of the protein or the receptor used in the present invention described above.

For example, there are used peptides containing, e.g., at least 20, preferably at least 50, more preferably at least 70, much more preferably at least 100 and most preferably at least 200 amino acids, in the constituent amino acid sequence of the protein or receptor used in the present invention, etc.

The partial peptide described above may be peptides containing the amino acid sequence, of which at least 1 or 2 (preferably about 1 to about 20, more preferably about 1 to about 10 and most preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 20, more preferably several and most preferably about 1 to about 5) amino acids may be substituted by other amino acids.

In the partial peptide used in the present invention, the C-terminus may be in any form of a carboxyl group (-COOH), a carboxylate (-COO⁻), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated peptides such as so-called glycopeptides having sugar chains; etc., as in the protein used in the present invention described above.

The partial peptide used in the present invention may also be used as an antigen for producing antibodies.

As salts of the protein, receptor or partial peptides used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or receptor, or its partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or non-human mammalian tissues or cells are homogenized, extracted with an acid or the like, and the extract is purified and isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; conversely when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Examples of the DNA encoding the protein used in the present invention may be any one of:
(i) a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein comprising the amino acid sequence represented by SEQ ID NO: 1 described above,
(ii) a DNA comprising the base sequence represented by SEQ ID NO: 5, or a DNA comprising a base sequence hybridizable to the base sequence represented by SEQ ID NO: 5 under high stringent conditions and encoding a protein which has the properties of substantially the same nature as those of the protein comprising the amino acid sequence represented by SEQ ID NO: 4 described above,
(iii) a DNA comprising the base sequence represented by SEQ ID NO: 8, or a DNA comprising a base sequence hybridizable to the base sequence represented by SEQ ID NO: 8 under high stringent conditions and encoding a protein which has the properties of substantially the same nature as those of the protein comprising the amino acid sequence represented by SEQ ID NO: 7 described above,
(iv) a DNA comprising the base sequence represented by SEQ ID NO: 11, or a DNA comprising a base sequence hybridizable to the base sequence represented by SEQ ID NO: 11 under high stringent conditions and encoding a protein which has the properties of substantially the same nature as those of the protein comprising the amino acid sequence represented by SEQ ID NO: 10 described above,

As the DNA that is hybridizable to the base sequence represented by SEQ ID NO: 2 under high stringent conditions, there are employed, for example, DNAs comprising base sequences having at least about 95% homology, preferably at least about 98% homology and preferably at least about 99% homology, to the base sequence represented by SEQ ID NO: 2; and the like.

As the DNA that is hybridizable to the base sequence represented by SEQ ID NO: 5 under high stringent conditions, there are employed, for example, DNAs comprising base sequences having at least about 99% homology, to the base sequence represented by SEQ ID NO: 5; and the like.

As the DNA that is hybridizable to the base sequence represented by SEQ ID NO: 8 under high stringent conditions, there are employed, for example, DNAs comprising base sequences having at least about 99% homology, to the base sequence represented by SEQ ID NO: 8; and the like.

As the DNA that is hybridizable to the base sequence represented by SEQ ID NO: 11 under high stringent conditions, there are employed, for example, DNAs comprising base sequences having at least about 99% homology, to the base sequence represented by SEQ ID NO: 11; and the like.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, there are employed: (i) a DNA comprising the base sequence represented by SEQ ID NO: 2, a DNA comprising the base sequence represented by SEQ ID NO: 3, etc. as the DNA encoding the protein comprising the amino acid sequence represented by SEQ ID NO: 1; (ii) a DNA comprising the base sequence represented by SEQ ID NO: 5, a DNA comprising the base sequence represented by SEQ ID NO: 6, etc. as the DNA encoding the protein comprising the amino acid sequence represented by SEQ ID NO: 4; (iii) a DNA comprising the base sequence represented by SEQ ID NO: 8, a DNA comprising the base sequence represented by SEQ ID NO: 9, etc. as the DNA encoding the protein comprising the amino acid sequence represented by SEQ ID NO: 7; (iv) a DNA comprising the base sequence represented by SEQ ID NO: 11, a DNA comprising the base sequence represented by SEQ ID NO: 12, etc. as the DNA encoding the protein comprising the amino acid sequence represented by SEQ ID NO: 10.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, SEQ ID NO: 5, SEQ ID NO: 8 or SEQ ID NO: 11, or a DNA comprising a base sequence hybridizable to the base sequence represented by SEQ ID NO: 2, SEQ ID NO: 5, SEQ ID NO: 8 or SEQ ID NO: 11 under high stringent conditions and comprising a part of DNA encoding a protein having the activities of substantially the same nature as those of the protein of the present invention, and the like.

The DNA hybridizable to the base sequence represented by SEQ ID NO: 2, SEQ ID NO: 5, SEQ ID NO: 8 or SEQ ID NO: 11 indicates the same meaning as described above.

Methods for the hybridization and the high stringent conditions that can be used are the same as those described above.

The polynucleotide encoding the receptor used in the present invention may be any polynucleotide so long as it contains the base sequence encoding the receptor used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

As the DNA encoding the receptor used in the present invention, there are employed, for example, a DNA comprising a base sequence represented by SEQ ID NO: 35, or a DNA comprising a base sequence hybridizable to the base sequence represented by SEQ ID NO: 35 under high stringent conditions and comprising a part of DNA encoding a protein having the activities of substantially the same nature as those of the protein comprising the amino acid sequence represented by SEQ ID NO: 26, and the like.

As the DNA that is hybridizable to the base sequence represented by SEQ ID NO: 35 under high stringent conditions, there are employed, for example, DNAs comprising base sequences having at least about 50% homology, preferably at least about 60% homology, preferably at least about 70% homology, preferably at least about 80% homology and preferably at least about 90% homology, to the base sequence represented by SEQ ID NO: 35; and the like.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, a DNA comprising the base sequence represented by SEQ ID NO: 35 or the like is employed as the DNA encoding the protein comprising the amino acid sequence represented by SEQ ID NO: 26.

For cloning of DNAs that completely encode the protein or receptor used in the present invention or its partial peptide (hereinafter sometimes merely referred to as the protein of the present invention in the description of cloning of DNAs encoding the same and their expression), the DNA can be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (Takara Bio) or Mutan™-K (Takara Bio), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include simian cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Vrology, 52, 456 (1973).

Thus, the transformants transformed with the expression vectors bearing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The "substance that inhibits the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof (the protein used in the present invention), to a protein comprising the same or substantially the same amino acid sequence as the amino acid sequence represented by SEQ ID NO: 26, its partial peptide or a salt thereof (the receptor used in the present invention)" may be any substance so long as it is a substance that inhibits the binding of the protein used in the present invention to the receptor used in the present invention (e.g., antibodies, peptides, proteins, non-peptide compounds, synthetic compounds, fermentation products, cell extracts, plant extracts, animal tissue extracts, etc.). Examples of the substance include an antibody reacting specifically with the protein used in the present invention, an antibody reacting specifically with the receptor used in the present invention, a bispecific antibody reacting specifically with the protein used in the present invention and reacting specifically with the receptor used in the present invention, an antibody that inhibits the activities of the receptor used in the present invention (e.g., the activity of binding the protein used in the present invention, to be phosphorylated, etc.), an antibody that inhibits the activities of the protein used in the present invention (e.g., the activity of binding the receptor used in the present invention, the phosphorylation inducing/promoting activity of the receptor used in the present invention, etc.) (hereinafter these substances are sometimes collectively referred to as the antibody of the present invention).

The "substance that inhibits the activity of the protein used in the present invention" may be any substance so long as it is a substance that inhibits the activities of the protein used in the present invention (e.g., the activity of binding the receptor used in the present invention, the phosphorylation inducing/promoting activity of the receptor used in the present invention, etc.) (e.g., antibodies, peptides, proteins, non-peptide compounds, synthetic compounds, fermentation products, cell extracts, plant extracts, animal tissue extracts, etc.), and the substance is exemplified by the antibody of the present invention, etc.

The "substance (e.g., antibodies, peptides, proteins, non-peptide compounds, synthetic compounds, fermentation products, cell extracts, plant extracts, animal tissue extracts, etc.) that inhibits the activity of the receptor used in the present invention" may be any substance so long as it is a substance that inhibits the activities of the receptor used in the present invention (e.g., the activity of binding the protein used in the present invention, to be phosphorylated, etc.), and is exemplified by the antibody of the present invention, etc.

The antibody of the present invention may be any one of polyclonal antibodies and monoclonal antibodies.

The antibody of the present invention is preferably an antibody having an activity of neutralizing the cancer cell growth stimulation induced by the binding of the protein used in the present invention to the receptor used in the present invention (neutralizing antibody). Or, the antibody is an antibody that inhibits the activities of the receptor used in the present invention (preferably to be phosphorylated, etc.). More preferably, the antibody is a monoclonal antibody.

Hereinafter, preparation of an antigen for the antibody of the present invention and preparation of the antibody will be described.

### (1) Preparation of antigen

As an antigen used to prepare the antibody of the present invention, any antigen such as a (synthetic) peptide having 1 or 2 more antigenic determinants, which are the same as in the protein comprising the amino acid sequence represented by SEQ ID NO: 1, SEQ ID NO: 4, SEQ ID NO: 7 or SEQ ID NO: 10, or SEQ ID NO: 26, its partial peptide or salts thereof, etc. may be used (hereinafter these antigens are sometimes merely referred to as the antigen of the present invention).

The aforesaid protein, its partial peptide or salts thereof can be produced by modifications of REFERENCE EXAMPLES later described or publicly known methods. They may also be (a) prepared from mammalian tissues or cells of human, simian, rat, mouse, etc., by publicly known methods or with modifications, (b) chemically synthesized by publicly known peptide synthesis methods using a peptide synthesizer, etc., or (c) produced by culturing a transformant bearing a DNA encoding a polypeptide comprising the amino acid sequence represented by SEQ ID NO: 1, SEQ ID NO: 4, SEQ ID NO: 7 or SEQ ID NO: 10, or SEQ ID NO: 26, or salts thereof.
(a) Where the antigen of the present invention is prepared from the mammalian tissues or cells, the crude extract (e.g., membrane fraction, soluble fraction) can be used per se as an antigen. Alternatively, the tissues or cells are homogenized, then extracted with an acid, an alcohol, etc., and the extract is purified and isolated by a combination of salting-out, dialysis, gel filtration, chromatography techniques such as reverse phase chromatography, ion exchange chromatography, affinity chromatography and the like.
(b) Where the antigen of the present invention is prepared chemically, the synthetic peptides used are, for example, a peptide having the same structure as the antigen of the present invention purified from natural one, a peptide containing 1 or 2 more amino acid sequences, which are the same amino acid sequences consisting of at least 3, preferably at least 6 amino acids in an optional region of the amino acid sequence represented by SEQ ID NO: 1, SEQ ID NO: 4, SEQ ID NO: 7 or SEQ ID NO: 10, or SEQ ID NO: 26, etc.
(c) Where the protein comprising the amino acid sequence represented by SEQ ID NO: 1, SEQ ID NO: 4, SEQ ID NO: 7 or SEQ ID NO: 10, or SEQ ID NO: 26, or salts thereof are produced using the DNA-bearing transformants, the DNA can be produced in accordance with publicly known cloning techniques [e.g., the method described in Molecular Cloning (2nd ed., J. Sambrook et al., Cold Spring Harbor Lab. Press, 1989), etc.]. The cloning techniques include (1) a method in which transformants containing DNAs encoding the protein comprising the amino acid sequence represented by SEQ ID NO: 1, SEQ ID NO: 4, SEQ ID NO: 7 or SEQ ID NO: 10, or SEQ ID NO: 26, or salts thereof are obtained from cDNA library by hybridization using DNA probes or DNA primers designed based on the amino acid sequence of the protein comprising the amino acid sequence represented by SEQ ID NO: 1, SEQ ID NO: 4, SEQ ID NO: 7 or SEQ ID NO: 10, or SEQ ID NO: 26, or salts thereof, or (2) a method in which transformants containing DNAs encoding the protein comprising the amino acid sequence represented by SEQ ID NO: 1, SEQ ID NO: 4, SEQ ID NO: 7 or SEQ ID NO: 10, or SEQ ID NO: 26, or salts thereof are obtained by PCR using DNA primers designed based on the amino acid sequence of a polypeptide comprising the amino acid sequence represented by SEQ ID NO: 1, SEQ ID NO: 4, SEQ ID NO: 7 or SEQ ID NO: 10, or SEQ ID NO: 26, or salts thereof, etc.

Mammalian cells which express the protein or receptor used in the present invention can also be used directly as the antigen of the present invention. As the mammalian cells, there can be used the naturally occurring cells as described in (a) above, cells transformed by the methods as described in (c) above, etc. Hosts used for the transformation may be any cells as far as they are cells collected from human, simian, rat, mouse, hamster, etc. and preferably used are HEK293, COS7, CHO-K1, NIH3T3, Balb3T3, FM3A, L929, SP2/0, P3U1, B16, P388, or the like. Naturally occurring mammalian cells or transformed mammalian cells, which express the protein or receptor used in the present invention, can be injected to immune animal as a suspension of the cells in a medium used for tissue culture (e.g., RPMI 1640) or buffer (e.g., Hanks' Balanced Salt Solution). Immunization may be done by any method, as long as it can stimulate antibody production, and preferably used are intravenous injection, intraperitoneal injection, intramuscular injection, subcutaneous injection, etc.

Peptides used as the antigen of the present invention can also be prepared (1) by peptide synthesis methods publicly known, or (2) by cleaving the protein comprising the amino acid sequence represented by SEQ ID NO: 1, SEQ ID NO: 4, SEQ ID NO: 7 or SEQ ID NO: 10, or SEQ ID NO: 26 with an appropriate peptidase.

For the methods for peptide synthesis, for example, any of solid phase synthesis and liquid phase syntheses may be used. That is, the partial peptides or amino acids that can construct the peptide are condensed with the remaining part. Where the product contains protecting groups, these protecting groups are removed to give the desired peptide. Publicly known methods for condensation and removal of the protecting groups are methods described below.
(i) M. Bodanszky & M.A. Ondetti: Peptide Synthesis, Interscience Publishers, New York(1966)
(ii) Schroeder & Luebke: The Peptide, Academic Press, New York (1965)

After completion of the reaction, the product may be purified and isolated by a combination of conventional purification methods such as solvent extraction, distillation, column chromatography, liquid chromatography and recrystallization to give the peptide. When the peptide obtained by the above methods is in a free form, the peptide can be converted into an appropriate salt by a publicly known method or its modification; conversely when the peptide is obtained in a salt form, it can be converted into a free form by a publicly known method.

To synthesize amides of the peptide, commercially available resins for peptide synthesis that are suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective peptide according to various condensation methods publicly known. At the end of the reaction, the peptide is excised from the resin and at the same time, the protecting groups are removed to obtain the objective peptide. Alternatively, the partially protected peptide may be taken out using chlorotrityl resin, oxime resin, 4-hydroxybenzoic acid resin, etc., the protective groups are removed in a conventional manner to obtain the objective peptide.

For condensation of the protected amino acids described above, a variety of activation reagents for peptide synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization-suppressing additive (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be appropriately chosen from solvents that are known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the amino groups in the starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups for carboxyl groups include a C₁₋₆ alkyl group, C₃₋₈ cycloalkyl group, a C₇₋₁₄ aralkyl group, 2-adamantyl, 4-nitrobenzyl, 4-methoxybenzyl, 4-chlorobenzyl, phenacyl, benzyloxycarbonyl hydrazide, trityl hydrazide, or the like.

The hydroxyl group of serine and threonine can be protected through, for example, its esterification or etherification. Examples of the groups suitable for the esterification include a lower (C₁₋₆) alkanoyl group, such as acetyl group, etc.; an aroyl group such as benzoyl group, etc., and a group derived from carbonic acid such as benzyloxycarbonyl group, ethoxycarbonyl group, etc. Examples of a group suitable for the etherification include benzyl group, tetrahydropyranyl group, t-butyl group, etc.

Examples of groups for protecting the phenolic hydroxyl group of tyrosine include Bzl, Cl-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, Bom, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrofluoric acid, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting groups by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups and activation of the functional groups involved in the reaction may be appropriately chosen from publicly known groups and publicly known means.

In another method for obtaining the amides of the peptide, for example, the α-carboxyl group of the carboxy terminal amino acid is first protected by amidation; the peptide chain is then extended to a desired length toward the amino group side. Thereafter, a peptide in which only the protecting group of the N-terminal α-amino group in the peptide chain has been eliminated from the peptide and a peptide (or amino acids) in which only the protecting group of the C-terminal carboxyl group has been eliminated are prepared. The two peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means. Lyophilization of the major fraction gives the amide of the desired peptide.

To prepare the esterified peptide, for example, the α-carboxyl group of the carboxy terminal amino acid is condensed with a desired alcohol to prepare the amino acid ester, which is followed by procedure similar to the preparation of the amidated peptide above to give the ester form of the desired peptide.

The antigen of the present invention may be provided for direct immunization in its immobilized form. The antigen of the present invention may also be bound or adsorbed to an appropriate carrier and the complex produced can be provided for immunization. A mixing ratio of the carrier to the antigen of the present invention (hapten) may be in any ratio of any type, as long as the antibody can be efficiently produced to the antigen of the present invention. A high molecular carrier conventionally used to produce an antibody to a hapten may be used in a weight ratio of 0.1 to 100 based on 1 of hapten. As such a high molecular carrier, there are used a naturally occurring high molecular carrier and a synthetic high molecular carrier. Examples of the naturally occurring high molecular carrier used are serum albumin from mammals such as bovine, rabbit, human, etc., thyroglobulins from mammals such as bovine, rabbit, etc., hemoglobins from mammals such as bovine, rabbit, human, ovine, etc or keyhole limpet KHL hemocyanin. Examples of the synthetic high molecular carrier, which can be used, are various latexes including polymers, copolymers, etc., for example, polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiols with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent (e.g., N-succinimidyl 3-(2-pyridyldithio)propionate (SPDP), etc.) having dithiopyridyl group and then reduced to introduce the thiol group, whereas another amino group is introduced with a maleimide group using a maleimide activated ester reagent, and the two groups may be reacted with each other.

### (2) Preparation of monoclonal antibody

The antigen of the present invention is administered to warm-blooded animals either solely or together with carriers or diluents to the site where the production of antibody is possible by administration routes such as intraperitoneal injection, intravenous injection, subcutaneous injection, etc. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are simian, rabbits, canine, guinea pigs, mice, rats, sheep, goats and fowl, with the use of mice being preferred for preparation of the monoclonal antibody.

In preparation of the monoclonal antibody, a warm-blooded animal, e.g., mice, immunized with the antigen of the present invention wherein the antibody titer is noted is selected, then spleen or lymph node is collected after 2 to 5 days from the final immunization and antibody-producing cells contained therein are fused with myeloma cells to give hybridomas producing the antibody of the present invention. Measurement of antibody titer of the antibody of the present invention in antisera may be carried out, for example, by labeling the protein or receptor used in the present invention with a radioisotope or enzyme, etc., reacting the labeled one with the antiserum followed by assaying the binding activity of the labeling agent bound to the antibody. The fusion may be carried out, for example, by the known method by Koehler and Milstein [Nature, 256, 495 (1975)]. Examples of the fusion accelerator are polyethylene glycol (PEG), Sendai virus, etc., of which PEG is preferably employed. Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of hybridomas producing the antibody of the present invention. Examples of such methods include a method which comprises adding the supernatant of a hybridoma to a solid phase (e.g., a microplate) adsorbed with the protein comprising the amino acid sequence represented by SEQ ID NO: 1, SEQ ID NO: 4, SEQ ID NO: 7 or SEQ ID NO: 10, or SEQ ID NO: 26, or is partial peptide, directly or together with a carrier, adding an anti-immunoglobulin antibody (where mouse cells are used for the cell fusion, anti-mouse immunoglobulin antibody is used) labeled with a radioisotope or enzyme or Protein A and detecting the antibody of the present invention bound to the solid phase, a method which comprises adding the supernatant of a hybridoma to a solid phase adsorbed with an anti-immunoglobulin antibody or Protein A, adding a polypeptide comprising the amino acid sequence represented by SEQ ID NO: 1, SEQ ID NO: 4, SEQ ID NO: 7 or SEQ ID NO: 10, or SEQ ID NO: 26, which is labeled with a radioisotope or enzyme and detecting the antibody of the present invention bound to the solid phase, and the like. The screening and growth of the antibody of the present invention can be performed in a medium for animal cells normally supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for incubation of a hybridoma (SFM-101, Nissui Seiyaku Co., Ltd.) and the like, can be used for the screening and growth medium. The culture is carried out generally at a temperature of 20 to 40°C, preferably at 37°C, for about 5 days to about 3 weeks, preferably 1 to 2 weeks. Incubation can be made normally in 5% carbon dioxide gas.

Separation and purification of the antibody of the present invention can be made by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody alone.]

As described above, the antibody of the present invention can be produced by culturing the hybridoma cell in vivo in a warm-blooded animal or in vitro and collecting the antibody from its body fluid or culture.

Screening of (a) a hybridoma which produces the antibody of the present invention reacting with a segment of the protein comprising the amino acid sequence represented by SEQ ID NO: 1, SEQ ID NO: 4, SEQ ID NO: 7 or SEQ ID NO: 10, or SEQ ID NO: 26, and (b) a hybridoma which produces the antibody of the present invention reacting with the protein described above but not reacting with its segment can be made, for example, by assaying the binding property of the peptide corresponding to its segment and the antibody produced by the hybridoma.

The bispecific monoclonal antibody, which reacts specifically with the protein used in the present invention and the receptor used in the present invention can be manufactured by a modification of publicly known methods.

### [Preparation of polyclonal antibody]

The polyclonal antibody of the present invention can be manufactured by publicly known methods or modifications thereof. For example, a warm-blooded animal is immunized with an immunogen per se, or with a complex of immunogen and a carrier protein formed in a manner similar to the method described above for the manufacture of monoclonal antibodies. The product containing the antibody of the present invention is collected from the immunized animal followed by separation and purification of the antibody.

In the complex of immunogen and carrier protein used to immunize a warm-blooded animal, the type of carrier protein and the mixing ratio of carrier protein to hapten may be any type and in any ratio, as long as the antibody is efficiently produced to the hapten immunized by crosslinking to the carrier protein. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier protein to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed, for example, by the same procedure as the assay of antibody titer of the hybridoma culture supernatant described in (2) above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or receptor used in the present invention or its partial peptide (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, and antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, SEQ ID NO: 3, SEQ ID NO: 5, SEQ ID NO: 6, SEQ ID NO: 8, SEQ ID NO: 9, SEQ ID NO: 11, SEQ ID NO: 12 or SEQ ID NO: 35, preferably an antisense polynucleotide containing the entire or part of a base sequence complementary to a base sequence of DNA containing the base sequence represented by SEQ ID NO: 2, SEQ ID NO: 3, SEQ ID NO: 5, SEQ ID NO: 6, SEQ ID NO: 8, SEQ ID NO: 9, SEQ ID NO: 11, SEQ ID NO: 12 or SEQ ID NO: 35, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2, SEQ ID NO: 3, SEQ ID NO: 5, SEQ ID NO: 6, SEQ ID NO: 8, SEQ ID NO: 9, SEQ ID NO: 11, SEQ ID NO: 12 or SEQ ID NO: 35. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such an antisense polynucleotide is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating/controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and proteins usually refer to amino acids of a protein (under the order) derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense polynucleotide of the present invention can be modified preferably based on the following design, that is, by increasing the intracellular stability of the antisense polynucleotide, enhancing the cell permeability of the antisense polynucleotide, increasing the affinity of the nucleic acid to the targeted sense strand to a higher level, or minimizing the toxicity, if any, of the antisense polynucleotide. Most of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibition activity of the antisense polynucleotide can be investigated using the transformant of the present invention, the in vivo or in vitro gene expression system of the present invention, or the in vivo or in vitro translation system of the protein or receptor used in the present invention.

Applications of (a) the substance that inhibits the binding of the protein used in the present invention to the receptor used in the present invention, (b) the substance that inhibits the activities of the protein used in the present invention, (c) the substance that inhibits the activities of the receptor used in the present invention, (d) the antibody of the present invention, (e) the antisense polynucleotide of the present invention, etc. are described below.

### [1] Agent for preventing/treating cancer, agent for promoting the apoptosis in cancer cells and agent for inhibiting the growth of cancer cells

The protein used in the present invention is increasingly expressed in cancer tissues and bind to the receptor used in the present invention. Since the protein used in the present invention and the receptor used in the present invention are simultaneously expressed in cancer cells (e.g., human lung cancer cells, etc.), cell growth stimulation occurs in a self-contained way (autocrine growth stimulation) accompanied by an enhanced invasive capacity, which is induced by the receptor used in the present invention, and contributes to the progression/malignant alternation of cancer. The receptor used in the present invention is activated (e.g., phosphorylated) by the protein used in the present invention being bound to the receptor. In the mechanism of this activation, a tyrosine kinase like hepatocyte growth factor (HGF) receptor, etc. is involved. The phenomenon of cancer cell growth stimulation described above disappears by inhibiting, for example, (i) the binding of the protein used in the present invention to the receptor used in the present invention, (ii) the activities of the protein used in the present invention (e.g., the activity of inducing/promoting the phosphorylation of the receptor used in the present invention, the activity of binding the receptor used in the present invention, etc.), (iii) the induction of activation of the receptor used in the present invention (e.g., induction/promotion of the phosphorylated activation, etc.), or the like. Also, the antibody of the present invention and the antisense polynucleotide of the present invention possess the apoptosis inducing/promoting action and growth inhibiting action, etc. of cancer cells. The antibody of the present invention or the antisense polynucleotide of the present invention binds to the protein used in the present invention to inhibit the expression of said protein to suppress the growth of cancer cells and induce/promote apoptosis.

Accordingly, the pharmaceuticals comprising (a) the substance that inhibits the binding of the protein used in the present invention to the receptor used in the present invention, (b) the substance that inhibits the activities of the protein used in the present invention, (c) the substance that inhibits the activities of the receptor used in the present invention, (d) the antibody of the present invention (including its salts) or the antisense polynucleotide of the present invention, etc. can be used as low-toxic and safe pharmaceuticals such as agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), agents for promoting apoptosis in cancer cells, agents for inhibiting cancer cell growth, or the like.

The aforesaid agents comprising the antibody of the present invention or the substance described above are low-toxic and can be administered as they are in the form of liquid preparations, or as pharmaceutical compositions of suitable preparations to human or mammals (e.g., rats, rabbits, sheep, swine, bovine, feline, canine, simian, etc.) orally or parenterally (e.g., intravascularly, intraperitoneally, subcutaneously, etc.).

The antibody of the present invention or the substance described above may be administered in itself, or may be administered as an appropriate pharmaceutical composition. The pharmaceutical composition used for the administration may contain a pharmacologically acceptable carrier with the antibody of the present invention or the substance described above, a diluent or excipient. Such a pharmaceutical composition is provided in the form of pharmaceutical preparations suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the antibody of the present invention or the substance described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the antibody of the present invention or the substance described above with conventional bases for suppositories.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Each composition described above may further contain other active components unless formulation causes any adverse interaction with the antibody or the substance described above.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the antibody or the substance described above is contained in about 5 to about 100 mg especially in the form of injection, and in 10 to 250 mg for the other forms.

The dose of the agent described above may vary depending upon subject to be administered, target disease, conditions, route of administration, etc. For example, when used for the purpose of treating/preventing, e.g., breast cancer in adult, it is advantageous to administer the antibody or the substance of the present invention intravenously in a dose of about 0.01 to about 20 mg/kg body weight, preferably about 0.1 to about 10 mg/kg body weight and more preferably about 0.1 to about 5 mg/kg body weight, about 1 to 5 times/day, preferably about 1 to 3 times/day. In other parenteral and oral administration, the agent can be administered in a dose corresponding to the dose given above. When the condition is especially severe, the dose may be increased according to the condition.

Furthermore, the antibody of the present invention and the substance described above may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, etc. The antibody of the present invention or the substance described above may be administered simultaneously or at staggered times to the patient.

### [2] Quantification for the protein or receptor used in the present invention

The antibody of the present invention can be used to quantify the protein or receptor used in the present invention, or detect the protein or receptor by means of a tissue staining, etc. For these purposes, the antibody molecule per se may be used or F (ab')₂, Fab' or Fab fractions of the antibody molecule may also be used.

The quantification method using the antibody of the present invention is not particularly limited. Any quantification method may be used, so long as the amount of an antibody, antigen or antibody-antigen complex corresponding to the amount of antigen (e.g., the amount of the protein or receptor used in the present invention) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is preferred to use the sandwich method and the competitive method later described, particularly the sandwich method.

### (1) Sandwich method

In the sandwich method, the immobilized antibody of the present invention is reacted with a test fluid (primary reaction), then with a labeled form of another antibody of the present invention (secondary reaction), and the activity of the label on the immobilizing carrier is measured, whereby the amount of the protein or receptor used in the present invention in the test fluid can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity. That is, in the antibodies used for the primary and secondary reactions are, for example, when the antibody used in the secondary reaction recognizes the C-terminal region of the protein or receptor used in the present invention, it is preferable to use the antibody recognizing the region other than the C-terminal region for the primary reaction, e.g., the antibody recognizing the N-terminal region.

### (2) Competitive assay

The antibody of the present invention, a test fluid and a labeled form of the protein or receptor used in the present invention are competitively reacted, and a ratio of the labeled protein or receptor used in the present invention, is determined, thereby to quantify the protein or receptor used in the present invention in the test fluid.

The competitive assay is performed by, e.g., a solid phase technique.

Specifically, anti-mouse IgG antibody (manufactured by ICN/CAPPEL) is used as a solid phase antibody, (i) the antibody of the present invention, (ii) the protein or receptor used in the present invention, and (iii) a test fluid are added to a plate where the solid phase antibody is present; after the reaction, the HRP activity adsorbed onto the solid phase is assayed to quantify the protein or receptor used in the present invention.

### (3) Immunometric assay

In the immunometric assay, an antigen in a test fluid and a solid phase antigen are competitively reacted with a given amount of a labeled form of the antibody of the present invention followed by separating the solid phase from the liquid phase; or an antigen in a test fluid and an excess amount of labeled form of the antibody of the present invention are reacted, then a solid phase antigen is added to bind an unreacted labeled form of the antibody of the present invention to the solid phase and the solid phase is then separated from the liquid phase. Thereafter, the labeled amount in any of the phases is measured to determine the antigen level in the test fluid.

### (4) Nephrometry

In the nephrometry, the amount of insoluble sediment, which is produced as a result of the antigen-antibody reaction in a gel or in a solution, is measured. Even when the amount of an antigen in a test fluid is small and only a small amount of the sediment is obtained, a laser nephrometry utilizing laser scattering can be suitably used.

Examples of labeling agents, which are employed for the aforesaid assay methods (1) to (4) using labeling agents, are radioisotopes, enzymes, fluorescent substances, luminescent substances, lanthanides, etc. Examples of radioisotopes are [¹²⁵I], [¹³¹I], [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include cyanine fluorescent dyes (e.g., Cy2, Cy3, Cy5, Cy5.5, Cy7 (manufactured by Amersham Biosciences), etc.), fluorescamine, fluorescein isothiocyanate, etc. Examples of the luminescent substances are luminol, a luminol derivative, luciferin, lucigenin, etc. Furthermore, a biotin-avidin system may be used as well for binding an antibody or antigen to a labeling agent.

In the immobilization of antigens or antibodies, physical adsorption may be used. Alternatively, chemical binding that is conventionally used for immobilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

For applying each of these immunological methods to the quantification method of the present invention, any particular conditions or procedures are not required. For the details of these general technical means, reference can be made to the following reviews and texts. For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc. Thus, where the assay system of the present invention is constructed by applying the sandwich immunoassay method, etc., its method is not limited to EXAMPLES later described.

As described above, the antibody of the present invention can quantify the protein or receptor used in the present invention with high sensitivity, and is thus useful for further elucidation of the physiological functions of the protein or receptor used in the present invention and for diagnosis of diseases associated with the protein or receptor used in the present invention. Specifically, a level of the protein or receptor used in the present invention, which is contained in tissues or body fluids (blood, plasma, serum, urine, etc.) is determined using the antibody of the present invention, whereby diagnosis can be made on, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), or the like.

### [3] Screening of drug candidates for disease

The protein used in the present invention is increasingly expressed in cancer tissues and binds to the receptor used in the present invention. Since the protein used in the present invention and the receptor used in the present invention are simultaneously expressed in cancer cells (e.g., human lung cancer cells, etc.), cell growth stimulation occurs in a self-contained way (autocrine growth stimulation) accompanied by an enhanced invasive capacity, which is induced by the receptor used in the present invention, and contributes to the progression/malignant alternation of cancer. The receptor used in the present invention is activated (e.g., phosphorylated) by the protein used in the present invention being bound thereto. In the mechanism of this activation, a tyrosine kinase like hepatocyte growth factor (HGF) receptor, etc. is involved. The phenomenon of cancer cell growth stimulation described above disappears by inhibiting, for example, (i) binding of the protein used in the present invention to the receptor used in the present invention, (ii) the activities of the protein used in the present invention (e.g., the activity of inducing/promoting the phosphorylation of the receptor used in the present invention, the activity of binding the receptor used in the present invention, etc.), (iii) the induction of activation of the receptor used in the present invention (e.g., induction/promotion of the phosphorylated activation, etc.), or the like, resulting in cancer cell growth inhibition, whereby apoptosis is induced.

Therefore, the compounds or their salts that inhibit the activities of the protein or receptor used in the present invention are useful as agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), agents for promoting apoptosis in cancer cells, agents for inhibiting cancer cell growth, or the like.

Accordingly, the protein or receptor used in the present invention is useful as a reagent for screening the substance that inhibit the activities of the protein or receptor of the present invention.

That is, the present invention provides a method of screening the substance or its salts that inhibit the activities of the protein or receptor used in the present invention, which comprises using the protein or receptor used in the present invention.

In the method of screening the substance that inhibits the activities of the protein used in the present invention (e.g., the activity of binding the receptor used in the present invention, etc.), for example, the protein of the present invention and the receptor used in the present invention are expressed as tagged recombinant proteins, respectively, in animal cells. FLAG, His, V5, myc, HA, etc. are used as the tag. A tag (Tag A) employed to attach the tag to the protein of the present invention is different from a tag (Tag B) employed to attach the tag to the receptor used in the present invention. (i) A mixture of the aforesaid Tag A-tagged protein and the aforesaid Tag B-tagged receptor or (ii) a mixture of a test compound, the aforesaid Tag A-tagged protein and the aforesaid Tag B-tagged receptor is immunoprecipitated with an antibody to Tag B. By subjecting the precipitates obtained to the western blotting operations using an antibody to Tag A, the amount of the protein used in the present invention, which is bound to the receptor used in the present invention, is determined and comparison is made between (i) and (ii) described above.

In the method of screening the substance that inhibits the activities of the protein used in the present invention (e.g., the activity of inducing/promoting phosphorylation of the receptor used in the present invention, etc.), for example, the receptor used in the present invention, which is added with, for example, a tag (e.g., FLAG, His, V5, myc, HA, etc.) at the C-terminus, is expressed as a recombinant protein in animal cells. After separately reacting with (i) the protein used in the present invention or (ii) a test compound and the protein used in the present invention, the cells are disrupted to prepare a cell-free extract and immunoprecipitated with an anti-tag antibody. The amount of the resulting receptor used in the present invention, which has been phosphorylated, is determined by publicly known methods (e.g., western blotting, etc.) using an anti-phosphorylated tyrosine antibody, etc., and comparison is made between the cases (i) and (ii) described above. The phosphorylation inducing/promoting activity described above can be assayed by publicly known methods, e.g., the method described in Methods in Enzymology, 200, 98-107, 1991, or a modification of the method.

For example, when a test compound inhibits the activity of the protein used in the present invention in the case (ii) described above by at least about 20%, preferably at least 30% and more preferably at least about 50%, as compared to the case (i) above, the test compound can be selected to be a substance capable of inhibiting the activity of the protein used in the present invention.

In a specific example of the method of screening the substance that inhibits the activities of the receptor used in the present invention (e.g., the phosphorylated activity, etc.), for example, the receptor used in the present invention, which is tagged with, for example, a tag (e.g., FLAG, His, V5, myc, HA, etc.) at the C-terminus, is expressed as a recombinant protein in animal cells. After separately reacting with (i') the protein used in the present invention or (ii') the protein used in the present invention and a test compound, the cells are disrupted to prepare a cell-free extract and immunoprecipitated with an anti-tag antibody. The amount of the resulting receptor used in the present invention, which has been phosphorylated, is quantified by publicly known methods (e.g., western blotting, etc.) using an anti-phosphorylated tyrosine antibody, etc., and comparison is made between the cases (i') and (ii') described above.

For example, when a test compound inhibits the activity of the receptor used in the present invention in the case (ii') described above by at least about 20%, preferably at least 30% and more preferably at least about 50%, as compared to the case (i') above, the test compound can be selected to be a substance capable of inhibiting the activity of the receptor used in the present invention.

As the cells capable of producing the protein or receptor used in the present invention described above, there are used, for example, the aforesaid host (transformant) transformed with a vector containing the DNA encoding the protein or receptor used in the present invention. Preferably, animal cells such as COS7 cells, CHO cells, HEK293 cells, MCF-7 cells, etc. are used as the host. For the screening, the transformant, in which the protein of the present invention has been expressed in the cells, e.g., by culturing through the procedure described above, is preferably employed. The procedure for incubating the cells capable of expressing the protein of the present invention is similar to the incubation procedure for the transformant of the present invention described above.

Examples of the test compound include peptides, proteins, antibody, non-peptide compounds, synthetic compounds, fermentation products, cell extracts, plant extracts, animal tissue extracts, blood plasma, etc.

In addition, a gene for the protein used in the present invention is also increasingly expressed in cancer cells and a substance that inhibits the expression of the gene for the protein or receptor used in the present invention can also be used as agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), agents for promoting apoptosis in cancer cells, agents for inhibiting cancer cell growth, or the like.

Therefore, the polynucleotide (e.g., DNA) encoding the protein or receptor used in the present invention is useful as a reagent for screening the compound or its salts that inhibits the expression of the gene for the protein or receptor used in the present invention.

For the screening method, there is a method of screening which comprises comparing (iii) the case where a cell capable of producing the protein or receptor used in the present invention is incubated and (iv) the case where a cell capable of producing the protein or receptor used in the present invention is incubated in the presence of a test compound.

In the screening method described above, the expression level of the gene described above (specifically, the level of the protein or receptor used in the present invention or the level of mRNA encoding the protein or receptor used in the present invention) is determined, followed by comparison between the cases (iii) and (iv).

Examples of the test compound and the cells capable of producing the protein of the present invention are the same as described above.

The level of the protein can be determined by publicly known methods, e.g., by measuring the aforesaid protein present in the cell extract, etc., using the antibody of the present invention, in accordance with methods such as western blot analysis, ELISA, etc., or their modifications.

The mRNA level can be determined by publicly known methods, e.g., in accordance with methods such as Northern hybridization using a nucleic acid containing the entire or a part of SEQ ID NO: 2, SEQ ID NO: 5, SEQ ID NO: 8, SEQ ID NO: 11 or SEQ ID NO: 35 as a probe, or PCR using a nucleic acid containing the entire or a part of SEQ ID NO: 2, SEQ ID NO: 5, SEQ ID NO: 8, SEQ ID NO: 11 or SEQ ID NO: 35 as a primer, or modifications thereof.

For example, when a test compound inhibits the expression of the gene in the case (iv) described above by at least about 20%, preferably at least 30% and more preferably at least about 50%, as compared to the case (iii) above, the test compound can be selected to be a compound inhibiting the expression of the gene for the protein or receptor used in the present invention.

The screening kit of the present invention comprises the protein or receptor used in the present invention, or a cell capable of producing the protein or receptor used in the present invention.

The substance obtained by using the screening method or screening kit of the present invention is selected from the test compound described above, e.g., peptides, proteins, non-peptide compounds, synthetic compounds, fermentation products, cell extracts, plant extracts, animal tissue extracts, plasma, etc.

The salts used are those given above as the salts of the protein of the present invention.

Where the substance obtained by using the screening method or screening kit of the present invention is used as the agents described above, the substance can be converted into pharmaceutical preparations in a conventional manner.

Examples of the composition for oral or parenteral administration are the same as given in [1] above. Such a composition can be manufactured in a similar manner and used in a similar manner.

### [4] Gene diagnostic agent

By using the polynucleotide (e.g., DNA) encoding the protein or receptor used in the present invention, e.g., as a probe, an abnormality (gene abnormality) of the DNA or mRNA encoding the protein or receptor used in the present invention or its partial peptide in human or warm-blooded animal (e.g., rat, mouse, guinea pig, rabbit, fowl, sheep, swine, bovine, horse, feline, canine, simian, chimpanzee, etc.) can be detected. Therefore, the polynucleotide is useful as a gene diagnostic agent for detecting damages to the DNA or mRNA, its mutation, or decreased expression, increased expression, overexpression, etc. of the DNA or mRNA, and so on.

The gene diagnosis described above can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., Northern hybridization or DNA mutation is detected by the PCR-SSCP assay, it can be diagnosed that it is highly likely to suffer from, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.)

### [5] Pharmaceutical comprising the antisense polynucleotide

The antisense polynucleotide of the present invention is low toxic and can suppress the in vivo functions or actions of the protein or receptor used in the present invention or the DNA encoding the same to induce apoptosis in cancer cells. Thus, the antisense polynucleotide can also be used as agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), agents for promoting apoptosis in cancer cells, agents for inhibiting cancer cell growth, or the like.

Where the antisense polynucleotide described above is used as the aforesaid prophylactic/therapeutic agent or as the promoter, it can be prepared into pharmaceutical preparations by publicly known methods, which are provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or a mammal (e.g., rat, rabbit, sheep, swine, bovine, feline, canine, simian, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.

In addition, the antisense polynucleotide may also be used as an oligonucleotide probe for diagnosis to examine the presence of the DNA of the present invention in tissues or cells and states of its expression.

As the antisense polynucleotide described above can, the double-stranded RNA containing a part of RNA encoding the protein or receptor used in the present invention, ribozyme containing a part of RNA encoding the protein or receptor used in the present invention, etc. can also prevent expression of a gene for the protein or receptor used in the present invention to suppress the in vivo function of the protein or receptor used in the present invention or the DNA encoding the same and thus can be used as agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), agents for promoting apoptosis in cancer cells, agents for inhibiting cancer cell growth, or the like.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the agents described above, the double-stranded RNA or ribozyme is prepared into a pharmaceutical preparation as in the antisense polynucleotide, and the preparation can be provided for administration.

### [6] Pharmaceutical comprising the protein or receptor used in the present invention

Since the protein used in the present invention is overexpressed in cancer and the receptor used in the present invention is also expressed in cancer cells, the protein or receptor used in the present invention can be used as a cancer vaccine to activate the immune system in patients with cancer.

For example, the so-called adoptive immunotherapy, which involves culturing potent antigen presenting cells (e.g., dendritic cells) in the presence of the protein or receptor used in the present invention to engulf the protein and putting the cells back into the body, can preferably be used. The dendritic cells, returned back into the body, can induce and activate cytotoxic T cells specific to a cancer antigen whereby to kill cancer cells.

The protein or receptor of the present invention can also be administered to a mammal (e.g. human, simian, mouse, rat, rabbit, swine) safely as a vaccine preparation to prevent or treat a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.)

The vaccine preparation usually contains the protein or receptor used in the present invention and a physiologically acceptable carrier. Such a carrier includes a liquid carrier such as water, saline (including physiological saline), buffer (e.g., phosphate buffer), an alcohol (e.g., ethanol), etc.

The vaccine preparation can be prepared according to a conventional method of manufacturing a vaccine preparation.

In general, the protein or receptor used in the present invention is dissolved or suspended in a physiologically acceptable carrier. Alternatively, the protein or receptor used in the present invention and the physiologically acceptable carrier may be separately prepared and then mixed at use.

The vaccine preparation may be further formulated with, for example, an adjuvant (e.g., aluminum hydroxide gel, serum albumin, etc.), a preservative (e.g., thimerosal, etc.), a soothing agent (e.g., glucose, benzyl alcohol, etc.), in addition to the protein or receptor used in the present invention and the physiologically acceptable carrier. Furthermore, the vaccine preparation may also be formulated with, for example, a cytokine (e.g., an interleukin such as interleukin-2, an interferon such as interferon-y) to enhance the production of the antibody to the protein or receptor used in the present invention.

When used as a vaccine preparation, the protein or receptor used in the present invention may be used in its active form, or may be denatured to enhance the antigenicity. The protein or receptor may be denatured usually by heating or treating with a protein-denaturing agent (e.g., formalin, guanidine hydrochloride and urea).

The thus obtained vaccine preparation is low toxic and may usually be administered in an injectable form, e.g., subcutaneously, intracutaneously, intramuscularly, or topically into or near a mass of cancer cells.

The dose of the protein or receptor used in the present invention varies depending on a target disease, a subject to be administered, a route for administration, etc. For example, for subcutaneous administration of the protein or receptor used in the present invention to an adult cancer patient (60 kg body weight) in an injectable form, the single dose is normally about 0.1 mg to about 300 mg, preferably about 100 mg to about 300 mg. The administration of the vaccine preparation may be carried out once, or 2 to 4 times in total approximately in every 2 weeks to 6 months to increase the production of the antibody.

### [7] DNA transgenic animal

The present invention provides a non-human mammal bearing a DNA encoding the protein or receptor used in the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, canine, feline, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, canine, feline, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, canine, feline, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

It is possible to obtain homozygous animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening test of agents for preventing/treating diseases associated with the protein of the present invention, for example, agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows an increased symptom of the protein of the present invention liberated, the animal is also expected to serve for screening test of agents preventing/treating the function inactive type inadaptability of the protein of the present invention, for example, agents for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

Other potential applications of two kinds of the DNA transgenic animals of the present invention described above further include:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually cultured only with difficulty, using cells in tissues bearing the DNA cultured by a standard tissue culture technique;
(iv) Screening a drug that enhances the functions of cells using the cells described in
(iii) above; and,
(v) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### [8] Knockout animal

The present invention provides a non-human mammal embryonic stem cell bearing the DNA encoding the protein or receptor used in the present invention (DNA of the present invention) inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli*);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from *Escherichia coli)* therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. Besides, embryos are preferably collected at the 8-cell stage after culturing until the blastocyte stage and the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony ofES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities derived from the protein of the present invention, such an animal can be a disease model suspected of inactivated biological activities of the protein of the present invention and thus, offers an effective study to investigate the causes for and therapy for these diseases.

### [8a] Method of screening the compound having a therapeutic/prophylactic effect on diseases caused by deficiency, damages, etc. of the DNA of the present invention

The non-human mammal deficient in expression of the DNA of the present invention can be employed for screening the compound having a therapeutic/prophylactic effect on diseases caused by deficiency, damages, etc. of the DNA of the present invention.

That is, the present invention provides a method of screening the compound having a therapeutic/prophylactic effect on diseases, e.g., cancer, caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

For screening of the compound having a therapeutic/prophylactic effect on a cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc., a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having the therapeutic/prophylactic effect on the diseases described above.

For screening of the compound having a therapeutic/prophylactic effect on a cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc., a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal, the test compound can be selected as a compound having therapeutic/prophylactic effects against the above-mentioned diseases, if the symptoms described above is relieved up about 10%, preferably up about 30%, more preferably up about 50%.

Since the compound obtained using the screening method is a compound selected from the above compounds, and have therapeutic/prophylactic effects against diseases caused by deficiency and/or disorder of the protein of the present invention, it can be used as a safe and low toxic medicament such as a prophylactic/therapeutic agent for the disease. Further, a compound derived from the compound that is obtained from the above-mentioned screening can also be used.

The compound obtained by the screening method above may form salts, and may be used in the form of salts with physiologically acceptable acids (e.g., inorganic acids, organic acids, etc.) or bases (e.g., alkali metals, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

The dose of the compound or its salt may vary depending upon target disease, subject to be administered, route of administration, etc. For example, when the compound is orally administered, the compound is administered to the adult patient with breast cancer (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with breast cancer (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### [8b] Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that inhibit the promoter activity to the DNA of the present invention can inhibit the expression of the protein of the present invention to inhibit the functions of the protein. Thus, the compound or its salts are useful as agents for preventing/treating cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.).

In addition, compounds derived from the compound obtained by the screening described above may be used as well.

A pharmaceutical comprising the compound obtained by the above screening method or salts thereof can be manufactured in a manner similar to the method for preparing the pharmaceutical comprising the protein of the present invention described above.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

A dose of the compound or salts thereof may vary depending on target disease, subject to be administered, route for administration, etc.; when the compound that inhibits the promoter activity to the DNA of the present invention is orally administered, the compound is administered to the adult patient (as 60 kg body weight) with breast cancer normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the adult patient (as 60 kg body weight) with breast cancer in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification, where bases, amino acids, etc. are denoted by their codes, they are based on conventional codes in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid
- Sec :: selenocysteine

Substituents, protecting groups and reagents frequently used in this specification are presented by the codes described below.
- Me :: methyl group
- Et :: ethyl group
- Bu :: butyl group
- Ph :: phenyl group
- TC :: thiazolidine-4(R)-carboxamido group
- Tos :: p-toluenesulfonyl
- CHO :: formyl
- Bzl :: benzyl
- Cl₂-Bzl :: 2,6-dichlorobenzyl
- Bom :: benzyloxymethyl
- Z :: benzyloxycarbonyl
- Cl-Z :: 2-chlorobenzyloxycarbonyl
- Br-Z :: 2-bromobenzyl oxycarbonyl
- Boc :: t-butoxycarbonyl
- DNP :: dinitrophenol
- Trt :: trityl
- Bum :: t-butoxymethyl
- Fmoc :: N-9-fluorenyl methoxycarbonyl
- HOBt: : 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC :: N,N'-dicyclohexylcarbodiimide

The sequence identification numbers in the sequence listing of the specification indicate the following sequences.

### [SEQ ID NO: 1]

This shows the amino acid sequence of SEMA4B.

### [SEQ ID NO: 2]

This shows the base sequence of DNA encoding SEMA4B having the amino acid sequence represented by SEQ ID NO: 1.

### [SEQ ID NO: 3]

This shows the base sequence of DNA containing the full-length gene encoding SEMA4B.

### [SEQ ID NO: 4]

This shows the amino acid sequence of SEMA4B-M1.

### [SEQ ID NO: 5]

This shows the base sequence of DNA encoding SEMA4B-M1 having the amino acid sequence represented by SEQ ID NO: 4.

### [SEQ ID NO: 6]

This shows the base sequence of DNA containing the full-length gene encoding SEMA4B-M1.

### [SEQ ID NO: 7]

This shows the amino acid sequence of SEMA4B-M2.

### [SEQ ID NO: 8]

This shows the base sequence of DNA encoding SEMA4B-M2 having the amino acid sequence represented by SEQ ID NO: 7.

### [SEQ ID NO: 9]

This shows the base sequence of DNA containing the full-length gene encoding SEMA4B-M2.

### [SEQ ID NO: 10]

This shows the amino acid sequence of SEMA4B-M3.

### [SEQ ID NO: 11]

This shows the base sequence of DNA encoding SEMA4B-M3 having the amino acid sequence represented by SEQ ID NO: 10.

### [SEQ ID NO: 12]

This shows the base sequence of DNA containing the full-length gene encoding SEMA4B-M3.

### [SEQ ID NO: 13]

This shows the base sequence of the antisense oligonucleotide used in REFERENCE EXAMPLES 2, 3, 15 and 16.

### [SEQ ID NO: 14]

This shows the base sequence of the oligonucleotide used in REFERENCE EXAMPLES 2, 3, 15 and 16.

### [SEQIDNO: 15]

This shows the base sequence of the antisense oligonucleotide used in REFERENCE EXAMPLE 3.

### [SEQ ID NO: 16]

This shows the base sequence of the oligonucleotide used in REFERENCE EXAMPLE 3

### [SEQIDNO: 17]

This shows the base sequence of the primer used in REFERENCE EXAMPLE 3.

### [SEQ ID NO: 18]

This shows the base sequence of the primer used in REFERENCE EXAMPLE 3.

### [SEQ ID NO: 19]

This shows the base sequence of the primer used in REFERENCE EXAMPLES 4, 6 and 7.

### [SEQ ID NO: 20]

This shows the base sequence of the primer used in REFERENCE EXAMPLES 4 and 7.

### [SEQ ID NO: 21]

This shows the base sequence of the primer used in REFERENCE EXAMPLE 6.

### [SEQ ID NO: 22]

This shows the amino acid sequence of peptide 1 used in REFERENCE EXAMPLE 8.

### [SEQ ID NO: 23]

This shows the amino acid sequence of peptide 2 used in REFERENCE EXAMPLE 8.

### [SEQ ID NO: 24]

This shows the amino acid sequence of peptide 3 used in REFERENCE EXAMPLE 8.

### [SEQ ID NO: 25]

This shows the amino acid sequence of peptide 4 used in REFERENCE EXAMPLE 8.

### [SEQ ID NO: 26]

This shows the amino acid sequence of Plexin B1.

### [SEQ ID NO: 27]

This shows the base sequence of the primer used in EXAMPLE 1.

### [SEQ ID NO: 28]

This shows the base sequence of the primer used in EXAMPLE 1.

### [SEQ ID NO: 29]

This shows the base sequence of the primer used in EXAMPLE 1.

### [SEQ ID NO: 30]

This shows the base sequence of the primer used in EXAMPLE 1.

### [SEQ ID NO: 31]

This shows the base sequence of the primer used in EXAMPLE 1.

### [SEQ ID NO: 32]

This shows the base sequence of the primer used in EXAMPLE 1.

### [SEQ ID NO: 33]

This shows the base sequence of the primer used in EXAMPLE 1.

### [SEQ ID NO: 34]

This shows the base sequence of the primer used in EXAMPLE 1.

### [SEQ ID NO: 35]

This shows the base sequence of DNA containing the full-length gene encoding Plexin B 1.

### [SEQ ID NO: 36]

This shows the base sequence of the primer used in EXAMPLE 3.

### [SEQ ID NO: 37]

This shows the base sequence of the primer used in EXAMPLE 3.

### [SEQ ID NO: 38]

This shows the base sequence of the primer used in EXAMPLE 3.

The transformant, Escherichia coli TOP10/SEMA4B-M1/pCR4-TOPO obtained in REFERENCE EXAMPLE 4 later described has been on deposit since March 4, 2003 under the Accession Number FERM BP-8316 at the National Institute of Advanced Industrial Science and Technology, International Patent Organism Depositary, located at Central 6, 1-1-1 Higashi, Tsukuba, Ibaraki, Japan (postal code 305-8566).

The transformant, Escherichia coli TOP 10/SEMA4B-M2/pCR4-TOPO obtained in REFERENCE EXAMPLE 4 later described has been on deposit since March 4, 2003 under the Accession Number FERM BP-8317 at the National Institute of Advanced Industrial Science and Technology, International Patent Organism Depositary, located at Central 6, 1-1-1 Higashi, Tsukuba, Ibaraki, Japan (postal code 305-8566).

The transformant, Escherichia coli TOP10/SEMA4B-M3/pCR4-TOPO obtained in REFERENCE EXAMPLE 4 later described has been on deposit since March 4, 2003 under the Accession Number FERM BP-8318 at the National Institute of Advanced Industrial Science and Technology, International Patent Organism Depositary, located at Central 6, 1-1-1 Higashi, Tsukuba, Ibaraki, Japan (postal code 305-8566).

Hereinafter, the present invention is described in more detail with reference to REFERENCE EXAMPLES and EXAMPLES, but is not deemed to limit the scope of the present invention thereto.

### REFERENCE EXAMPLE 1

### Gene expression analysis

In order to clarify the gene group in which its expression was specifically increased in the lung cancer tissue, the total RNAs (TABLE 1) extracted from 4 lung cancer tissues and 5 normal lung tissues were used as materials and gene expression analysis was made using oligonucleotide microarray (Human Genome U95A, U95B, U95C, U95D, U95E; Affymetrix Inc.). The experiment was carried out following the expression analysis technical manual ofAffymetrix Inc.

As a result, the expression of Semaphorin 4B (SEMA4B) and Semaphorin 4B-M1 (SEMA4B-M1), Semaphorin 4B-M2 (SEMA4B-M2) and Semaphorin 4B-M3 (SEMA4B-M3) genes later described in REFEENCE EXAMPLE 4 were detected in the 3 lung cancer tissues (lot. 0011-192-01285, lot. 0011-192-01293 and lot. 0011-192-01297) (TABLE 2).

**[TABLE 1]**

| RNA-Extracted Tissue | Distribution Source |
|---|---|
| Lung cancer tissue (lot. 0009-192-00122) | BioClinical Partners, Inc. |
| Lung cancer tissue (lot. 0011-192-01285) | BioClinical Partners, Inc. |
| Lung cancer tissue (lot. 0011-192-01293) | BioClinical Partners, Inc. |
| Lung cancer tissue (lot. 0011-192-01297) | BioClinical Partners, Inc. |
| Normal lung tissue (lot. 0009-192-00150) | BioClinical Partners, Inc. |
| Normal lung tissue (lot. 0009-192-00168) | BioClinical Partners, Inc. |
| Normal lung tissue (lot. 0011-192-01283) | BioClinical Partners, Inc. |
| Normal lung tissue (lot. 0011-192-01285) | BioClinical Partners, Inc. |
| Normal lung tissue (lot. 0011-192-01297) | BioClinical Partners, Inc. |

**[TABLE 2]**

| Tissue | Gene Expression Level |
|---|---|
| Lung cancer tissue (lot. 0009-192-00122) | ND |
| Lung cancer tissue (lot. 0011-192-01285) | 10 |
| Lung cancer tissue (lot. 0011-192-01293) | 9.5 |
| Lung cancer tissue (lot. 0011-192-01297) | 1.9 |
| Normal lung tissue (lot. 0009-192-00150) | ND |
| Normal lung tissue (lot. 0009-192-00168) | ND |
| Normal lung tissue (lot. 0011-192-01283) | ND |
| Normal lung tissue (lot. 0011-192-01285) | ND |
| Normal lung tissue (lot. 0011-192-01297) | ND |

The gene expression level was standardized by taking as 1 the median value in the expression levels of all genes that the expression was detected with the oligonucleotide microarray.
ND: not detected

### REFERENCE EXAMPLE 2

### Induction of apoptosis in human lung cancer cell line

By inhibiting the expression of SEMA4B and SEMA4B-M1, SEMA4B-M2 and SEMA4B-M3 genes later described in REFERENCE EXAMPLE 4, it was examined if apoptosis in the human lung cancer cell line would be induced.

First, human non-small-cell lung cancer cell line NCI-H1703, purchased from American Type Culture Collection (ATCC), was suspended in RPMI-1640 medium (containing 25 mM HEPES) (Invitrogen Corp.) supplemented with 10% fetal bovine serum (ATCC) and the cells were plated on a 96-well flat bottomed tissue culture plate (BD Falcon) at a cell density of 10,000 cells/well (0.1 ml of a culture medium volume), and then incubated overnight at 37°C in a 5% carbon dioxide gas flow, followed by transfection of the antisense oligonucleotide.

Specifically, after an antisense oligonucleotide sequence (SEQ ID NO: 13) hybridizable to the 3' untranslated region of the proteins having the amino acid sequences represented by SEQ ID NO: 1, SEQ ID NO: 4, SEQ ID NO: 7 and SEQ ID NO: 10 was designed, the phosphorothioated oligonucleotide was synthesized, purified on HPLC and provided for use in transfection experiment (hereinafter merely referred to as the antisense oligonucleotide). For control, a reverse sequence (SEQ ID NO: 14) of the base sequence represented by SEQ ID NO: 13 was similarly phosphorothioated and purified on HPLC, which was provided for use (hereinafter merely referred to as the control oligonucleotide).

The antisense oligonucleotide or the control oligonucleotide diluted in Opti-MEM (Invitrogen), was mixed with OligofectAMINE (Invitrogen) diluted to 5-fold with Opti-MEM (Invitrogen), followed by allowing to stand at room temperature for 5 minutes, in a 8:3 ratio (by volume). The mixture was then added to the plate in 40 µL/well. The final concentration of the oligonucleotide was adjusted to become 250 nM. After incubation was continued for further 3 days under the conditions described above, the apoptosis induction activity of the two oligonucleotides above was assayed with Cell Death Detection ELISA^{PLUS} Kit (Roche Diagnostics) in accordance with the protocol attached thereto.

The results revealed that the antisense oligonucleotide (SEQ ID NO: 13) showed the apoptosis induction activity of approximately 1.6 times higher than the control oligonucleotide (SEQ ID NO: 14), indicating that there was a statistically significant difference (P≦0.01) (TABLE 3).

**[TABLE 3]**

| | Apoptosis Induction Activity (A₄₀₅-A₄₉₂) | |
|---|---|---|
| | Mean Value | Standard Deviation |
| Blank | 0.212 | 0.032 |
| Control oligonucleotide (SEQ ID NO: 14) | 0.410 | 0.017 |
| Antisense oligonucleotide (SEQ ID NO: 13) | 0.538 | 0.035 |

### REFERENCE EXAMPLE 3

### Reduction in gene expression level by SEMA4B antisense oligonucleotide

It was examined if the gene expression levels of SEMA4B and SEMA4B-M1, SEMA4B-M2 and SEMA4B-M3 later described in REFERENCE EXAMPLE 4 were reduced by providing the antisense oligonucleotide.

Human non-small-cell lung cancer cell line NCI-H1703 used in REFERENCE EXAMPLE 2 was suspended in the same medium as in REFERENCE EXAMPLE 2, and plated on a 24-well flat bottomed tissue culture plate (BD Falcon) at a cell density of 60,000 cells/well (0.6 ml of a culture medium volume). The cells were incubated overnight at 37°C in a 5% carbon dioxide gas flow, followed by transfection of the antisense oligonucleotide as in REFERENCE EXAMPLE 2, except that a volume of the oligonucleotide added was made 240 µL/well and two (SEQ ID NO: 13 and SEQ ID NO: 15) as antisense oligonucleotides and two oligonucleotides (SEQ ID NO: 14 and SEQ ID NO: 16) for control were used.

With respect to the antisense oligonucleotide and the control oligonucleotide derived from SEQ ID NO: 15 and SEQ ID NO: 16, the antisense oligonucleotide sequence (SEQ ID NO: 15) hybridizable to the 3' untranslated region of the proteins having the amino acid sequences represented by SEQ ID NO: 1, SEQ ID NO: 4, SEQ ID NO: 7 and SEQ ID NO: 10 was designed and then the phosphorothioated oligonucleotide was synthesized and purified on HPLC, which was provided for use in transfection experiment. Reverse sequence (SEQ ID NO: 16) of the base sequence represented by SEQ ID NO: 15 was similarly phosphorothioated and purified on HPLC, which was provided for use.

Following the transfection, incubation was continued at 37°C for 24 hours in a 5% carbon dioxide gas flow and the total RNA was extracted by RNeasy® Mini Total RNA Kit (QIAGEN). Using as a template about 300 ng of the total RNA, reverse transcription was carried out on TaqMan Reverse Transcription Reagents (Applied Biosystems) in accordance with the protocol attached thereto. Using as a template cDNA in an amount corresponding to 7-9 ng when converted into the total RNA, the number of copies of the expressed SEMA4B, SEMA4B-M1, SEMA4B-M2 and SEMA4B-M3 genes were determined using two primers (SEQ ID NO: 17 and SEQ ID NO: 18) and SYBR Green PCR Master Mix (Applied Biosystems). The expression level of a gene for β-actin contained in the same amount of template cDNA was assayed on TaqMan β-actin Control Reagents (Applied Biosystems), which was used as an internal standard.

Where distilled water was used instead of the oligonucleotide solution (hereinafter merely referred to as non-transfection group), the total gene expression level of the SEMA4B, SEMA4B-M1, SEMA4B-M2 and SEMA4B-M3 was 6.6% of the gene expression level of β-actin, whereas the expression levels were 0.98% and 1.1% in the groups given with the antisense oligonucleotides (SEQ ID NO: 13 and SEQ ID NO: 15), indicating that a statistically significant reduction in the gene expression level was observed (P ≦ 0.05).

On the other hand, the expression levels were 4.1% and 3.4% in the groups given with the control oligonucleotides (SEQ ID NO: 14 and SEQ ID NO: 16), indicating that any statistically significant reduction in the expression level was not observed when compared to the non-transfection group.

These results reveal that the inhibition of gene expression of SEMA4B, SEMA4B-M1, SEMA4B-M2 and SEMA4B-M3 was correlated to apoptosis induction.

### REFERENCE EXAMPLE 4

### Cloning and base sequencing of cDNAs encoding SEMA4B, SEMA4B-M1, SEMA4B-M2 and SEMA4B-M3

Using Human Lung Cancer Cell Line (A549)-derived Marathon-Ready cDNA (CLONTECH) as a template, PCR was carried out by using two primers (SEQ ID NO: 19 and SEQ ID NO: 20). For PCR, 50 µl of the reaction solution was prepared to have a composition containing 1 µl of the cDNA above, 2.5 U of PfuTurbo Hotstart DNA Polymerase (STRATAGENE), 1.0 µM each of the primers (SEQ ID NO: 19 and SEQ ID NO: 20), 200 µM of dNTPs and 25 µl of 2 x GC Buffer I (Takara Bio) in the solution. PCR was carried out by reacting at 95°C for 1 minute and then repeating 30 times the cycle set to include 95°C for 1 minute, 60°C for 1 minute and 72°C for 4 minutes, and elongation was further carried out at 72°C for 5 minutes. In order to add dATP to the PCR product at the 3' end, 5U of Ex Taq DNA Polymerase (Takara Bio) was added thereto, which was then kept warm at 72°C for 7 minutes. The PCR product obtained was purified using PCR Purification Kit (QIAGEN). The purified product was subcloned to plasmid vector pCR4-TOPO (Invitrogen) according to the protocol of TOPO TA PCR Cloning Kit (Invitrogen). The clones were transfected to Escherichia coli TOP10 and the clones bearing cDNA were selected in ampicillin-containing LB agar medium. The sequences of individual clones were analyzed to give the base sequences of cDNAs represented by SEQ ID NO: 2, SEQ ID NO: 5, SEQ ID NO: 8 and SEQ ID NO: 11, respectively.

The 1st-237th and 2749th-3766th base sequences in the base sequence for the SEMA4B gene (GenBank Accession No. XM_044533 gene; NM_198925 gene; NM_020210 gene) were added to the 5' and 3' ends of the base sequences represented by SEQ ID NO: 2, SEQ ID NO: 5, SEQ ID NO: 8 and SEQ ID NO: 11, which are shown by SEQ ID NO: 3, SEQ ID NO: 6, SEQ ID NO: 9 and SEQ ID NO: 12, respectively.

The amino acid sequence (SEQ ID NO: 1) encoded by the base sequence represented by SEQ ID NO: 2 fully coincided with the SEMA4B protein encoded by the SEMA4B gene (GenBank Accession No. XM_044533 gene; NM_198925 gene; NM_020210gene).

The protein having the amino acid sequence (SEQ ID NO: 4) encoded by the base sequence represented by SEQ ID NO: 5 was named SEMA4B-M1; the protein having the amino acid sequence (SEQ ID NO: 7) encoded by the base sequence represented by SEQ ID NO: 8 was named SEMA4B-M2; and the protein having the amino acid sequence (SEQ ID NO: 10) encoded by the base sequence represented by SEQ ID NO: 11 was named SEMA4B-M3, respectively.

The amino acid sequence (SEQ ID NO: 4) of SEMA4B-M1 is the amino acid sequence (SEQ ID NO: 1) of SEMA4B in which the 208th Ser is replaced by Ile.

In the base sequence (SEQ ID NO: 5) of SEMA4B-M1-encoding DNA, the 90th g is replaced by a, the 111th g by a and the 623rd g by t, respectively, and the 623rd replacement is accompanied by amino acid substitution, in the base sequence (SEQ ID NO: 2) of SEMA4B-encoding DNA.

The amino acid sequence (SEQ ID NO: 7) of SEMA4B-M2 is the amino acid sequence (SEQ ID NO: 1) of SEMA4B in which the 163rd Met is replaced by Ile.

In the base sequence (SEQ ID NO: 8) of SEMA4B-M2-encoding DNA, the 150th g is replaced by a, the 489th g by a, the 528th c by t, the 1266th t by c, the 1588th c by a and the 2343rd a by g, respectively, and the 489th replacement is accompanied by amino acid substitution, in the base sequence (SEQ ID NO: 2) of SEMA4B-encoding DNA.

The amino acid sequence (SEQ ID NO: 10) of SEMA4B-M3 is the amino acid sequence (SEQ ID NO: 1) of SEMA4B in which the 364th Lys is replaced by Asn.

In the base sequence (SEQ ID NO: 11) of SEMA4B-M3-encoding DNA, the 1092nd g is replaced by t and this replacement is accompanied by amino acid substitution in the base sequence (SEQ ID NO: 2) of SEMA4B-encoding DNA.

The plasmid bearing DNA having the base sequence represented by SEQ ID NO: 2, the plasmid bearing DNA having the base sequence represented by SEQ ID NO: 5, the plasmid bearing DNA having the base sequence represented by SEQ ID NO: 8 and the plasmid bearing DNA having the base sequence represented by SEQ ID NO: 11 were named SEMA4B/pCR4-TOPO, SEMA4B-M1/pCR4-TOPO, SEMA4B-M2/pCR4-TOPO and SEMA4B-M3/pCR4-TOPO, respectively.

Furthermore, the transformant with plasmid SEMA4B/pCR4-TOPO introduced, the transformant with plasmid TOP10/SEMA4B-M1/pCR4-TOPO introduced, the transformant with plasmid TOP10/SEMA4B-M2/pCR4-TOPO introduced and the transformant with plasmid TOP10/SEMA4B-M3/pCR4-TOPO introduced were named Escherichia coli TOP10/SEMA4B/pCR4-TOPO, Escherichia coli TOP10/SEMA4B-M1/pCR4-TOPO, Escherichia coli TOP10/SEMA4B-M2/pCR4-TOPO and Escherichia coli TOP 10/SEMA4B-M3/pCR4-TOPO, respectively.

### REFERENCE EXAMPLE 5

### Study of gene expression level in human culture cell line

The 86 strains used below were purchased from ATCC: brain tumor cell lines SK-N-MC, SK-N-AS, SK-N-BE, SK-N-DZ, SK-N-FI, SK-N-SH, D341Med, Daoy, DBTRG-05MG, U-118 MG, U-87 MG, CCF-STTG1 and SW 1088; human breast cancer cell lines HCC1937, ZR-75-1, AU565, MCF-7 and MDA-MB-231; human colon cancer cell lines Caco-2, COLO201, COLO 205, COLO 320DM, HCT-8, HT-29, LoVo, LS123, SNU-C1, SK-CO-1, SW 403, SW 48, SW480, SW 620, SW 837 and SW 948; human embryonic kidney cell line HEK293; human small cell lung cancer cell lines NCI-H187, NCI-H378, NCI-H526, NCI-H889, NCI-H1672, NCI-H1836, NCI-H2227, NCI-N417 and SHP-77; human non-small cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1417, NCI-H1435, NCI-H1581, NCI-H1651, NCI-H1703, NCI-H1793, NCI-H1963, NCI-H2073, NCI-H2085, NCI-H2106, NCI-H2228, NCI-H2342 and NCI-H2347; human ovarian cancer cell lines ES-2, Caov-3, MDAH2774, NIH:OVCAR3, OV-90, SK-OV-3, TOV-112D and TOV-21G; human pancreas cancer cell lines PANC-1, MIA-PaCa-2, AsPC-1, BxPC-3, Capan-1 and Capan-2; human prostate cancer cell line DU145; human retinoblastoma cell lines WERI-Rb-1 and Y79; and human testicular cancer cell line Cates-1B. Human normal small airway epithelial cells SAEC and human normal prostate epithelial cells HPrEC were purchased from Clonetics. Human colon cancer cell line COCM1, human non-small cell lung cancer cell line VMRC-LCD and human prostate cancer cell line PC3 were purchased from JCRB. These cell lines were used not only in REFERENCE EXAMPLE 9 but also in subsequent REFERENCE EXAMPLES.

Total RNA was prepared from the 91 cell lines described above using RNeasy Mini Total RNA Kit (QIAGEN). Reverse transcription was performed on the total RNA as a template using a random primer to prepare cDNA. Then, quantitative PCR was carried out to examine the expression levels of SEMA4B gene (SEQ ID NO: 2), SEMA4B-M1 gene (SEQ ID NO: 5), SEMA4B-M2 gene (SEQ ID NO: 8) and SEMA4B-M3 gene (SEQ ID NO: 11).

The PCR described above was carried out under the same conditions as in REFERENCE EXAMPLE 3, using cDNA obtained from 3-4 ng of the total RNA described above as the template to determine the copy number of the SEMA4B, SEMA4B-M1, SEMA4B-M2 and SEMA4B-M3 genes. In parallel, the copy number of the gene for β-actin contained in 1 ng of the total RNA above was calculated using TaqMan™ Human β-actin Control Reagents (Applied Biosystems) and provided for use as an internal standard.

A relative expression rate obtained by normalizing the total gene expression level with the gene expression level of β-actin is shown in TABLE 4.

The cancer cell lines in which the total gene expression level exceeds 1% of the gene expression level of β-actin were found to be 17 strains, indicating that enhanced expression of the genes above was noted in the cancer cell lines.

**[TABLE 4]**

| Cell Line | % of β-actin | Cell Line | % of β-actin | Cell Line | % of β-actin |
|---|---|---|---|---|---|
| SK-N-MC | 0.02 | COLO 201 | 0.66 | NCI-H889 | 0.07 |
| SK-N-AS | 0.07 | COLO 205 | 0.40 | NCI-H1672 | 0.10 |
| SK-N-BE | 0.04 | COLO 320DM | 0.12 | NCI-H1836 | 0.08 |
| SK-N-DZ | 0.05 | HCT-8 | 0.36 | NCI-H2227 | 0.15 |
| SK-N-FI | 0.20 | HT-29 | 0.52 | NCI-N417 | 0.04 |
| SK-N-SH | 0.11 | LoVo | 0.58 | SHP-77 | 0.16 |
| D341 Med | 0.05 | LS123 | 0.04 | A549 | 0.35 |
| Daoy | 0.08 | SNU-C1 | 0.52 | NCI-H23 | 0.98 |
| DBTRG-05MG | 0.01 | SK-CO-1 | 0.45 | NCI-H226 | 0.04 |
| U-118 MG | 0.01 | SW 403 | 0.31 | NCI-H358 | 1.09 |
| U-87 MG | 0.20 | SW 48 | 0.06 | NCI-H460 | 0.08 |
| CCF-STTG1 | 0.23 | SW 480 | 0.03 | NCI-H522 | 0.05 |
| SW 1088 | 0.06 | SW 620 | 0.12 | NCI-H661 | 0.05 |
| HCC1937 | 0.17 | SW 837 | 0.59 | NCI-H810 | 0.03 |
| ZR-75-1 | 0.30 | SW 948 | 0.18 | NCI-H1155 | 0.07 |
| AU565 | 0.06 | HEK293 | 0.05 | NCI-H1299 | 0.10 |
| MCF-7 | 0.06 | SAEC | 1.73 | NCI-H1395 | 0.39 |
| MDA-MB-231 | 0.06 | NCI-H187 | 0.38 | NCI-H1417 | 0.21 |
| Caco-2 | 0.04 | NCI-H378 | 0.17 | NCI-H1435 | 0.26 |
| COCM1 | 0.10 | NCI-H526 | 0.14 | NCI-H1581 | 0.16 |
| NCI-H1651 | 1.03 | ES-2 | 0.02 | BxPC-3 | 0.17 |
| NCI-H1703 | 0.21 | Caov-3 | 0.13 | Capan-1 | 0.07 |
| NCI-H1793 | 0.29 | MDAH2774 | 0.37 | Capan-2 | 0.27 |
| NCI-H1963 | 0.12 | NIH:OVCAR3 | 0.14 | HPrEC | 2.87 |
| NCI-H2073 | 0.15 | OV-90 | 0.23 | DU 145 | 3.05 |
| NCI-H2085 | 0.02 | SK-OV | 2.44 | PC3 | 0.43 |
| NCI-H2106 | 0.07 | TOV-112D | 0.06 | WERI-Rb-1 | 0.90 |
| NCI-H2228 | 1.89 | TOV-21G | 1.00 | Y79 | 0.06 |
| NCI-H2342 | 0.18 | PANC-1 | 1.88 | Cates-1B | 0.01 |
| NCI-H2347 | 0.24 | MIA-PaCa-2 | 0.02 | | |
| VMRC-LCD | 0.09 | AsPC-1 | 0.24 | | |

### REFERENCE EXAMPLE 6

### Construction of animal cell expression vectors for recombinant full-length protein

Using the plasmid SEMA4B/pCR4-TOPO obtained in REFERENCE EXAMPLE 4 as a template, the SEMA4B gene was amplified by PCR. In the reaction solution for PCR, 2 ng of SEMA4B/pCR4-TOPO was used as a template; 2.5 U of PfuTurbo Hotstart DNA Polymerase (STRATAGENE), 1 µM each of the two primers (SEQ ID NO: 19 and SEQ ID NO: 21), 200 µM of dNTPs and 5 µl of 10 x Pfu Buffer were added to make the volume of the solution 50 µl. PCR was carried out by reacting at 95°C for 1 minute and then repeating 25 times the cycle set to include 95°C for 1 minute, 60°C for 1 minute and 72°C for 4 minutes. Next, the PCR product was purified with PCR Purification Kit (QIAGEN) followed by treatment with restriction enzymes XbaI and EcoRI. The plasmid p3xFLAG-CMV-14 (Sigma) was also treated with restriction enzymes XbaI and EcoRI. After the respective DNA fragments were subjected to ligation using DNA Ligation Kit ver. 2 (Takara Bio), the ligation products were transfected to Escherichia coli TOP10, followed by selection in ampicillin-supplemented LB agar medium. As a result of the sequence analysis of individual clones, the plasmid pCMV-14-SEMA4B bearing the cDNA fragment, which corresponds to the SEMA4B gene (SEQ ID NO: 2), was obtained.

### REFERENCE EXAMPLE 7

### Construction of animal cell expression vector for recombinant full-length tagged protein

Expression vector for animal cells capable of expressing SEMA4B protein tagged with 3xFLAG at the C terminus was constructed. Escherichia coli transformants were selected in accordance with the procedures described in REFERENCE EXAMPLE 6, except that the primer pair used for amplification of the SEMA4B gene by PCR was changed to another primer pair (SEQ ID NO: 19 and SEQ ID NO: 20). As a result, the plasmid pCMV-14-SEMA4B-3xFLAG containing a cDNA fragment encoding 3xFLAG tagged-protein at the C terminus of SEMA4B protein (SEQ ID NO: 1) was obtained.

### REFERENCE EXAMPLE 8

### Preparation and purification of peptide antibodies

Based on the amino acid sequences of SEMA4B protein (SEQ ID NO: 1), SEMA4B-M1 protein (SEQ ID NO: 4), SEMA4B-M2 protein (SEQ ID NO: 7) and SEMA4B-M3 protein (SEQ ID NO: 10), the following 4 peptides (peptides 1 to 4) composed of 12 to 15 amino acids were synthesized by the Fmoc solid phase synthesis.

The amino acid sequence of peptide 1 [Asn-Ser-Ala-Arg-Glu-Arg-Lys-Ile-Asn-Ser-Ser-Cys (SEQ ID NO: 22)] is the 402nd-412th amino acid sequence in SEMA4B protein (SEQ ID NO: 1), in which Cys is added to the amino acid sequence at the C terminus.

The amino acid sequence of peptide 2 [Ser-Val-Val-Ser-Pro-Ser-Phe-Val-Pro-Thr-Gly-Glu-Lys-Pro-Cys (SEQ ID NO: 23)] is the 582nd-596th amino acid sequence in SEMA4B protein (SEQ ID NO: 1).

The amino acid sequence of peptide 3 [Pro-Leu-Asp-His-Arg-Gly-Tyr-Gln-Ser-Leu-Ser-Asp-Ser-Pro-Cys (SEQ ID NO: 24)] is the 781st-794th amino acid sequence in SEMA4B protein (SEQ ID NO: 1), in which Cys is added to the amino acid sequence at the C terminus.

The amino acid sequence of peptide 4 [Ser-Arg-Val-Phe-Thr-Glu-Ser-Glu-Lys-Arg-Pro-Leu-Ser-Cys (SEQ ID NO: 25)] is the 797th-809th amino acid sequence in SEMA4B protein (SEQ ID NO: 1), in which Cys is added to the amino acid sequence at the C terminus.

Keyhole limpet hemocyanin (KLH) as a carrier protein was coupled to each of peptides 1, 2, 3 and 4, which were used as antigens to produce rabbit polyclonal antibodies, as described below.

One male rabbit KBL:JW (11 weeks old, Oriental Yeast) was used as an immune animal. Complete Freund's adjuvant (Difco Laboratories) suspension was used for primary sensitization and incomplete adjuvant (Difco Laboratories) suspension for the second sensitization and thereafter. The sensitization was performed by subcutaneous injection at the back and 0.5 mg each of the respective antigens was used for each sensitization. After the primary sensitization, it was repeated 3 times every 14 days. On day 52 after the primary sensitization, blood was collected through the carotid artery under anesthesia to give about 50 ml of serum. The serum thus obtained was concentrated by means of ammonium sulfate salting out. The total amount of the crude IgG fractions obtained were purified on protein A-affinity column (Amersham-Bioscience) to give about 103 mg, about 76 mg, about 112 mg and about 122 mg of the purified IgGs from peptides 1, 2, 3 and 4, respectively. In addition, the IgG fraction bound to the column where each immunogenic peptide was immobilized was acquired. For immobilization, the C-terminal Cys of each peptide was utilized and the peptide was coupled to Sepharose column (Amersham-Bioscience) using borate buffer. For elution from the column, 8M urea/phosphate buffered saline (PBS) was used. The eluate was dialyzed to PBS to remove urea, which was followed by ultraconcentration and sterilization by filtering. Thus, affinity-purified antibodies AS-2531, AS-2532, AS-2591 and AS-2592 to peptides 1, 2, 3 and 4, were acquired in about 15 mg, about 126 mg, about 17 mg and about 35 mg, respectively.

### REFERENCE EXAMPLE 9

### Western blotting using rabbit peptide antibodies

SEMA4B protein (SEQ ID NO: 1) was detected using the purified peptide antibodies prepared in REFERENCE EXAMPLE 8. Human non-small-cell lung cancer cell line NCI-H358 cells were suspended in 10 ml ofRPMI-1640 medium (Invitrogen) supplemented with 10% fetal bovine serum (JRH) at a concentration of 1.5 x 10⁶ and plated on a Petri dish of 10 cm in diameter. After incubation at 37°C overnight in a 5% carbon dioxide flow, 6 µg of the plasmid pCMV-14- SEMA4B prepared in REFERENCE EXAMPLE 6 was mixed with Plus reagent (Invitrogen) and OPTI-MEM I (Invitrogen). After the mixture was allowed to stand at room temperature for 15 minutes, LipofectAMINE transfection reagent (Invitrogen) and OPTI-MEM I were added to the mixture. The mixture was allowed to stand at room temperature for further 15 minutes. The liquid mixture was dropwise added to the culture medium and incubation was continued. Two days after transfection of the expression plasmid, the cells were washed with ice-chilled PBS and 1 ml of ice-chilled RIPA buffer [50 mM Tris-hydrochloride buffer, pH 7.5, 150 mM sodium chloride, 1% Triton X-100, 0.1% SDS, 1% deoxycholic acid, Complete™ Tablet (Roche Diagnostics) and Phosphatase Inhibitor Cocktail-2 (Sigma)] was added to the cells. The mixture was allowed to stand at 4°C for 30 minutes. This RIPA buffer was recovered and centrifuged at 15,000 rpm for 20 minutes. The supernatant separated was used as the cell-free extract. This cell-free extract and 2-fold concentrated sample buffer [125 mM Tris-hydrochloride buffer, pH 6.8, 40% glycerol, 4% SDS, 0.04% bromophenol blue and 5% 2-mercaptoethanol] for SDS-PAGE were mixed in an equal volume. After heating at 95°C for 5 minutes, 10 µl of the mixture was provided for SDS-PAGE on 10% acrylamide gel. The protein electrophoresed and then isolated was transferred onto Clear Blotting P Membrane (ATTO) in a conventional manner, which was then allowed to stand in a blocking buffer (50 mM Tris-hydrochloride buffer, pH 7.5, 500 mM sodium chloride, 0.1% Tween 20, 5% skimmed milk) at room temperature for an hour. Next, the peptide antibody AS-2531, AS-2532, AS-2591 or AS-2592 prepared in REFERENCE EXAMPLE 8 were diluted in the blocking buffer to a concentration of 3 µg/ml, followed by reacting at 4°C overnight. Subsequently, the mixture was allowed to stand at room temperature for an hour in HRP-labeled anti-rabbit IgG antibody (Amersham-Bioscience) diluted in the blocking buffer to 50,000-fold or 100,000-fold. Detection was performed using ECL plus (Amersham-Bioscience) according to the protocol attached.

In all of AS-2532, AS-2591 and AS-2592 except AS-2531, a specific band attributed to the SEMA4B protein was noted at the position near 100 kD molecular weight.

### REFERENCE EXAMPLE 10

### Immunoprecipitation using rabbit peptide antibodies

Using the purified peptide antibodies prepared in REFERENCE EXAMPLE 8, immunoprecipitation was performed on the SEMA4B protein under non-denaturing conditions.

Using the plasmid pCMV-14-SEMA4B-3xFLAG obtained in REFERENCE EXAMPLE 7, the cell-free extract was prepared by the procedures similar to REFERENCE EXAMPLE 9. Protein G-Sepharose 4FF (Amersham-Bioscience) was suspended in an equal volume of RIPA buffer and 400 µl of the cell-free extract was added to 50 µl of the resulting suspension. Further 5 µg of any one of the peptide antibodies AS-2531, AS-2532, AS-2591 and AS-2592 described in REFERENCE EXAMPLE 8 was added thereto. The mixture prepared was agitated at 4°C overnight. After the Protein G-Sepharose 4FF co-precipitated fraction was washed with RIPA buffer, the fraction was suspended in 50 µl of SDS-PAGE sample buffer [62.5 mM Tris-hydrochloride buffer, pH 6.8, 20% glycerol, 2% SDS, 0.02% bromophenol blue and 2.5% 2-mercaptoethanol] (Bio-Rad Laboratories). The mixture was heated at 95°C for 5 minutes and then, 5 µl or 10 µl of the mixture was provided for SDS-PAGE on 10% acrylamide gel. Detection was performed by the same procedures as in REFERENCE EXAMPLE 9, except that mouse anti- FLAG M2 antibody (Sigma) diluted with the blocking buffer to 0.2 µg/ml or 0.1 µg/ml was used a primary antibody, and HRP-labeled anti-mouse IgG antibody (Amersham-Bioscience) diluted with the blocking buffer to 25,000-fold or 50,000-fold was used as a secondary antibody.

Even when immunoprecipitation was performed using any of the three rabbit sera containing the peptide antibodies AS-2531, AS-2532, AS-2591 and AS-2592, respectively, a specific band attributed to the SEMA4B protein was noted at the position near 100 kD molecular weight.

The foregoing results reveal that the peptide antibodies AS-2531, AS-2532, AS-2591 and AS-2592 bind to the non-denaturing SEMA4B protein.

### [REFERENCE EXAMPLE 11]

### Study of expression of SEMA4B protein in cancer cell lines

Lung cancer cell lines NCI-H2228, NCI-H1651, NCI-H358, NCI-H23 and NCI-H1703; ovarian cancer cell lines SKOV-3 and TOV-21G; prostate cancer cell line DU145; and pancreas cancer cell line PANC-1 were plated, respectively, on two Petri dishes of 10 cm each in diameter. In the respective cells, one Petri dishful of the cells were dispersed in trypsin-EDTA (Invitrogen) and the cell count was measured. Based on the cell count measured, ice-chilled RIPA buffer (described in REFERENCE EXAMPLE 9) was added to the remaining one Petri dish in 1 ml/5 x 10⁶ cells, which was allowed to stand at for 4°C 30 minutes. This RIPA buffer was recovered and centrifuged at 15,000 rpm for 20 minutes. The supernatant was used as a cell-free extract. On the other hand, a resin in which the peptide antibody AS-2531 described in REFERENCE EXAMPLE 8 was crosslinked to Protein G-Sepharose 4FF (Amersham-Bioscience) was prepared following the protocol attached to Size™ X Protein G Immunoprecipitation Kit (Pierce). The resin was suspended in an equal volume of the RIPA buffer. To 30 µl of this suspension, 400 µl of the cell-free extract was added and the mixture was agitated at 4°C overnight. After the Protein G-Sepharose 4FF co-precipitated fraction was washed with RIPA buffer, the fraction was suspended in 30 µl of SDS-PAGE sample buffer described in REFERENCE EXAMPLE 10. The mixture was heated at 95°C for 5 minutes and then, 20 µl of the mixture was provided for SDS-PAGE on 10% acrylamide gel. Detection was performed by the same procedures as in REFERENCE EXAMPLE 9, using the peptide antibody AS-2532.

In each cell line of NCI-H2228, NCI-H358, NCI-H23, SKOV-3, DU145 and PANC-1 among the 9 cell lines described above, a specific band attributed to the SEMA4B protein was noted at the position near 100 kD molecular weight. These results reveal that the SEMA4B protein was highly expressed in the 6 cancer cell lines described above.

### REFERENCE EXAMPLE 12

### Establishment of the cell line stably expressing the recombinant full-length protein

Human non-small lung cancer-derived NCI-H358 cells, 2.0 x 10⁵, were suspended in 2 ml of RPMI-1640 medium (Invitrogen) supplemented with 10% fetal bovine serum (JRH), 1 mM sodium pyruvate and 25 mM HEPES. After plating on a 6-well plate, the cells were incubated at 37°C overnight in a 5% carbon dioxide gas flow. On the other hand, 1 µg of the plasmid pCMV-14-SEMA4B described in REFERENCE EXAMPLE 6, which had been diluted in OPTI-MEM I (Invitrogen), was mixed with 6 µl of Plus Reagent (Invitrogen). After the mixture was allowed to stand at room temperature for 15 minutes, 4 µl of LipofectAMINE transfection reagent (Invitrogen), which had been diluted in OPTI-MEM I, was added to the mixture, which was then allowed to stand at room temperature for further 15 minutes. This mixture was dropwise added to the medium. After incubation was further continued for a day, the cells were dispersed in trypsin-EDTA, diluted to 10-fold in the medium described above containing G418 (Promega) in 400 µg/ml, and plated on a 24-well plate. While exchanging the medium described above containing G418 (G418 selection medium) every 3 or 4 days, incubation was continued at 37°C in a carbon dioxide gas flow. The cells were recovered from the wells where 1 to 3 cells grew to form colonies and equally plated on 2 wells of a 48-well plate. After incubation was continued to reach 50% of the cell density or more, 50 µl of SDS-PAGE sample buffer described in REFERENCE EXAMPLE 10 was added to the cells corresponding to one well to prepare the cell lysate. After heat treatment at 95°C for 5 minutes, 5 µl of the suspension was provided for SDS-PAGE on 10% acrylamide gel. Using the peptide antibody AS-2532, western blotting was performed by a modification of the procedures described in REFERENCE EXAMPLE 9 to search stable cells capable of constitutively expressing the SEMA4B protein (SEQ ID NO: 1). The cells recovered from the other well were diluted to become 0.7 cell/well, and then plated on a 96-well plate. While exchanging the G418 selection medium every 3 or 4 days, incubation was continued at 37°C in a carbon dioxide gas flow to reach about 50% of the cell density. Again, the cells were equally plated on 2 wells of a 48-well plate and incubation was continued to reach 50% of the cell density or more. Using the cell lysate prepared from the cells corresponding to one well, western blotting was carried out as described above. A clone which most highly expressed the SEMA4B protein (SEQ ID NO: 1) was selected to obtain SEMA4B/H358 as the cell line stably expressing SEMA4B.

### REFERENCE EXAMPLE 13

### Study of localization of the SEMA4B protein (biotin labeling)

Using human non-small lung cancer cell lines NCI-H2228 and NCI-H358 and the cell line stably expressing the recombinant full-length protein (SEMA4B/H358) prepared in REFERENCE EXAMPLE 12, the proteins exposed on the cell surfaces were biotin-labeled with Cellular Labeling and Immunoprecipitation Kit (Roche Diagnostics). Using 1 ml of the cell-free extract prepared by the procedures of REFERENCE EXAMPLE 9 and 5 µg of the peptide antibody AS-2591 prepared in REFERENCE EXAMPLE 8, immunoprecipitation was performed in accordance with the process of REFERENCE EXAMPLE 10, followed by SDS-PAGE. By detection with HRP-labeled streptoavidin (Amersham-Bioscience), bands attributed to the SEMA4B protein were observed near 100 kD molecular weight, indicating that the SEMA4B protein, SEMA4B-M1 protein, SEMA4B-M2 protein and SEMA4B-M3 protein were localized on the cell surfaces.

### REFERENCE EXAMPLE 14

### Study of localization of SEMA4B protein (FACS analysis)

Human non-small cell lung cancer cell lines NCI-H2228 and NCI-H358 and SEMA4B/H358 described in REFERENCE EXAMPLE 12 were plated on a Petri dish of 10 cm in diameter and cultured to become subconfluent. After the respective cells were washed with PBS, PBS supplemented with 0.5% BSA and 5 mM EDTA was added thereto. The mixture was allowed to stand at room temperature for 15 minutes to disperse the cells. Next, the cells were suspended in Buffer A [HBSS (Hanks' Balanced Salt Solutions, Invitrogen) supplemented with 2% fetal bovine serum (JRH) and 0.1% sodium azide] in a concentration of 4 x 10⁶/ml, and AS-2532 or non-immunized rabbit IgG (Jackson) was added to the suspension in a final concentration of 10 µg/ml. The mixture was allowed to stand on ice for 3 hours. After the cells were washed with Buffer A and suspended in Buffer A containing 10 µg/ml ofAlexa488-labeled anti-rabbit IgG antibody (Molecular Probes), the mixture was allowed to stand on ice for 2 hours. After washing again with Buffer A, the cells were analyzed by FACScan (BD Biosciences). As a result, the cells were all stained specifically to rabbit peptide antibody AS-2532, which revealed that the SEMA4B protein, SEMA4B-M1 protein, SEMA4B-M2 protein and SEMA4B-M3 protein were localized on the cell surfaces.

### REFERENCE EXAMPLE 15

### Induction of apoptosis in human non-small cell lung cancer cell line NCI-H358 by transfection of the antisense oligonucleotide

It was examined whether or not apoptosis could be induced also in human non-small cell lung cancer cell line other than NCI-H1703 described in REFERENCE EXAMPLE 2, by transfection of the antisense oligonucleotide.

NCI-H358 was suspended in RPMI-1640 medium (Invitrogen) supplemented with 10% fetal bovine serum (JRH), 1 mM sodium pyruvate and 25 mM HEPES. NCI-H358 was plated on a 96-well flat bottomed tissue culture plate (BD Falcon) at a cell density of 8 x 10³ cells/well (80 µl of a culture medium volume) and then incubated overnight at 37°C in a 5% carbon dioxide gas flow. On the other hand, 0.06 µg each of the antisense oligonucleotide (SEQ ID NO: 13) and control oligonucleotide (SEQ ID NO: 14) described in REFERENCE EXAMPLE 2 were diluted in OPTI-MEM I (Invitrogen) and each dilution was mixed with 0.5 µl of Plus Reagent (Invitrogen). The mixture was then allowed to stand at room temperature for 15 minutes. Then, 0.4 µl of LipofectAMINE transfection reagent (Invitrogen) diluted with OPTI-MEM I was added to the mixture, which was then allowed to stand at room temperature for further 15 minutes. The whole volume of this liquid mixture was added to the culture solution of NCI-H358. After incubation was continued for 3 days, the apoptosis induction activity of the oligonucleotide described above was assayed according to the protocol attached to Cell Death Detection ELISA^{PLUS} (Roche Diagnostics) and Caspase-Glo 3/7 assay (Promega).

As a result, in NCI-H358, the antisense oligonucleotide showed the apoptosis induction activity higher by 1.42 times and 1.77 times than the control oligonucleotide used as a negative control both by Cell Death Detection ELISA^{PLUS} and by Caspase-Glo 3/7 assay, indicating that there was a statistically significant difference (P≦0.01) (TABLES 5 and 6).

**[TABLE 5]**

| | Apoptosis Induction Activity (A₄₀₅-A₄₉₂) | |
|---|---|---|
| | Mean Value | Standard Deviation |
| Blank | 0.217 | 0.007 |
| Control oligonucleotide (SEQ ID NO: 14) | 0.330 | 0.041 |
| Antisense oligonucleotide (SEQ ID NO: 13) | 0.467 | 0.029 |

**[TABLE 6]**

| | Apoptosis Induction Activity (CPS) | |
|---|---|---|
| | Mean Value | Standard Deviation |
| Blank | 7625 | 235 |
| Control oligonucleotide (SEQ ID NO: 14) | 8727 | 188 |
| Antisense oligonucleotide (SEQ ID NO: 13) | 15452 | 570 |

### REFERENCE EXAMPLE 16

### Induction of apoptosis in human non-small cell lung cancer cell lines NCI-H2228, NCI-H1651 and NCI-H23 by transfection of the antisense oligonucleotide

It was examined whether or not apoptosis could be induced also in human non-small cell lung cancer cell lines other than NCI-H1703 (REFERENCE EXAMPLE 2) and NCI-H358 (REFERENCE EXAMPLE 15), by transfection of the antisense oligonucleotide.

For NCI-H2228, RPMI-1640 medium (Invitrogen) supplemented with 10% fetal bovine serum (JRH), 1 mM sodium pyruvate and 25 mM HEPES was used. For NCI-H1651, ACL-4 medium (ATCC) supplemented with 10% fetal bovine serum (JRH) and 25 mM HEPES was used. For NCI-H23, RPMI-1640 medium (Invitrogen) supplemented with 10% fetal bovine serum (JRH) and 25 mM HEPES was used. The respective cells were suspended in the respective media and plated on a 96-well flat bottomed tissue culture plate (BD Falcon) at cell densities (125 µl of a culture medium volume) of 7.5 x 10³ (NCI-H2228), 7.5 x 10³ (NCI-H1651) and 5 x 10³ (NCI-H23)/well, respectively. Incubation was performed overnight at 37°C in a 5% carbon dioxide gas flow. On the other hand, 0.135 µg each of the antisense oligonucleotide (SEQ ID NO: 13) and control oligonucleotide (SEQ ID NO: 14) described in REFERENCE EXAMPLE 2 were diluted in OPTI-MEM I (Invitrogen). After mixing with 0.75 µl of Plus Reagent (Invitrogen), the mixture was allowed to stand at room temperature for 15 minutes. Then, 0.4 µl of LipofectAMINE transfection reagent (Invitrogen) diluted in OPTI-MEM I was added to the mixture, which was allowed to stand at room temperature for further 15 minutes. The whole volume of this liquid mixture was added to the culture solution of each cell. After incubation was continued for 3 days, the apoptosis induction activity of the oligonucleotide described above was assayed according to the protocol attached to Cell Death Detection ELISA^{PLUS} (Roche Diagnostics).

As a result, the antisense oligonucleotide showed the apoptosis induction activity in all cell lines higher by 1.58 times (NCI-H2228), 1.21 times (NCI-H1651) and 1.25 times (NCI-H23) than the control oligonucleotide used as a negative control, and p-values were found to be P≦0.05 (NCI-H2228), P≦0.05 (NCI-H1651) and P ≦0.01 (NCI-H23), indicating that there was a statistically significant difference (TABLES 7, 8 and 9).

**[TABLE 7]**

| | Apoptosis Induction Activity (A₄₀₅-A₄₉₂) | |
|---|---|---|
| | Mean Value | Standard Deviation |
| Blank | 0.312 | 0.009 |
| Control oligonucleotide (SEQ ID NO: 14) | 0.526 | 0.043 |
| Antisense oligonucleotide (SEQ ID NO: 13) | 0.829 | 0.123 |

**[TABLE 8]**

| | Apoptosis Induction Activity (A₄₀₅-A₄₉₂) | |
|---|---|---|
| | Mean Value | Standard Deviation |
| Blank | 0.523 | 0.091 |
| Control oligonucleotide (SEQ ID NO: 14) | 1.152 | 0.101 |
| Antisense oligonucleotide (SEQ ID NO: 13) | 1.390 | 0.104 |

**[TABLE 9]**

| | Apoptosis Induction Activity (A₄₀₅-A₄₉₂) | |
|---|---|---|
| | Mean Value | Standard Deviation |
| Blank | 0.678 | 0.028 |
| Control oligonucleotide (SEQ ID NO: 14) | 1.081 | 0.050 |
| Antisense oligonucleotide (SEQ ID NO: 13) | 1.351 | 0.058 |

### REFERENCE EXAMPLE 17

### Induction of apoptosis using rabbit peptide antibodies

Human non-small lung cancer cell line NCI-H2228 was treated with rabbit peptide antibodies AS-2531 and AS-2532 produced in REFERENCE EXAMPLE 8 to determine the apoptosis induction activities of these rabbit peptide antibodies.

NCI-H2228 was suspended in RPMI-1640 medium (Invitrogen) supplemented with 10% fetal bovine serum (JRH), 1 mM sodium pyruvate and 25 mM HEPES and plated on a 96-well flat bottomed tissue culture plate (BD Falcon) coated with type I collagen at a cell density of 4 x 10³ cells/well, followed by incubation overnight at 37°C in a 5% carbon dioxide gas flow. Rabbit peptide antibodies AS-2531 and AS-2532 obtained in REFERENCE EXAMPLE 8 and non-immunized rabbit IgG (Jackson) were diluted in PBS, and each antibody was added to the culture solution in a final concentration of 15 µg/ml, 45 µg/ml and 150 µg/ml, respectively. After incubation was continued for further 5 days, the apoptosis induction activities of the rabbit peptide antibodies described above were determined according to the protocol attached to Cell Death Detection ELISA^{PLUS} (Roche Diagnostics).

As a result, the peptide antibodies showed the apoptosis induction activities higher by 1.26 times and 1.31 times than the non-immunized rabbit IgG of the same concentration in the presence of 45 µg/ml and 15 µg/ml of AS-2531 (P≦0.05 and P ≦0.01). In the presence of 150 µg/ml of AS-2532, the peptide antibodies showed the apoptosis induction activities higher by 1.27 times than the non-immunized rabbit IgG of the same concentration (P≦0.01).

As demonstrated above, it became clear that SEMA4B protein, SEMA4B-M1 protein, SEMA4B-M2 protein and SEMA4B-M3 protein play an important role in maintaining the survival of human lung cancer cells.

### EXAMPLE 1

### Binding of Plexin B 1 to SEMA4B

### (1) cDNA cloning of the extracellular domain of Plexin B 1

Plexin B 1 cDNA was acquired by PCR using human kidney-derived cDNA (MTC Panel, BD CLONTECH) as a template. The composition of the reaction solution used in the reaction was as follows: 1 µl of the cDNA above was used as a template, and 1.25 U of Pfu Turbo DNA Polymerase (STRATAGENE), 2 µM each of two primers (SEQ ID NO: 27 and SEQ ID NO: 28), 200 µM of dNTPs and 10 µl of 2x GC Buffer I (Takara Bio) were added thereto. The volume of the reaction solution was adjusted to 20 µl. PCR was carried out by heating at 95°C for 1 minute, followed by 40 cycles of 95°C for 15 seconds, 60°C for 30 seconds and 72°C for 6 minutes and 30 seconds and an additional reaction at 72°C for 7 minutes. PCR using another primer pair (SEQ ID NO: 29 and SEQ ID NO: 30) was also carried out in parallel. The respective PCR products were separated on 0.8% agarose gel added with 1.6 µg/ml of crystal violet according to the protocol of TOPO XL PCR Cloning Kit (Invitrogen) and purified using MinElute Gel Extraction Kit (QIAGEN). The purified product was subcloned to plasmid vector pCR-XL-TOPO (Invitrogen), transfected to Escherichia coli TOP10 (Invitrogen) and then selected in kanamycin-supplemented LB agar medium. Sequencing of individual clones gave a plasmid bearing the cDNA fragment corresponding to the -95th to the 3016th (pCR-XL-PLXNB 1-NT) and a plasmid bearing the cDNA fragment corresponding to the 1628th to the 5840th (pCR-XL-PLXNB 1-CT), in the full length cDNA for Plexin B1, respectively.

First, pCR-XL-PLXNB1-NT was simultaneously digested with two restriction enzymes (EcoRI and SnaBI) to give a DNA fragment of about 2 kbp (DNA Fragment-1). Subsequently, pCR-XL-PLXNB1-CT was simultaneously digested with three restriction enzymes (SnaBI, DraI and XbaI) to give a DNA fragment of about 4.1 kbp (DNA Fragment-2). Finally, pcDNA3.1 (+) plasmid (Invitrogen) was simultaneously digested with two restriction enzymes (EcoRI and XbaI) to give a DNA fragment of about 5.4 kbp (DNA Fragment-3). After DNA Fragment-1, DNA Fragment-2 and DNA Fragment-3 were separately purified on MinElute Gel Extraction Kit, the DNA fragments were mixed in a ratio of 2:2:1 by volume to make the whole volume 5 µl. Furthermore, 5 µl of Solution I of DNA Ligation Kit ver. 2 (Takara Bio) was added to the mixture, which was reacted at 16°C for 30 minutes. Then, 2 µl was withdrawn and Escherichia coli TOP10 was transformed. Sequencing of individual clones gave a plasmid bearing the cDNA fragment corresponding to the -95th to the 5840th (pcDNA3.1(+)-PLXNB1-NT2) in the full length cDNA for Plexin B 1.

Next, in order to acquire a cDNA fragment encoding the extracellular domain only, PCR was carried out using the aforesaid plasmid (pcDNA3.1(+)-pLXNB1-NT2) as a template. The composition of the reaction solution in the reaction was as follows: 10 ng of the plasmid described above, 1.25 U of Pfu Turbo DNA Polymerase, 1 µM each of two primers (SEQ ID NO: 31 and SEQ ID NO: 32), 200 µM of dNTPs and 10 µl of 2x GC Buffer I were added to make the volume of the reaction solution 20 µl. PCR was carried out by heating at 95°C for 1 minute, followed by 30 cycles of 95°C for 30 seconds, 65°C for 30 seconds and 72°C for 5 minutes, and an additional reaction at 72°C for 7 minutes. Following the protocol of TOPO XL PCR Cloning Kit, the PCR product was separated on 0.8% agarose gel added with 1.6 µg/ml of crystal violet and the DNA of 4 - 5 kbp was purified using MinElute Gel Extraction Kit. The purified product was subcloned to plasmid vector pCR-XL-TOPO, transfected to Escherichia coli TOP10 and then selected in kanamycin-supplemented LB agar medium. Sequencing of individual clones gave plasmid pCR-XL-PLXNB1-ECD bearing the DNA fragment corresponding to the -44th to the 4443rd with recognition sequence of restriction enzyme BamHI at the both ends in the full length cDNA for Plexin B1.

### (2) Construction of protein-expressing vector in the extracellular domain of Plexin B1

In order to destroy the BamHI recognition sequence present in the Plexin B 1 sequence of the plasmid (pCR-XL-PLXNB1-ECD) obtained in (1) above, the 3483rd T located in the full length Plexin B 1 cDNA was changed to C in accordance with the protocol of QuikChange Site-Directed Mutagenesis Kit (STRATAGENE), and plasmid pCR-XL-PLXNB1-ECD-M1 wherein silent mutation was introduced was prepared.

Meanwhile, in order to express the extracellular domain of Plexin B1 as a fused protein added with the constant (Fc) domain of human immunoglobulins at the C terminus, human Fc domain was amplified by PCR. The composition of the reaction solution was as follows: 1 µl of human spleen-derived cDNA (MTC Panel, BD CLONTECH), 1.25 U of Pfu Turbo DNA Polymerase, 0.5 µM each of two primers (SEQ ID NO: 33 and SEQ ID NO: 34), 200 µM of dNTPs and 10 µl of 2x GC Buffer I were added to make the volume of the reaction solution 20 µl. PCR was carried out by heating at 95°C for 1 minute, followed by 30 cycles of 95°C for 30 seconds, 65°C for 30 seconds and 72°C for 1 minute, and an additional reaction at 72°C for 7 minutes. The PCR product was purified using MinElute PCR Purification Kit (QIAGEN) and subcloned to plasmid vector pCR4-TOPO (Invitrogen). The transfected Escherichia coli TOP10 was selected in kanamycin-supplemented LB agar medium. Sequencing of individual clones gave plasmid pCR4-TOPO-Fc containing cDNA fragment encoding human Fc domain.

First, pCR4-TOPO-Fc was simultaneously digested with two restriction enzymes (BamHI and HindIII) to give a DNA fragment of about 0.7 kbp (DNA Fragment-4). Next, the aforesaid pCR-XL-PLXNB1-ECD-M1 was digested with BamHI to give a DNA fragment of about 4.5 kbp (DNA Fragment-5). Finally, pcDNA3.1 (-) plasmid (Invitrogen) was simultaneously digested with two restriction enzymes (BamHI and HindIII) to give a DNA fragment of about 5.4 kbp (DNA Fragment-6). After DNA Fragment-4, DNA Fragment-5 and DNA Fragment-6 were separately purified using MinElute Gel Extraction Kit, the DNA fragments were mixed in a volume ratio of 2:2:1 to make the volume 5 µl. Furthermore, 5 µl of Solution I of DNA Ligation Kit ver. 2 was added to the mixture, which was reacted at 16°C overnight. Then, 2 µl was withdrawn and Escherichia coli TOP 10 was transfected. Sequencing of individual clones selected from ampicillin-supplemented LB agar medium gave plasmid pcDNA3.1(-)-PLXNB1-ECD/Fc expressing a Plexin B1 chimeric protein connecting the Fc domain of human at the C terminus.

### (3) Preparation of recombinant Plexin B1/Fc fused protein

Human embryonic kidney-derived HEK293 (1.5 x 10⁶) described in REFERENCE EXAMPLE 5 was suspended in 10 ml of DMEM medium (Invitrogen) supplemented with 10% fetal bovine serum (JRH) and plated on a Petri dish of 10 cm in diameter (BD Falcon) coated with type I collagen, followed by incubation overnight at 37°C in a 5% carbon dioxide gas flow. The plasmid pcDNA3.1(-)-PLXNB1-ECD/Fc, 6 µg, obtained in EXAMPLE 1-(2) was diluted in OPTI-MEM I (Invitrogen) to make the volume 200 µl and 18 µl of FuGENE6 Transfection Reagent (Roche Diagnostics) was added to the dilution, which was then allowed to stand at room temperature for 30 minutes. The mixture was dropwise added to the culture solution and incubation was continued overnight. On the following day, the cells were washed with PBS and the medium was exchanged by 10 ml of OPTI-MEM I (Invitrogen). Incubation was continued for further 2 days. The culture solution was then filtrated through MILLEX-GV (MILLIPORE) and the filtrate was concentrated about 20-fold using a centrifugal ultrafiltration device (MILLIPORE) with a 10,000 molecular weight cut-off membrane. The concentrate was named PLXNB1/Fc. The supernatant obtained when transfected without adding the plasmid pcDNA3.1(-)-PLXNB1-ECD/Fc was also prepared in parallel and named a negative control.

### (4) Experiment of binding Plexin B1 to SEMA4B

Following the procedures described in REFERENCE EXAMPLE 10, the plasmid pCMV-14-SEMA4B-3xFLAG (described in REFERENCE EXAMPLE 7) was transfected to human non-small cell lung cancer cell line NCI-H358 to prepare the cell-free extract. A mixture obtained by adding 250 µl of the cell-free extract described above to 200 µl of PLXNB1/Fc prepared in EXAMPLE 1-(3) or 200 µl of the negative control was prepared and 50 µl of the Protein G-Sepharose 4FF (Amersham-Bioscience) suspension described in REFERENCE EXAMPLE 10 was added to the mixture, followed by stirring at 4°C overnight. After the Protein G-Sepharose 4FF co-precipitated fraction was washed with RIPA buffer (described in REFERENCE EXAMPLE 9), the fraction was suspended in 50 µl of SDS-PAGE sample buffer (described in REFERENCE EXAMPLE 9). The mixture was heated at 95°C for 5 minutes and then 20 µl of the mixture was provided for SDS-PAGE on 7.5% acrylamide gel. A dilution obtained by diluting the AS-2591 antibody (described in REFERENCE EXAMPLE 8) in the blocking buffer described in REFERENCE EXAMPLE 9 in 3 µg/ml, or a dilution obtained by diluting mouse anti-FLAG M2 antibody (Sigma) in the blocking buffer described above in 0.2 µg/ml was used as a primary antibody. Subsequently, a dilution obtained by diluting HRP-labeled anti-rabbit IgG antibody (Amersham- Bioscience) in the blocking buffer described above to 100,000-fold, or a dilution obtained by diluting HRP-labeled anti-mouse IgG antibody (Amersham- Bioscience) in the blocking buffer described above to 50,000-fold was used as a secondary antibody. Following the protocol attached to ECL plus (Amersham-Bioscience), SEMA4B protein was detected. As a result, the SEMA4B protein could be detected only in the case where PLXNB1/Fc was used.

The foregoing reveals that the SEMA4B protein binds to Plexin B 1 protein.

### EXAMPLE 2

### Binding of Plexin B1 to SEMA4B (FACS analysis)

### (1) Establishment of the cell line stably expressing the recombinant Plexin B1/Fc-fused protein

Human embryonic kidney-derived HEK293 (2 x 10⁶) described in REFERENCE EXAMPLE 5 was suspended in 10 ml of DMEM medium (Invitrogen) supplemented with 10% fetal bovine serum (JRH) and plated on a Petri dish (Iwaki Glass) of 10 cm in diameter coated with type I collagen, followed by incubation overnight at 37°C in a 5% carbon dioxide gas flow. The plasmid pcDNA3.1(-)-PLXNB1-ECD/Fc, 6 µg, obtained in EXAMPLE 1-(2) was diluted in OPTI-MEM I (Invitrogen) to make the volume 1200 µl and 18 µl of FuGENE6 Transfection Reagent (Roche Diagnostics) was added to the dilution, which was then allowed to stand at room temperature for 30 minutes. The mixture was dropwise added to the culture solution and incubation was further continued for a day. The cells were then dispersed in trypsin-EDTA (Invitrogen), diluted to 10-fold in the above culture medium supplemented with 750 µg/ml of G418 (Promega) (G418 selection medium) and plated on a Petri dish of 10 cm in diameter coated with type I collagen. Every 3 or 4 days the cells were dispersed in trypsin-EDTA and diluted to 10-fold, followed by incubation in the G418 selection medium. Two weeks after the incubation started on the G418 selection medium, the cells grown were collected and named the HEK293 cell line stably expressing the Plexin Bl/Fc-fused protein (PLXNB1-Fc/HEK293).

### (2) Analysis of Plexin B 1/SEMA4B binding activity

PLXNB1-Fc/HEK293 obtained in EXAMPLE 2-(1) was suspended in 10 ml of DMEM medium (Invitrogen) supplemented with 10% fetal bovine serum (JRH) and plated on a Petri dish (Iwaki Glass) of 10 cm in diameter coated with type I collagen, followed by incubation overnight at 37°C in a 5% carbon dioxide gas flow. On the following day, the cells were washed with PBS and the medium was exchanged by 10 ml of M199 medium (Invitrogen) containing non-essential amino acids (Invitrogen) and ITS-X Supplement (Invitrogen). Incubation was continued for further 2 days. The culture solution was then filtrated through a filter (BD Falcon) having a pore size of 0.45 µm and the filtrate was concentrated about 10-fold using a ultrafiltration device (MILLIPORE) with a 100,000 molecular weight cut-off. The concentrate was named PLXNB1/Fc (lot No. 2) and provided for the following analysis.

Human non-small cell lung cancer cell line NCI-H358 described in REFERENCE EXAMPLE 5 and the cell line SEMA4B/H358 stably expressing SEMA4B obtained in REFERENCE EXAMPLE 12 were suspended in RPMI-1640 medium (Invitrogen) supplemented with 10% fetal bovine serum (JRH), 1 mM sodium pyruvate and 25 mM HEPES. The respective cells were plated on a Petri dish (BD Falcon) of 10 cm in diameter and cultured to become subconfluent. After the respective cells were washed with PBS, PBS supplemented with 0.25% BSA and 2.5 mM EDTA was added thereto. The mixture was allowed to stand at room temperature for 15 minutes to disperse the cells. Next, the cells were suspended in 250 µl of Buffer A [described in REFERENCE EXAMPLE 14 above] prepared to contain 50 µl of PLXNB1/Fc (lot No. 2) above in a concentration of 3 x 10⁶/ml. The mixture was allowed to stand on ice for 3 hours. After the cells were washed with Buffer A and resuspended in Buffer A containing 10 µg/ml of Alexa488-labeled anti-human IgG antibody (Molecular Probes), the mixture was left in ice for an hour. After washing again with Buffer A, the fluorescence intensity of the Alexa488 bound to the cells was measured by FACScan (BD Biosciences).

As a result, the cells were all bound to PLXNB1/Fc (lot No. 2) but the binding amount to PLXNB1/Fc (lot No. 2) was larger in the SEMA4B/H358 cell line, than in the NCI-H358 cell line, indicating good correlation with the expression level of the SEMA4B protein. This reveals that the SEMA4B protein binds specifically to the Plexin B 1 protein.

### EXAMPLE 3

### Binding inhibition activity of rabbit anti-SEMA4B polyclonal antibodies to SEMA4B/Plexin B 1

### (1) Expression and purification of recombinant SEMA4B protein

In the recombinant SEMA4B protein, a vector was prepared to express a FLAG-tagged or 6xHis-tagged protein at the extracellular domain of SEMA4B and its C terminus and the protein expression system was constructed using BAC-TO-BAC Baculovirus Expression System (Invitrogen). That is, in the case of FLAG-tagged protein, there were employed primer (SEQ ID NO: 36) added with EcoR I restriction enzyme site at the N terminus and primer (SEQ ID NO: 37), which was to add FLAG-tag, termination codon and HindIII restriction enzyme site at the C terminus. In the case of 6xHis-tagged protein, there were employed primer (SEQ ID NO: 36) added with EcoR I restriction enzyme site at the N terminus and primer (SEQ ID NO: 38), which was to add 6xHis-tag, termination codon and HindIII restriction enzyme site at the C terminus. Using each primer pair and using as a template pCMV-14-SEMA4B described in REFERENCE EXAMPLE 6, PCR was carried out to acquire cDNA fragment (2151 bp) encoding the SEMA4B extracellular domain. PCR was carried out by repeating 30 cycles set to include 94°C for 30 seconds, 60°C for 30 seconds, 72°C for 3 minutes. The PCR products were separated by agarose gel electrophoresis, recovered using QIAquick Gel Extraction Kit (QIAGEN) and then cloned to pCR2.1-TOPO (Invitrogen) so that pCR2.1/SEMA4B-FLAG and pCR2.1/SEMA4B-His were acquired. After confirmation of the base sequences, pCR2.1/SEMA4B-FLAG and pCR2.1/SEMA4B-His were simultaneously digested with EcoR I (Takara Bio) and Hind III (Takara Bio), respectively, followed by agarose gel electrophoresis. The DNA digestion fragments were then recovered using QIAquick Gel Extraction Kit. pFASTBAC1 (Invitrogen) was similarly treated to recover the DNA digestion fragments, which were subjected to ligation using Ligation High (Toyobo) to prepare the vector pFB/SEMA-FLAG with FLAG-tag at its C terminus or the vector pFB/SEMA-His with 6xHis-tag at its C terminus. Next, recombinant Bacmid DNA was prepared in accordance with the protocol attached to BAC-TO-BAC Baculovirus Expression System to acquire recombinant baculovirus.

The recombinant SEMA4B protein was expressed by infecting 1/100 (v/v) of the recombinant virus with the Sf+ cell line (Nosan Corporation). After infection, the cells were incubated in the serum-free medium Sf-900 II SFM by shake culture at 27°C and 100 rpm for 3 days to recover the supernatant containing the recombinant protein. The protein was separated and purified using ANTI-FLAG M2 Affinity Gel (Sigma) for the culture supernatant containing the FLAG-tagged protein and using Ni-NTA Superflow (QIAGEN) for the culture supernatant containing the 6xHis-tagged protein and further purified on Superdex 200pg (Amersham-Bioscience). Recombinant SEMA4B-FLAG and SEMA4B-His were thus acquired.

### (2) Preparation and purification of rabbit anti-SEMA4B polyclonal antibodies

Rabbit polyclonal antibodies were prepared using as an immunogen the recombinant SEMA4B-FLAG protein prepared in EXAMPLE 3-(1). A PBS solution of the SEMA4B-FLAG protein was mixed with Freund's complete adjuvant in an equal volume and 3 domestic rabbits (Oryctolagus cuniculus, female, 3 kg) were immunized with 0.1 mg of the protein/animal subcutaneously on the back or intracutaneously with the resulting emulsion. For the second and subsequent immunizations, a protein emulsion was prepared in a similar manner except that the adjuvant was replaced by Freund's incomplete adjuvant and repeatedly boostered 7 times every 2 weeks.

Prior to the immunization and one week after the fourth and sixth boosters, blood was taken from the ear vein. An increased serum antibody titer was confirmed by ELISA using an immunoplate coated with the SEMA4B-FLAG protein. One week after the final immunization, blood was taken from the carotid vein of 3 rabbits under anesthesia to collect antisera in 65.1 ml from No. 1 rabbit, 79.5 ml from No. 2 rabbit and 68.0 ml from No. 4 rabbit. These antisera were diluted in PBS to 2-fold and centrifuged to obtain the supernatant. Subsequently, the recombinant SEMA4B-His protein described in EXAMPLE 3-(1) was adsorbed onto an antigen column immobilized to HiTrap NHS-Activated HP (Amersham-Bioscience). The column was washed with PBS and eluted with 0.1 M Glycine-HCl /0.15 M NaCl (pH 3). The eluate was neutralized with 1 M Tris-HCl (pH 8) and then dialyzed against PBS at 4°C overnight to purify and acquire rabbit anti-SEMA4B polyclonal antibodies.

### (3) Binding inhibition activity of rabbit anti-SEMA4B polyclonal antibodies to SEMA4B/Plexin B1

Following the method described in EXAMPLE 2-(2), the binding activity of Plexin B 1 to SEMA4B protein was analyzed by flow cytometry using the SEMA4B/H358 cell line described in REFERENCE EXAMPLE 12. The binding inhibition activity of rabbit IgG was determined by adding the rabbit anti-SEMA4B polyclonal antibody No. 1, No. 2 or No.4 acquired in EXAMPLE 3-(2) or non-immunized rabbit IgG (Jackson). The Plexin B 1 binding activity in the presence of non-immunized rabbit IgG was defined as 100%, and the binding inhibition activity of the rabbit anti-SEMA4B polyclonal antibody described above was calculated. To calculate the Plexin B 1 binding activity, a median value of the fluorescence intensity observed was used.

As a result, the rabbit polyclonal antibodies No. 1, No. 2 and No. 4 showed 63%, 74% and 60% of the binding inhibition activities at the final concentration of 100 µg/ml, respectively. Further study of the binding inhibition activity using the F(ab')₂ fragment of rabbit polyclonal antibody No. 4 showed 73% and 30% of the binding inhibition activities at the final concentrations of 100 µg/ml and 10 µg/ml, respectively.

### EXAMPLE 4

### Growth inhibition activity of rabbit anti-SEMA4B polyclonal antibodies

The NCI-H358 cell line described in REFERENCE EXAMPLE 5 or the SEMA4B/H358 cell line described in REFERENCE EXAMPLE 12 was suspended in RPMI-1640 medium (Invitrogen) supplemented with 1% fetal bovine serum (JRH), 1 mM sodium pyruvate and 25 mM HEPES and plated on a 96-well flat bottomed tissue culture plate (BD Falcon) at a cell density of 5 x 10³/well. In parallel to the plating, rabbit anti-SEMA4B polyclonal antibody No. 1, No. 2 or No. 3 obtained in EXAMPLE 3-(2) or non-immunized IgG (Jackson) was added in a final concentration of 1 µg/ml, 10 µg/ml or 100 µg/ml, followed by incubation at 37°C for 6 days in a 5% carbon dioxide gas flow. After completion of the incubation, 20 µl of Cell Counting Kit-8 (Dojindo) was added thereto. After reacting at 37°C for 90 minutes in a 5% carbon dioxide gas flow, a difference in absorbance between 450 nm and 620 nm was determined. The cell growth inhibition activity of the rabbit polyclonal antibodies described above was calculated based on the activity in the absence of any antibody as a negative control.

As a result, the rabbit polyclonal antibodies No. 1, No. 2 and No. 4 showed 26%, 19% and 32% of the growth inhibition activities, respectively, against NCI-H358 at the final concentration of 100 µg/ml and No. 4 showed 11% of the growth inhibition activity at the final concentration of 10 µg/ml, indicating that there was a statistically significant difference (P≦0.01) (TABLE 10).

The rabbit polyclonal antibodies No. 1, No. 2 and No. 4 showed 45%, 46% and 55% of the growth inhibition activities against SEMA4B/H358 at the final concentration of 100 µg/ml, respectively, and No. 1 and No. 4 showed 11% and 21% of the growth inhibition activities at the final concentration of 10 µg/ml, respectively, indicating that there was a statistically significant difference (P≦0.01) (TABLE 11).

Since non-immunized rabbit IgG did not show any statistically significant growth inhibition activity against any of the cell lines (TABLES 10 and 11), it became clear that the growth inhibition activity was SEMA4B-specifically induced

**[TABLE 10]**

| | Concentration | Cell Growth Activity (A₄₅₀-A₆₂₀) | |
|---|---|---|---|
| | (µg/ml) | Mean Value | Standard Deviation |
| No cell added | | 0.123 | 0.002 |
| Absence of antibody | | 1.563 | 0.018 |
| Non-immunized rabbit IgG | 100 | 1.539 | 0.047 |
| | 10 | 1.552 | 0.027 |
| | 1 | 1.596 | 0.027 |
| Rabbit polyclonal antibody (No. 1) | 100 | 1.164 | 0.048 |
| | 10 | 1.444 | 0.051 |
| | 1 | 1.522 | 0.067 |
| Rabbit polyclonal antibody (No. 2) | 100 | 1.267 | 0.029 |
| | 10 | 1.502 | 0.033 |
| | 1 | 1.519 | 0.135 |
| Rabbit polyclonal antibody (No. 4) | 100 | 1.066 | 0.016 |
| | 10 | 1.395 | 0.004 |
| | 1 | 1.466 | 0.084 |

**[TABLE 11]**

| | Concentration | Cell Growth Activity (A₄₅₀-A₆₂₀) | |
|---|---|---|---|
| | (µg/ml) | Mean Value | Standard Deviation |
| No cell added | | 0.124 | 0.001 |
| Absence of antibody | | 1.561 | 0.048 |
| Non-immunized rabbit IgG | 100 | 1.518 | 0.095 |
| | 10 | 1.512 | 0.049 |
| | 1 | 1.532 | 0.045 |
| Rabbit polyclonal antibody (No. 1) | 100 | 0.866 | 0.052 |
| | 10 | 1.382 | 0.028 |
| | 1 | 1.503 | 0.067 |
| Rabbit polyclonal antibody (No. 2) | 100 | 0.844 | 0.037 |
| | 10 | 1.422 | 0.080 |
| | 1 | 1.627 | 0.059 |
| Rabbit polyclonal antibody (No. 4) | 100 | 0.705 | 0.019 |
| | 10 | 1.232 | 0.056 |
| | 1 | 1.530 | 0.034 |

### INDUSTRIAL APPLICABILITY

The substance in accordance with the present invention that inhibits the binding of a protein comprising the same or substantially the same amino acid sequence as the amino acid sequence represented by SEQ ID NO: 1, SEQ ID NO: 4, SEQ ID NO: 7 or SEQ ID NO: 10 (the protein used in the present invention), to a protein comprising the same or substantially the same amino acid sequence as the amino acid sequence represented by SEQ ID NO: 26 (the receptor used in the present invention) [preferably, an antibody having the activity of neutralizing cancer cell growth stimulation induced by the binding of the aforesaid protein to the aforesaid receptor (hereinafter sometimes simply referred to as the neutralizing antibody)] can be safely used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), an agent for promoting apoptosis in cancer cells, an agent for inhibiting cancer cell growth, or the like. Also, the substance (preferably, the neutralizing antibody, etc.) that inhibits the activity of the protein used in the present invention and/or the activity of the receptor used in the present invention can be safely used as an agent for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), an agent for promoting apoptosis in cancer cells, an agent for inhibiting cancer cell growth, or the like.

## Claims

1. A substance that inhibits the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.

2. The substance according to claim 1, wherein the substance is an antibody to a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.

3. The substance according to claim 1, wherein the substance is an antibody having the activity of neutralizing cancer cell growth stimulation induced by the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof

4. The substance according to claim 2 or 3, wherein the antibody is a monoclonal antibody.

5. An agent for preventing/treating cancer, which comprises the substance according to claim 1.

6. An agent for promoting the apoptosis in cancer cells, which comprises the substance according to claim 1.

7. An agent for inhibiting the growth of cancer cells, which comprises the substance according to claim 1.

8. An agent for inhibiting the growth of cancer cells, which comprises a substance that inhibits the activity of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof.

9. A substance that inhibits the activity of a protein comprising the same or substantially the same amino acid sequence as the amino acid sequence represented by SEQ ID NO: 26, its partial peptide or a salt thereof.

10. The substance according to claim 9, wherein the substance is an antibody having the activity of inhibiting phosphorylation of a protein comprising the same or substantially the same amino acid sequence as the amino acid sequence represented by SEQ ID NO: 26, its partial peptide or a salt thereof.

11. The substance according to claim 10, wherein the substance is an antibody having the activity of neutralizing cancer cell growth stimulation induced by the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.

12. An agent for preventing/treating cancer, which comprises the substance according to claim 9.

13. An agent for promoting the apoptosis in cancer cells, which comprises the substance according to claim 9.

14. An agent for inhibiting the growth of cancer cells, which comprises the substance according to claim 9.

15. A method of screening a substance that inhibits the binding of (a) a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to (b) a protein comprising the same or substantially the same amino acid sequence as the amino acid sequence represented by SEQ ID NO: 26, its partial peptide or a salt thereof, which comprises using (a) said protein, its partial peptide or a salt thereof, and (b) said protein, its partial peptide or a salt thereof.

16. A kit for screening a substance that inhibits the binding of (a) a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to (b) a protein comprising the same or substantially the same amino acid sequence as the amino acid sequence represented by SEQ ID NO: 26, its partial peptide or a salt thereof, which comprises (a) said protein, its partial peptide or a salt thereof, and (b) said protein, its partial peptide or a salt thereof.

17. A method of screening a substance that inhibits the activity of a protein comprising the same or substantially the same amino acid sequence as the amino acid sequence represented by SEQ ID NO: 26, its partial peptide or a salt thereof, which comprises using a protein comprising the same or substantially the same amino acid sequence as the amino acid sequence represented by SEQ ID NO: 26, its partial peptide or a salt thereof.

18. A kit for screening a substance that inhibits the activity of a protein comprising the same or substantially the same amino acid sequence as the amino acid sequence represented by SEQ ID NO: 26, its partial peptide or a salt thereof, which comprises a protein comprising the same or substantially the same amino acid sequence as the amino acid sequence represented by SEQ ID NO: 26, its partial peptide or a salt thereof.

19. A method of preventing/treating cancer, a method of promoting the apoptosis in cancer cells and/or a method of inhibiting the growth of cancer cells, which comprises inhibiting the binding of a protein comprising the same or substantially the same amino acid sequence as the amino acid sequence represented by SEQ ID NO: 1, SEQ ID NO: 4, SEQ ID NO: 7 or SEQ ID NO: 10, its partial peptide or a salt thereof, to a protein comprising the same or substantially the same amino acid sequence as the amino acid sequence represented by SEQ ID NO: 26, its partial peptide or a salt thereof.

20. The method according to claim 19, which comprises using an antibody to a protein comprising the same or substantially the same as the amino acid sequence represented by SEQ ID NO: 1, SEQ ID NO: 4, SEQ ID NO: 7 or SEQ ID NO: 10, its partial peptide or a salt thereof.

21. A method of preventing/treating cancer, a method of promoting the apoptosis in cancer cells and/or a method of inhibiting the growth of cancer cells, which comprises inhibiting the phosphorylation of a protein comprising the same or substantially the same amino acid sequence as the amino acid sequence represented by SEQ ID NO: 26, its partial peptide or a salt thereof.

22. The method according to claim 21, which comprises using an antibody to a protein comprising the same or substantially the same as the amino acid sequence represented by SEQ ID NO: 1, SEQ ID NO: 4, SEQ ID NO: 7 or SEQ ID NO: 10, its partial peptide or a salt thereof.

23. A method of preventing/treating cancer, a method of promoting the apoptosis in cancer cells and/or a method of inhibiting the growth of cancer cells, which comprises administering to a mammal an effective dose of the substance according to claim 1 or 9.

24. Use of the substance according to claim 1 or 9 to manufacture an agent for preventing/treating cancer, an agent for promoting the apoptosis in cancer cells and/or an agent for inhibiting the growth of cancer cells.
